# EUROPEAN PATENT APPLICATION

(11) **EP 4 831 156 A1**
(43) Date of publication of application: **28.10.2026**
(21) Application number: 24906443.7
(22) Date of filing: 19.12.2024
(51) Int. Cl.: C07C 255/42, C07C 255/30, A61K 31/277, A61P 37/02, A61P 35/00

(54) **SUBSTITUTED N-(4-TRIFLUOROMETHYL)-2-CYANOCROTONAMIDE COMPOUND, PREPARATION METHOD THEREFOR, AND PHARMACEUTICAL COMPOSITION AND USE THEREOF**

(30) Priority: 21.12.2023 CN 202311768913; 18.12.2024 CN 202411869302
(71) Applicant: Tianjin Gudui Bio-Pharm Co., Ltd., Tianjin 300384 (CN)
(72) Inventor: GAO, Qingzhi, Tianjin 300384 (CN); LIU, Shengnan, Tianjin 300384 (CN); GAO, Xiangqian, Tianjin 300384 (CN); HAN, Jianbin, Tianjin 300384 (CN); ZHANG, Shunjie, Tianjin 300384 (CN); ZHAO, Fuping, Tianjin 300384 (CN); HAN, Xu, Tianjin 300384 (CN); ZHU, Xiaoling, Tianjin 300384 (CN); LIU, Yang, Tianjin 300384 (CN)
(74) Representative: Bandpay & Greuter
(86) International application number: PCT/CN2024/140536
(87) International publication number: WO 2025/130964

(57) **Abstract**

An N-(4-trifluoromethyl)-2-cyanocrotonamide compound, a preparation method therefor, and a pharmaceutical composition and use thereof. The compound of the present disclosure has a more significant treatment effect on central nervous system diseases such as sclerosis and elitis optica.

## Description

### Technical Field

The present disclosure relates to an N-(4-trifluoromethyl)-2-cyanocrotonamide compound, a preparation method therefor, and a pharmaceutical composition and use thereof.

### Background

Located on the outer surface of the mitochondrial inner membrane, Dihydroorotate Dehydrogenase (DHODH) is a key enzyme in pyrimidine synthesis during nucleic acid catalysis and an enzyme related to the electron transport chain; it is also involved in mitochondrial energy metabolism, cell proliferation, apoptosis, cell membrane potential, and the generation of reactive oxygen species (ROS), and thus can mediate the occurrence of diseases such as cancer, autoimmune diseases, bacterial or viral infections, and parasitic diseases.

Leflunomide, an isoxazole compound, is the first FDA-approved inhibitor of DHODH. It inhibits the proliferation of activated immune cells and the secretion of cytokines by blocking pyrimidine synthesis in immune cells, and is used for the treatment of autoimmune diseases, such as rheumatoid arthritis, lupus nephritis, and psoriatic arthritis. After entering the human body, Leflunomide is rapidly hydrolyzed and converted into its active metabolite Teriflunomide in vivo, and this compound is stable in a matrix. However, the enzymatic conversion of Leflunomide in vivo may be impaired in cases of drug combination or certain disease states, which affects the therapeutic efficacy. As for direct administration of Teriflunomide, plasma drug concentrations are independent of enzymatic conversion, thereby ensuring drug release and higher bio-availability, and reducing exposure to minor metabolites. Therefore, the mechanism of action of Teriflunomide is to, by inhibiting DHODH, suppress T-cell proliferation and DNA/RNA synthesis in vitro, and inhibit cell surface antigens and nuclear antigens directly involved in T-cell activation and proliferation, thereby playing the role of anti-proliferative and anti-inflammatory effects, as well as immunomodulation.

Both Leflunomide and Teriflunomide demonstrate significant efficacy, favorable safety, and good tolerability in the treatment of various diseases such as multiple sclerosis, rheumatoid arthritis, lupus nephritis, and acute leukemia. Nevertheless, drug metabolism remains an unavoidable key issue for them both. The half-life of Leflunomide is approximately 15 days, and its active metabolite Teriflunomide enters the enterohepatic circulation in large quantities with a longer half-life, which is typically 1 to 4 weeks, and can be detected in serum even up to 2 years after drug discontinuation. Consequently, severe adverse reactions (e.g., hepatotoxicity, hematologic toxicity, or allergic reactions) may occur even after drug withdrawal. An elimination procedure must be followed for the rapid clearance of Teriflunomide from the body in cases of planned pregnancy, accidental pregnancy, occurrence of the aforementioned toxic and side effects, or any other reasons.

In addition, Teriflunomide cannot be metabolized by cytochrome P450 or flavin-containing monoamine oxidases. It acts as an inhibitor of CYP2C8 and a weak inducer of CYP1A2, and can inhibit activities of organic anion transporter 3 (OAT3) and organic anion transporting polypeptides B1 and B3 (OATP1B1/1B3), which may affect the in vivo exposure of various drugs.

Accordingly, development needs for novel inhibitors of this class are to enhance the inhibition of lymphocytes, to reduce the levels of inflammatory factors in vivo, to improve therapeutic efficacy, to decrease toxic and side effects, to optimize the absorption, distribution, metabolism and excretion profiles of drugs in vivo, to provide new therapeutic options for patients, and to address unmet clinical needs.

### Summary

The present disclosure provides an N-(4-trifluoromethyl)-2-cyanocrotonamide compound, namely a compound represented by the following Formula (I), or an optical isomer thereof, or a pharmaceutically acceptable salt thereof, or a solvate thereof (e.g., a hydrate), or an inclusion complex thereof, or a racemate thereof, or a co-crystal thereof, or an isotope-labeled compound thereof, or nitrogen oxides thereof, or a prodrug thereof, or an active metabolite thereof. The compound exhibits significantly improved pharmaceutical activity, and can effectively treat and/or prevent inflammation, pain, multiple sclerosis, neuromyelitis optica, autoimmune diseases or cancer. Further provided are preparation methods, pharmaceutical compositions and uses of drugs comprising the compound.

A compound represented by Formula (I), or an optical isomer thereof, or a pharmaceutically acceptable salt thereof, or a solvate thereof (optionally, the solvate is a hydrate), or an inclusion complex thereof, or a racemate thereof, or a co-crystal thereof, or an isotope-labeled compound thereof, or nitrogen oxides thereof, or a prodrug thereof, or an active metabolite thereof, 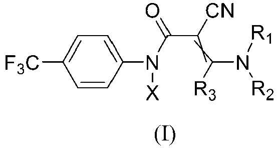 wherein:
R₁ is selected from: -H, alkyl, -alkylene-C(=O)O-alkyl, -S(=O)₂R₄₋₁, optionally substituted monosaccharide group, -C(=O)R₄₋₂, -C(=O)N(R₄₋₃)R₄₋₄, -O-R₄₋₅, -A-Linker-R₄₋₆, and -P(=O)(OR₄₋₇)OR₄₋₈;
R₂ is selected from: -H, alkyl, aryl, cycloalkyl, -alkylene-aryl, -alkylene-C(=O)OH, -alkylene-C(=O)O-alkyl, -S(=O)₂R₄₋₁, optionally substituted monosaccharide group, -C(=O)R₄₋₂, -C(=O)N(R₄₋₃)R₄₋₄, -O-R₄₋₅, -A-Linker-R₄₋₆, -P(=O)(OR₄₋₇)OR₄₋₈, -alkylene-O-C(=O)R₄₋₁₁, -alkylene-O-P(=O) (OR₄₋₇)OR₄₋₈, and -alkylene-O-C(=O)-OR₄₋₁₈;
alternatively, R₁ and R₂ are interconnected and, together with nitrogen atoms to which they are attached, jointly form an optionally substituted aliphatic heterocyclyl group;
alternatively, R₁ and R₂, together with nitrogen atoms to which they are attached, are represented as: 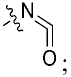
X is selected from -H, or X and R₁ jointly form a cyclic amide structure; 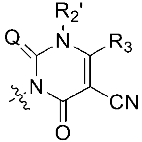
R₂' is selected from: -H, alkyl, aryl, cycloalkyl, -alkylene-aryl, -alkylene-C(=O)OH, -alkylene-C(=O)O-alkyl, -S(=O)₂R₄₋₁, optionally substituted monosaccharide group, -C(=O)R₄₋₂, -C(=O)N(R₄₋₃)R₄₋₄, -O-R₄₋₅, -A-Linker-R₄₋₆, -P(=O)(OR₄₋₇)OR₄₋₈, -alkylene-O-C(=O)R₄₋₁₁, -alkylene-O-P(=O) (OR₄₋₇)OR₄₋₈, and -alkylene-O-C(=O)-OR₄₋₁₀;
optionally, R₂' is -H;
Q is selected from O or -O-cycloalkyl;
optionally, Q is O;
each R₃ is individually and independently selected from: -H, and alkyl or -alkylene-O-C(=O)-OR₄₋₂;
R₃ is selected from: -H, and alkyl;
optionally, R₃ is methyl;
A is selected from: -C(=O)-, and alkylene;
R₄₋₁ is selected from: alkyl, hydroxy, amino, carboxy, halogen, nitro, and cyano; preferably, R₄₋₁ is alkyl;
R₄₋₂ is selected from: -OH, aryl, alkyl, alkoxy, -alkenyl-carboxy, -alkenyl-alkoxycarbonyl, and -alkylene-R₄₋₉;
R₄₋₃ and R₄₋₄ are the same or different, and are individually and independently selected from H, and alkyl;
alternatively, R₄₋₃ and R₄₋₄ are interconnected and, together with nitrogen atoms to which they are attached, jointly form an optionally substituted aliphatic heterocyclyl group;
R₄₋₅ is selected from: H, alkyl, and -alkylene-aryl;
Linker is an optionally present group, and, when present, is selected from: alkylene, -B-alkylene-, -B-alkylene-arylene-, and -B-arylene-alkylene-;
each B is individually and independently selected from -O-, -S-, and -NH-;
R₄₋₆ is 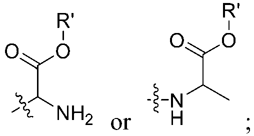
each R' is individually and independently selected from: -H, alkyl, -alkylene-aryl, and hydroxy-substituted alkyl;
each R₄₋₇ and R₄₋₈ are the same or different, and are individually and independently selected from H, alkyl, and -alkylene-aryl;
R₄₋₉ is selected from: 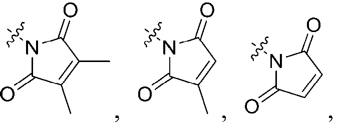 aryl, and -C(=O)O-alkyl;
the "optionally substituted monosaccharide group" means that one or more hydrogen atoms on a monosaccharide group are unsubstituted or are substituted with a substituent selected from: -C(=O)OR₄₋₁₀;
the monosaccharide group in the optionally substituted monosaccharide group is selected from the following monosaccharide groups, wherein 1-position substitution of the monosaccharide is α-substitution, or β-substitution, or both: 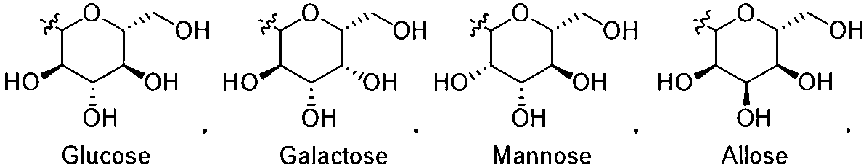 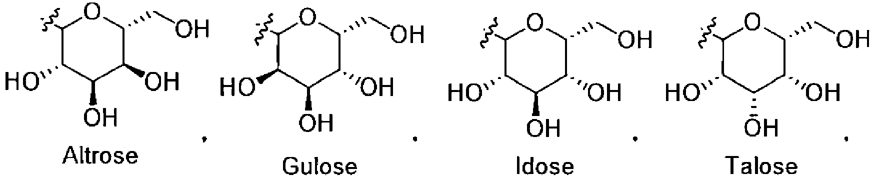 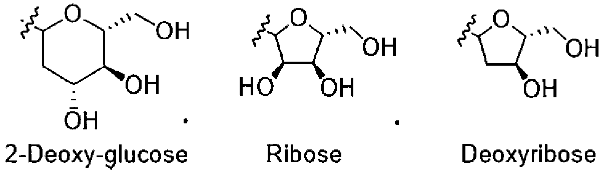 ;
2-position substitution of the monosaccharidei is α-configuration, β-configuration, or both: 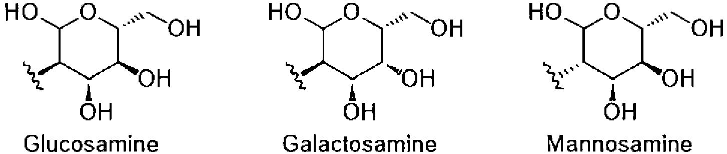
R₄₋₁₀ is selected from: H, and alkyl;
R₄₋₁₁ is selected from: alkyl, and aryl;
"optionally substituted aliphatic heterocyclyl group" means that one or more hydrogen atoms on the aliphatic heterocyclyl group are unsubstituted or are substituted with substituents, and the substituents in the "optionally substituted aliphatic heterocyclyl group" are individually and independently selected from: hydroxy, amino, carboxy, halogen, nitro, cyano, alkyl, alkylthio, alkanoyl, and hydroxy-substituted aryl; and
the "hydroxy-substituted alkyl" means that one or more hydrogen atoms on the alkyl are unsubstituted or are substituted with hydroxy.

According to another embodiment of the present disclosure, in Formula (I):
R₁ and R₂ are the same or different, and are individually and independently selected from: -H, alkyl, -alkylene-C(=O)O-alkyl, -S(=O)₂R₄₋₁, optionally substituted monosaccharide group, -C(=O)R₄₋₂, -C(=O)N(R₄₋₃)R₄₋₄, -O-R₄₋₅, -A-Linker-R₄₋₆, and -P(=O)(OR₄₋₇)OR₄₋₈;
alternatively, R₁ and R₂ are interconnected and, together with nitrogen atoms to which they are attached, jointly form an optionally substituted aliphatic heterocyclyl group;
alternatively, R₁ and R₂, together with nitrogen atoms to which they are attached, are represented as: 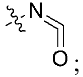
X is selected from -H, or X and R₁ jointly form a cyclic amide structure 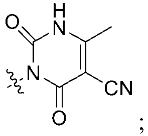
R₃ is selected from: -H, and alkyl;
A is selected from: -C(=O)-, and alkylene;
R₄₋₁ is selected from: alkyl, hydroxy, amino, carboxy, halogen, nitro, and cyano; preferably, R₄₋₁ is alkyl;
R₄₋₂ is selected from: -OH, aryl, alkyl, alkoxy, -alkenyl-carboxy, -alkenyl-alkoxycarbonyl, and -alkylene-R₄₋₉;
R₄₋₃ and R₄₋₄ are the same or different, and are individually and independently selected from H, and alkyl;
alternatively, R₄₋₃ and R₄₋₄ are interconnected and, together with nitrogen atoms to which they are attached, jointly form an optionally substituted aliphatic heterocyclyl group;
R₄₋₅ is selected from: H, alkyl, and aryl;
Linker is an optionally present group, and, when present, is selected from: alkylene, -B-alkylene-, -B-alkylene-arylene-, and -B-arylene-alkylene-;
each B is individually and independently selected from -O-, -S-, and -NH-;
R₄₋₆ is 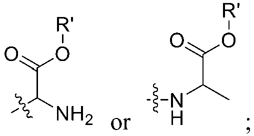
each R' is individually and independently selected from: -H, alkyl, -alkylene-aryl, and hydroxy-substituted alkyl;
R₄₋₇ and R₄₋₈ are the same or different, and are individually and independently selected from H, alkyl, and -alkylene-aryl;
R₄₋₉ is selected from: 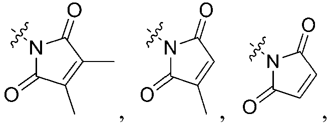 aryl, and -C(=O)O-alkyl;
the "optionally substituted monosaccharide group" means that one or more hydrogen atoms on a monosaccharide group are unsubstituted or are substituted with a substituent selected from: -C(=O)OR₄₋₁₀;
the monosaccharide group in the optionally substituted monosaccharide group is selected from the following monosaccharide groups, wherein 1-position substitution of the monosaccharide is α-substitution, or β-substitution, or both: 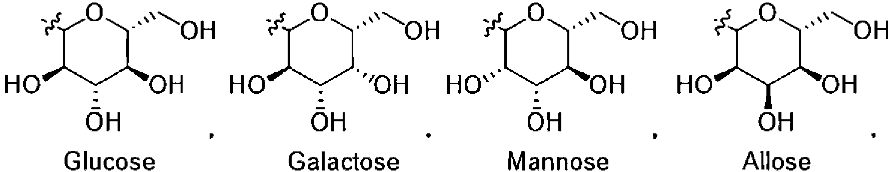 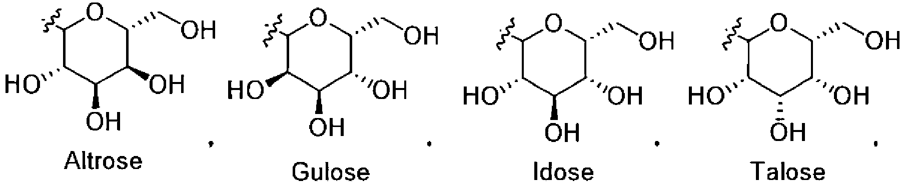 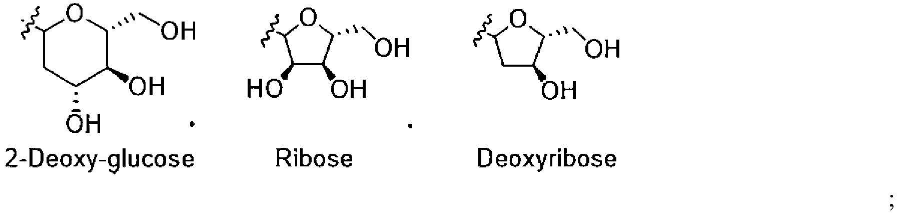
2-position substitution of the monosaccharidei is α-configuration, β-configuration, or both: 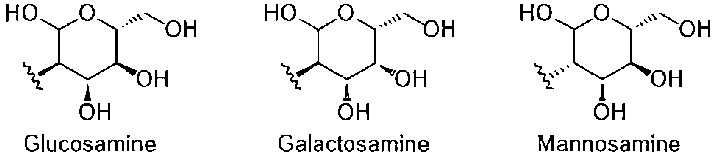
R₄₋₁₀ is selected from: H, and alkyl;
substituents in the "optionally substituted aliphatic heterocyclyl group" are individually and independently selected from: hydroxy, amino, carboxy, halogen, nitro, cyano, alkyl, alkylthio, alkanoyl, and hydroxy-substituted aryl; and
the "hydroxy-substituted alkyl" means that one or more hydrogen atoms on the alkyl are unsubstituted or are substituted with hydroxy.

In one embodiment of the present disclosure,
optionally, the halogen is selected from F, Cl, Br, and I; optionally, the alkyl moieties in "alkyl", "alkanoyl", "hydroxy-substituted alkyl", "alkoxy", "-C(=O)O-alkyl", and "alkoxycarbonyl" are individually and independently a C₁₋₂₀ straight or branched alkyl, optionally a C₁₋₁₇ straight or branched alkyl, optionally a C₁₋₁₃ straight or branched alkyl, optionally a C₁₋₁₀ straight or branched alkyl, optionally a C₁₋₇ straight or branched alkyl, optionally a C₁₋₅ straight or branched alkyl, optionally methyl, ethyl, n-propyl, isopropyl, n-butyl, isobutyl, tert-butyl, sec-butyl, n-pentyl, isopentyl, neopentyl, tert-pentyl, n-hexyl, isohexyl, heptyl, n-octyl, n-nonyl, n-decyl, undecyl, dodecyl, tridecyl, tetradecyl, pentadecyl, hexadecyl, or heptadecyl; and optionally methyl, ethyl, n-propyl, isopropyl, n-butyl, isobutyl, tert-butyl, sec-butyl;
optionally, the alkyl moieties in the "-alkylene-C(=O)O-alkyl" are individually and independently a C₁₋₂₀ straight or branched alkyl, optionally a C₁₋₁₇ straight or branched alkyl, optionally a C₁₋₁₃ straight or branched alkyl, optionally a C₁₋₁₀ straight or branched alkyl, optionally a C₁₋₇ straight or branched alkyl, optionally a C₁₋₅ straight or branched alkyl, optionally methyl, ethyl, n-propyl, isopropyl, n-butyl, isobutyl, tert-butyl, sec-butyl, n-pentyl, isopentyl, neopentyl, tert-pentyl, n-hexyl, isohexyl, heptyl, n-octyl, n-nonyl, n-decyl, undecyl, dodecyl, tridecyl, tetradecyl, pentadecyl, hexadecyl, or heptadecyl; and optionally methyl, ethyl, n-propyl, isopropyl, n-butyl, isobutyl, tert-butyl, sec-butyl;
optionally, the "alkylene" in "-alkylene-", "-alkylene-aryl", "-B-alkylene-", "-B-alkylene-arylene-", "-B-arylene-alkylene-", "-O-alkylene-R₄₋₅", and "-alkylene-R₄₋₉" is a C₁₋₂₀ straight or branched alkylene, optionally a C₁₋₁₇ straight or branched alkylene, optionally a C₁₋₁₀ straight or branched alkylene, optionally a C₁₋₈ straight or branched alkylene, optionally a C₁₋₅ straight or branched alkylene, optionally a C₁₋₃ straight or branched alkylene, and optionally methylene, ethylene, isopropylene, n-propylene, isopropylene, n-butylene, isobutylene, tert-butylene, sec-butylene, n-pentylene, isopentylene, neopentylene, tert-pentylene, n-hexylene, isohexylene, heptylene, n-octylene, n-nonylene, n-decylene;
optionally, the "alkylene" in the "-alkylene-C(=O)O-alkyl", "-alkylene-O-C(=O)R₄₋₁₁", "-alkylene-O-P(=O)(OR₄₋₇)OR₄₋₈", "-alkylene-O-C(=O)-OR₄₋₁₀", "-alkylene-O-C(=O)-OR₄₋₂", and "-alkylene-C(=O)OH" is a C₁₋₂₀ straight or branched alkylene, optionally a C₁₋₁₇ straight or branched alkylene, optionally a C₁₋₁₀ straight or branched alkylene, optionally a C₁₋₈ straight or branched alkylene, optionally a C₁₋₅ straight or branched alkylene, optionally a C₁₋₃ straight or branched alkylene, and optionally methylene, ethylene, isopropylene, n-propylene, isopropylene, n-butylene, isobutylene, tert-butylene, sec-butylene, n-pentylene, isopentylene, neopentylene, tert-pentylene, n-hexylene, isohexylene, heptylene, n-octylene, n-nonylene, n-decylene;
optionally, the aliphatic heterocycle in the "aliphatic heterocyclyl group" is a C₃₋₈ (preferably C₄₋₆) aliphatic heterocycle containing 1 to 3 heteroatoms selected from O, N, and S on the ring, and optionally dioxolane, aziridinyl, azetidinyl, pyrrolidinyl, morpholinyl, piperidinyl, or piperazinyl;
optionally, the aryl in the "aryl", "hydroxy-substituted aryl", and "-alkylene-aryl" is a 6- to 10-membered monocyclic or bicyclic fused aromatic ring group; and optionally phenyl or naphthyl;
optionally, the arylene in the "-B-alkylene-arylene-", "-B-arylene-alkylene-", and "-alkylene-arylene-" is a 6- to 10-membered monocyclic or bicyclic fused aromatic ring group; and optionally phenylene or naphthylene;
optionally, the alkenyl moieties in the "alkenyl-carboxy" and "alkenyl-alkoxycarbonyl" are individually and independently a C₂-C₈ straight or branched alkenyl containing one or more double bonds, optionally a C₂-C₆ straight or branched alkenyl, optionally a C₂-C₄ straight or branched alkenyl; and optionally ethenyl, propenyl, butenyl, pentenyl, ethynyl, and hexynyl;
optionally, the cycloalkyl in the "cycloalkyl" and "-O-cycloalkyl" is a 3- to 7-membered monocyclic cycloalkyl, and optionally selected from: cyclopropyl, cyclobutyl, cyclopentyl, and cyclohexyl; and
optionally, the present disclosure refers to the aforementioned compounds and any accompanying definitions, wherein the N-(4-trifluoromethyl)-2-cyanocrotonamide moiety is of Z-configuration or E-configuration.

Optionally, when R₁ and R₂ are interconnected and, together with nitrogen atoms to which they are attached, jointly form an optionally substituted aliphatic heterocyclyl group, the optionally substituted aliphatic heterocyclyl group is selected from: 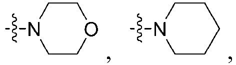 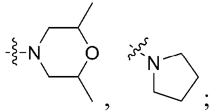
optionally, R₄₋₁ is C₁₋₅ straight or branched alkyl;
optionally, R₄₋₂ is selected from: -OH, phenyl, C₁₋₁₃ straight or branched alkyl, C₁₋₅ straight or branched alkoxy, -C=C-COOH, -C=C-C₁₋₅ straight or branched alkoxycarbonyl, -C₁₋₅ straight or branched alkylene-R₄₋₉;
optionally, R₄₋₃ and R₄₋₄ are the same or different, and are individually and independently selected from H, and alkyl; alternatively, R₄₋₃ and R₄₋₄ are interconnected and, together with nitrogen atoms to which they are attached, jointly form 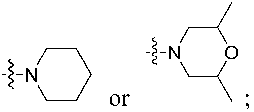
optionally, R₄₋₅ is selected from: H, C₁₋₅ straight or branched alkyl, -C₁₋₅ straight or branched alkylene-phenyl;
optionally, Linker is an optionally present group, and, when present, is selected from: C₁₋₅ straight or branched alkylene, -B-C₁₋₅ straight or branched alkylene-, -B-C₁₋₅ straight or branched alkylene-phenylene-, -B-phenylene-C₁₋₅ straight or branched alkylene-;
optionally, R' is individually and independently selected from: -H, C₁₋₅ straight or branched alkyl, -C₁₋₅ straight or branched alkylene-phenyl, hydroxy-substituted C₁₋₅ straight or branched alkyl;
optionally, the amino acid moiety of R₄₋₆ is of D-configuration or L-configuration;
optionally, R₄₋₇ and R₄₋₈ are the same or different, and are individually and independently selected from: H, C₁₋₅ straight or branched alkyl, -C₁₋₅ straight or branched alkylene-phenyl;
optionally, R₄₋₉ is selected from: 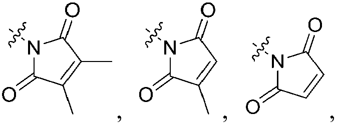 phenyl;
optionally, R₄₋₁₀ is selected from: H, C₁₋₅ straight or branched alkyl;
optionally, R₃ is selected from: -H, C₁₋₅ straight or branched alkyl;
optionally, A is selected from: -C(=O)-, C₁₋₅ straight or branched alkylene;
optionally, R₄₋₁₁ is selected from: H, C₁₋₅ straight or branched alkyl, phenyl;
optionally, Q is O or -O-cyclopentyl;
optionally, a compound represented by Formula (I), or an optical isomer thereof, or a pharmaceutically acceptable salt thereof, or a solvate thereof (optionally, the solvate is a hydrate), or an inclusion complex thereof, or a racemate thereof, or a co-crystal thereof, or an isotope-labeled compound thereof, or nitrogen oxides thereof, or a prodrug thereof, or an active metabolite thereof, wherein,
R₁ and R₂ are the same or different, and are individually and independently selected from: -H, C₁₋₅ straight or branched alkyl, -C₁₋₅ straight or branched alkylene-C(=O)O-C₁₋₅ straight or branched alkyl, -S(=O)₂-C₁₋₅ straight or branched alkyl, -C(=O) R₄₋₂, -C(=O)N(R₄₋₃)R₄₋₄, -O-R₄₋₅, -A-Linker-R₄₋₆, -P(=O)(OR₄₋₇)OR₄₋₈, and the following monosaccharide group, wherein 1-position substitution of the monosaccharide is α-substitution or β-substitution: 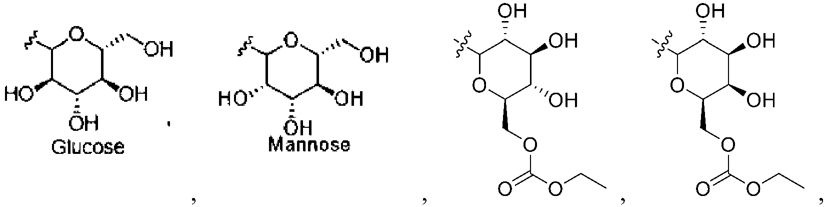 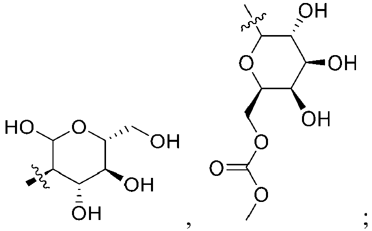
wherein:
   R₄₋₂ is selected from: -OH, phenyl, C₁₋₁₃ straight or branched alkyl, C₁₋₅ straight or branched alkoxy, -C=C-COOH, -C=C-C₁₋₅ straight or branched alkoxycarbonyl, -C₁₋₅ straight or branched alkylene-R₄₋₉;
   R₄₋₃ and R₄₋₄ are the same or different, and are individually and independently selected from: H, C₁₋₅ straight or branched alkyl;
   alternatively, R₄₋₃ and R₄₋₄ are are interconnected and, together with nitrogen atoms to which they are attached, jointly form 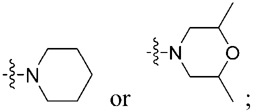
   R₄₋₅ is selected from: H, C₁₋₅ straight or branched alkyl, -C₁₋₅ straight or branched alkylene-phenyl;
   Linker is an optionally present group and, when present, is selected from: C₁₋₅ straight or branched alkylene, -B-C₁₋₅ straight or branched alkylene-, -B-C₁₋₅ straight or branched alkylene-phenylene-, -B-phenylene-C₁₋₅ straight or branched alkylene-;
   A is selected from: -C(=O)-, C₁₋₅ straight or branched alkylene;
   R₃ is selected from: -H, C₁₋₅ straight or branched alkyl;
   R₄₋₇ and R₄₋₈ are the same or different, and are individually and independently selected from: H, C₁₋₅ straight or branched alkyl, -C₁₋₅ straight or branched alkylene-phenyl;
   R₄₋₉ is selected from: 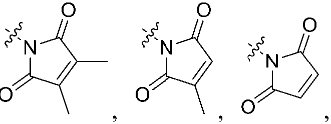 and phenyl;
   R₄₋₁₀ is selected from H, C₁₋₅ straight or branched alkyl;
   alternatively, R₁ and R₂ are are interconnected and, together with nitrogen atoms to which they are attached, jointly form: 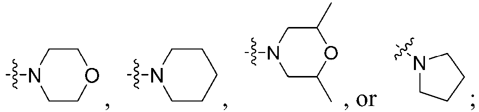
   alternatively, R₁ and R₂, together with nitrogen atoms to which they are attached, are represent as 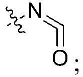
   X is selected from -H;
   each B is individually and independently selected from -O-, -S-, -NH-;
   alternatively, X and R₁ jointly form a cyclic amide structure 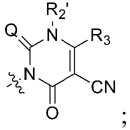
   R₂' is selected from: -H, C₁₋₅ straight or branched alkyl, phenyl, cyclopentyl, -C₁₋₅ straight or branched alkylene-phenyl, -C₁₋₅ straight or branched alkylene-C(=O)OH, -C₁₋₅ straight or branched alkylene-C(=O)O-C₁₋₅ straight or branched alkyl, -C(=O)-phenyl, -alkylene-O-C(=O)R₄₋₁₁, -alkylene-O-P(=O)(OR₄₋₇)OR₄₋₈, -alkylene-O-C(=O)-OR₄₋₁₀; optionally, R₂' is -H;
   R₄₋₁₀ is C₁₋₅ straight or branched alkyl;
   R₄₋₁₁ is C₁₋₅ straight or branched alkyl, phenyl;
   Q is O or -O-cyclopentyl; optionally, Q is O;
   R₃ is selected from: C₁₋₅ straight or branched alkyl, -C₁₋₅ straight or branched alkylene-O-C(=O)-O-C₁₋₅ straight or branched alkyl.

Optionally, a compound represented by Formula (I) in the present disclosure, or an optical isomer thereof, or a pharmaceutically acceptable salt thereof, or a solvate thereof (optionally, the solvate is a hydrate), or an inclusion complex thereof, or a racemate thereof, or a co-crystal thereof, or an isotope-labeled compound thereof, or nitrogen oxides thereof, or a prodrug thereof, or an active metabolite thereof, is selected from the following compounds:

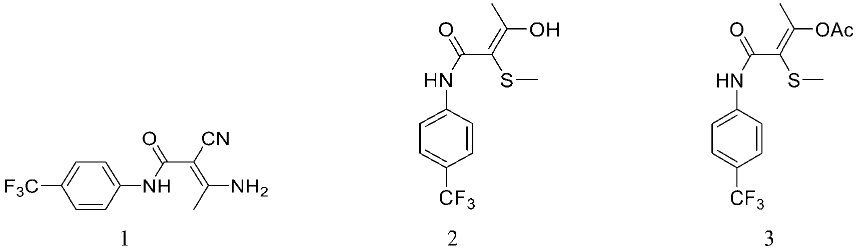

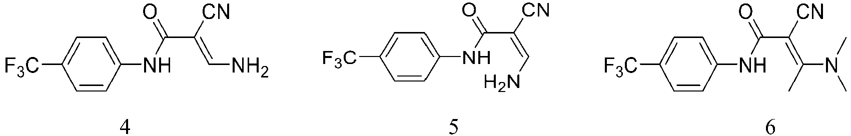

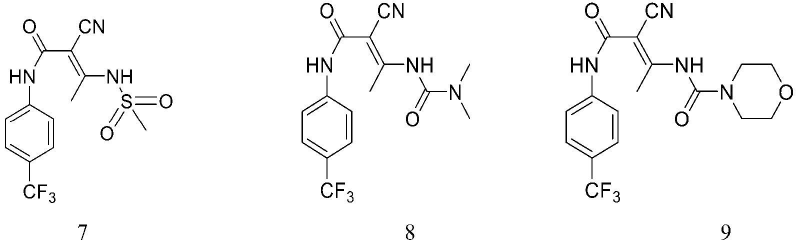

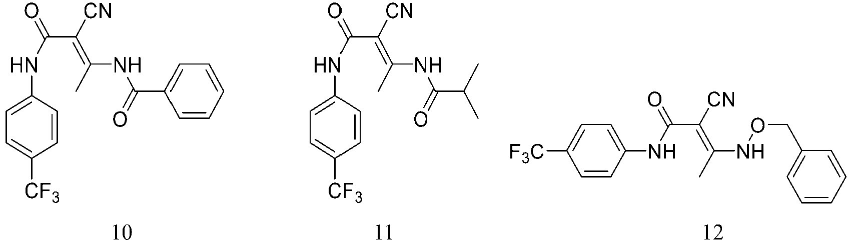

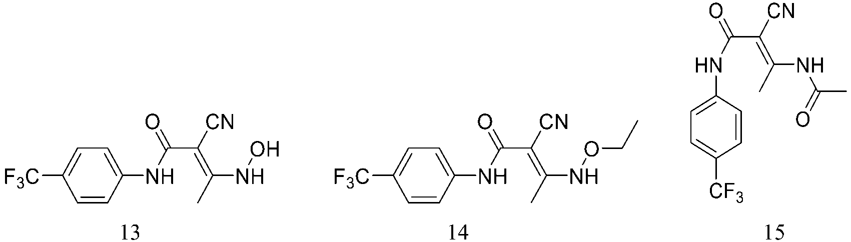

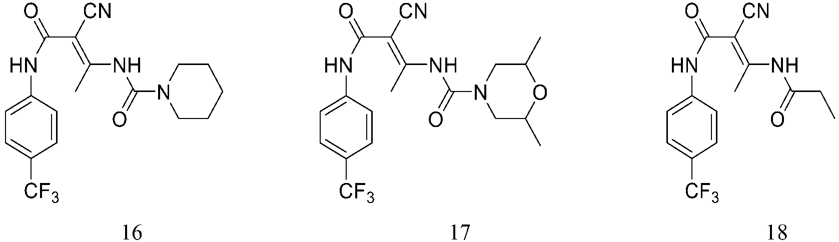

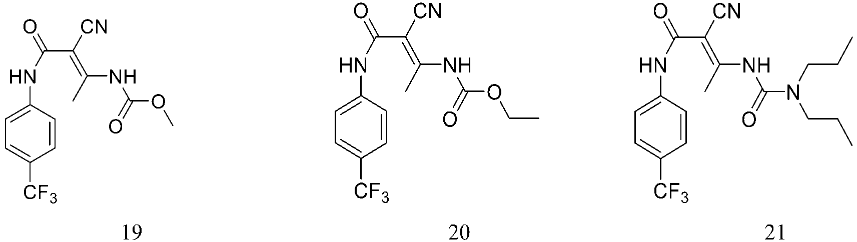

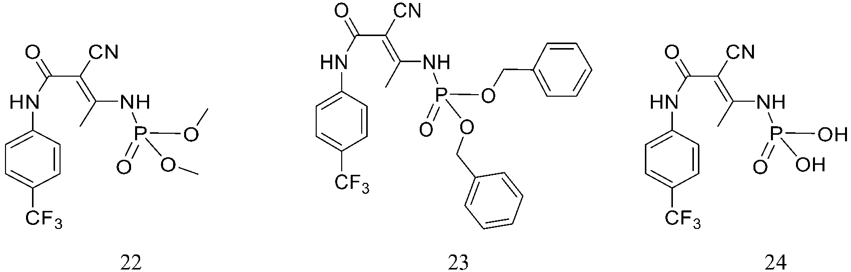

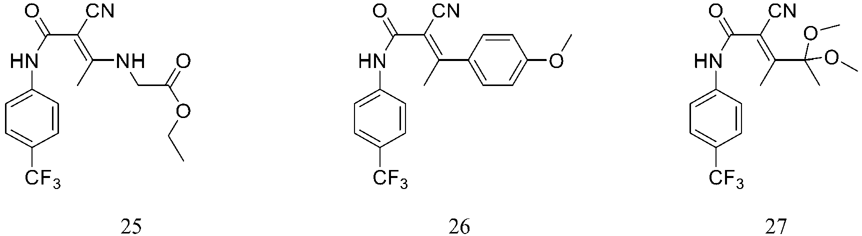

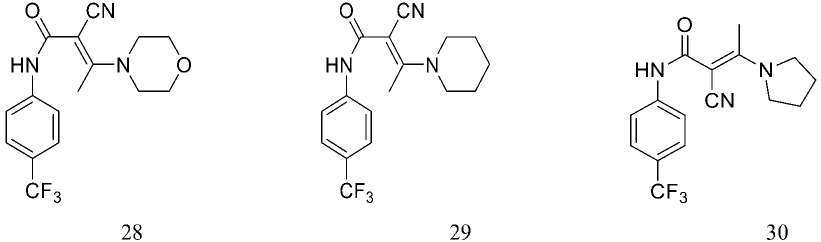

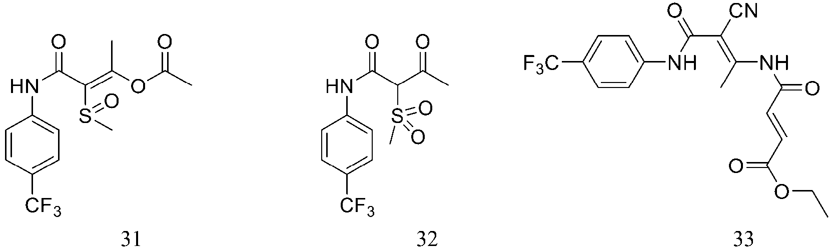

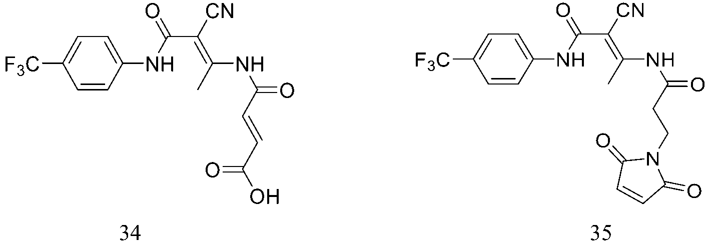

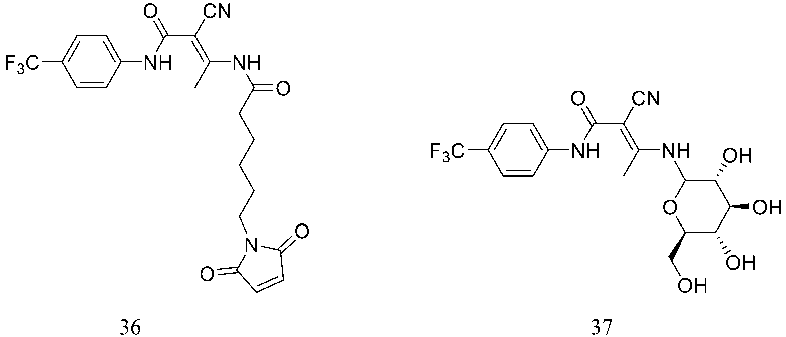

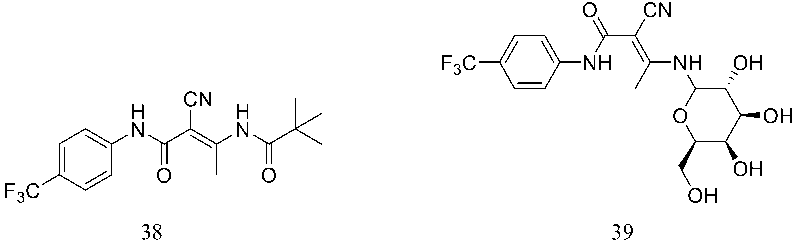

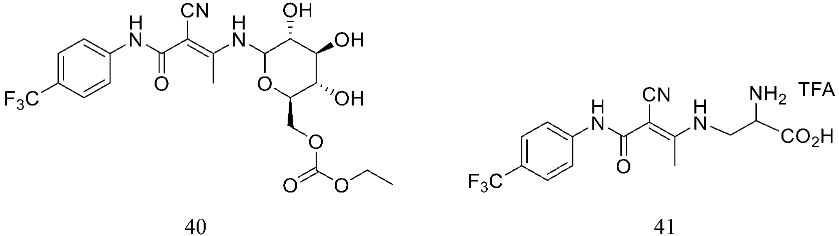

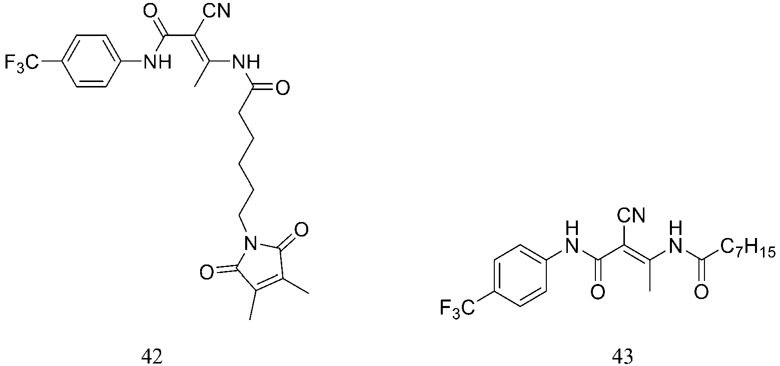

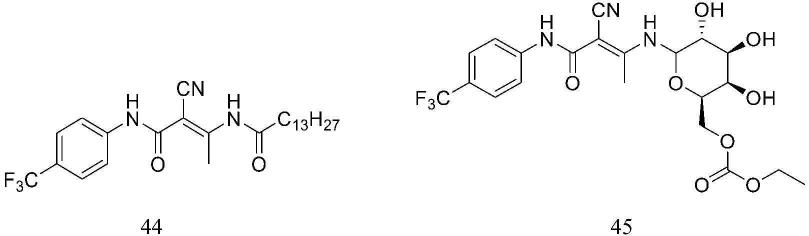

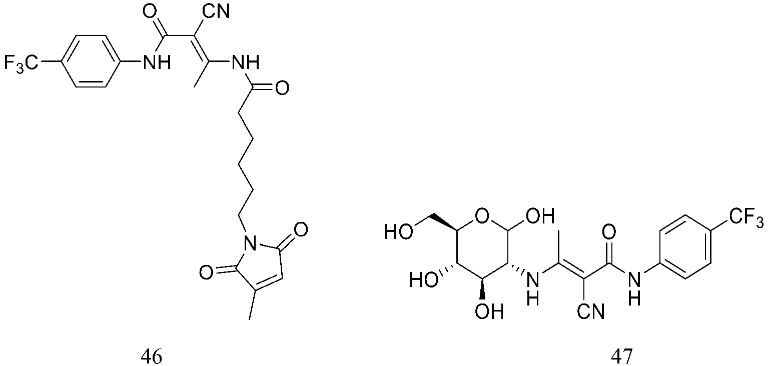

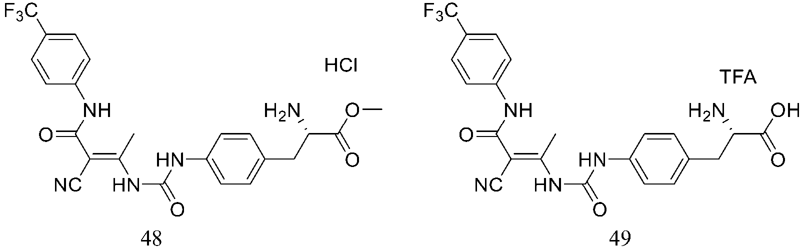

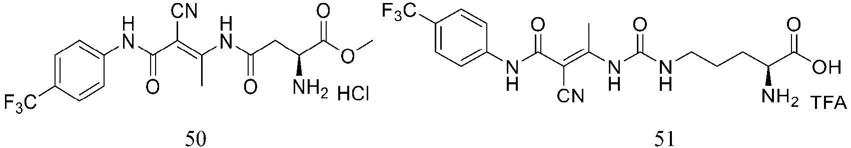

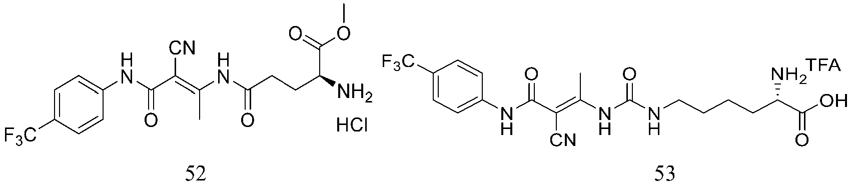

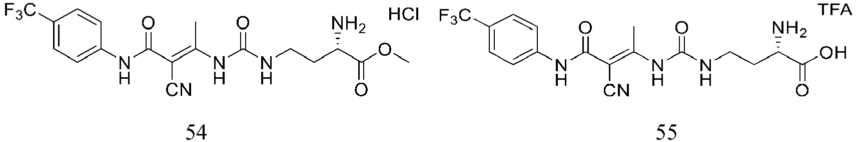

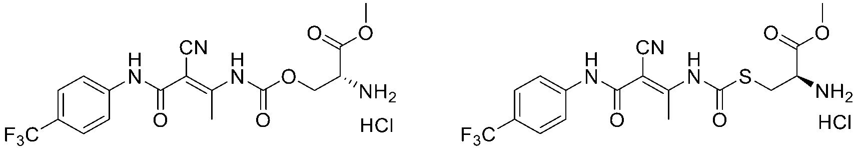

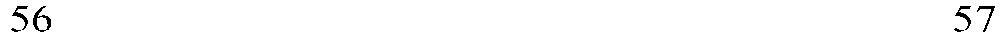

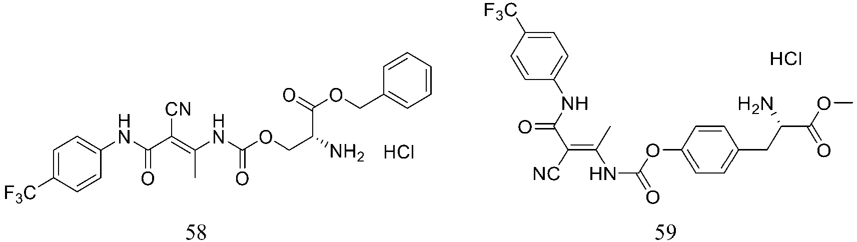

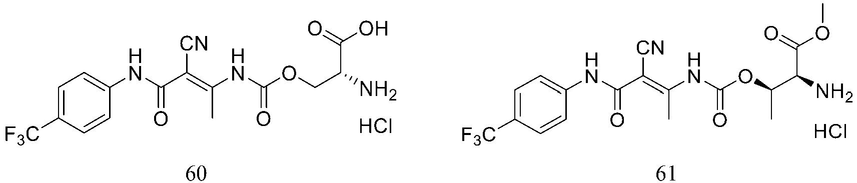

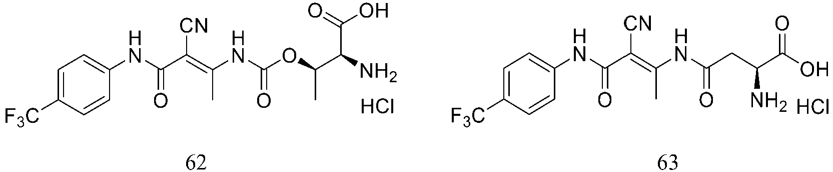

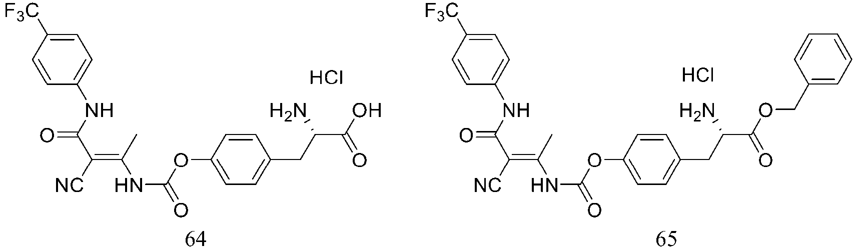

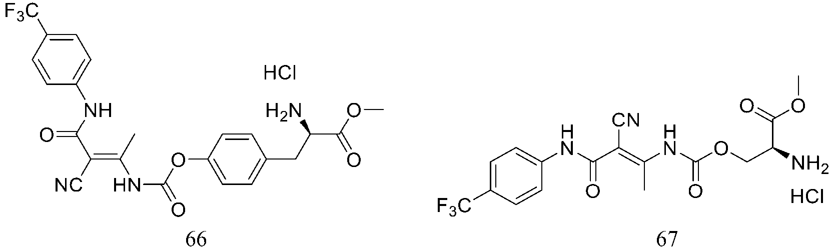

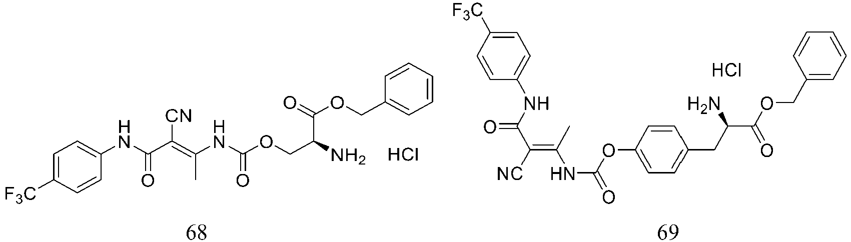

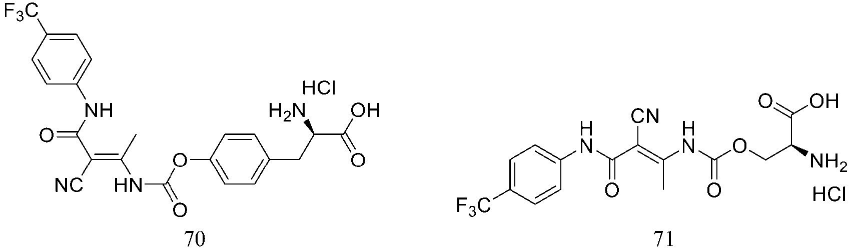

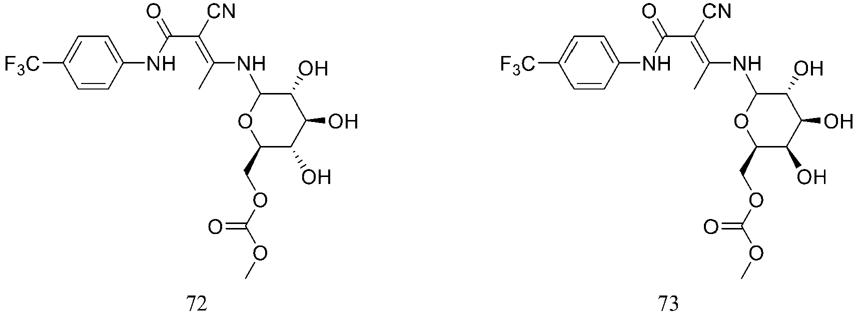

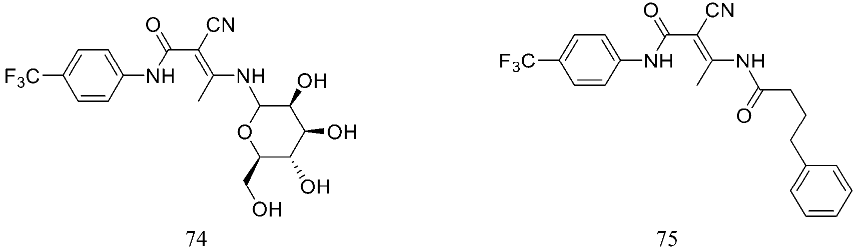

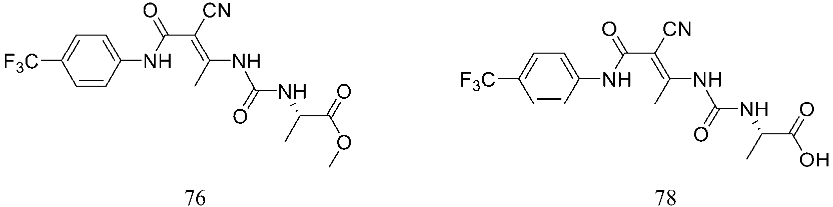

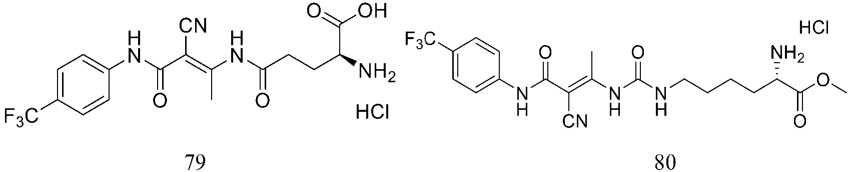

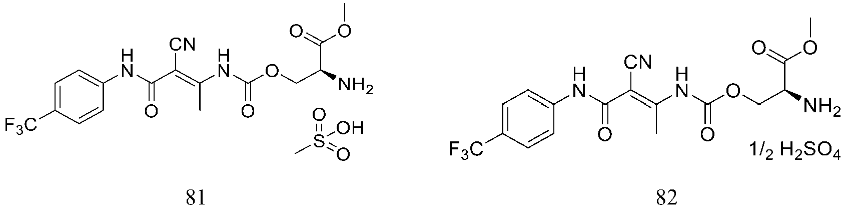

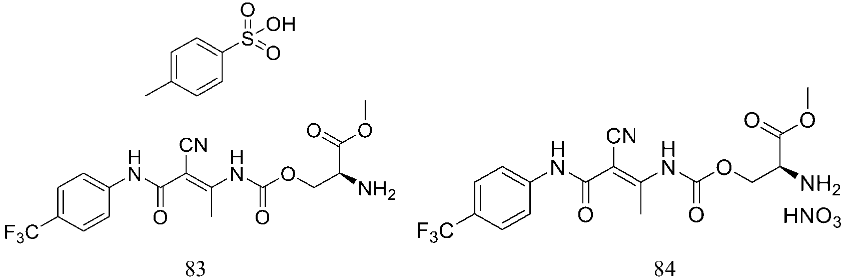

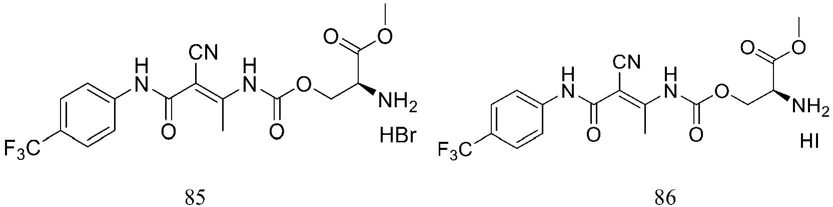

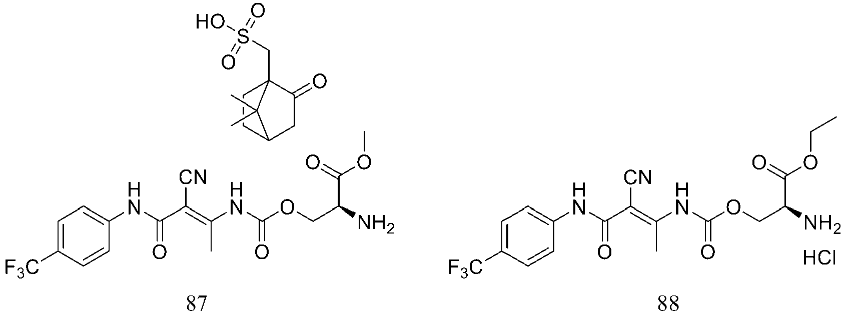

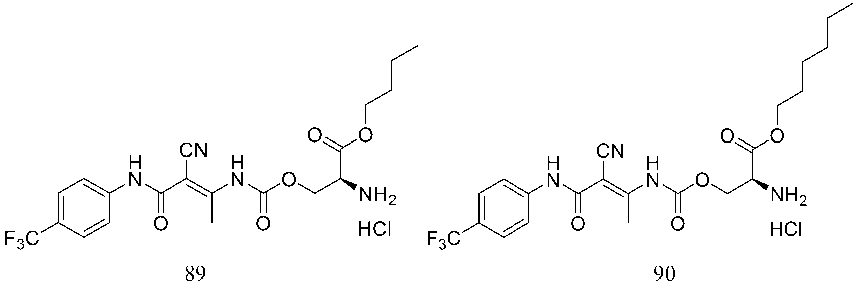

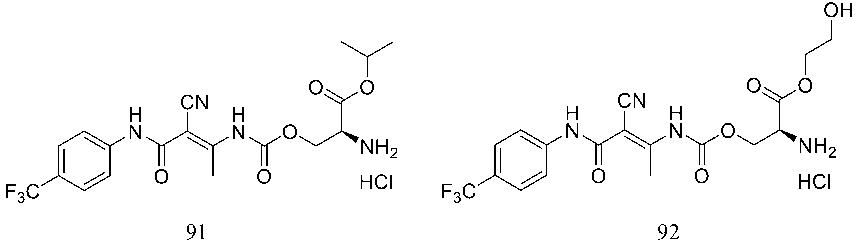

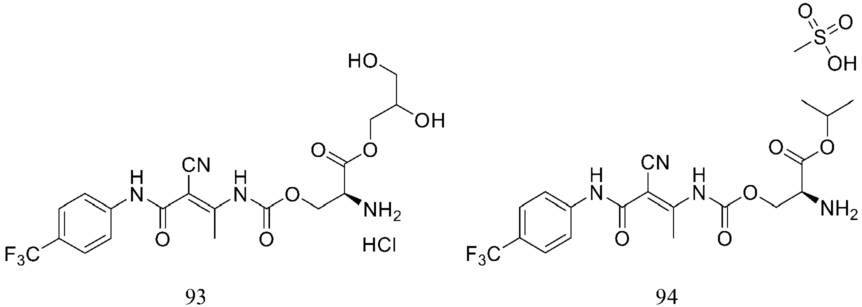

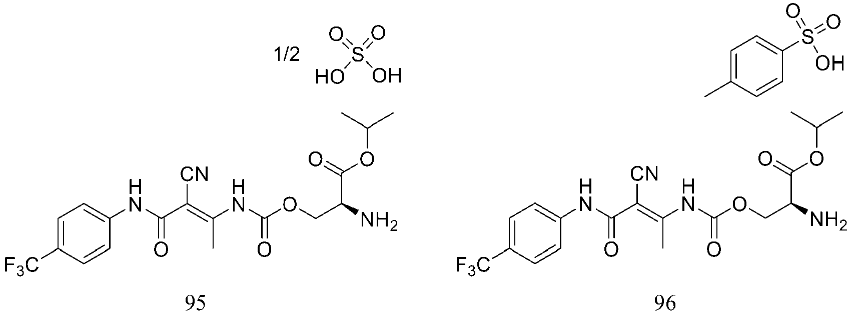

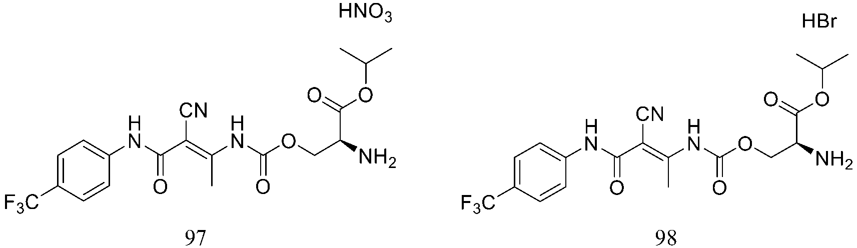

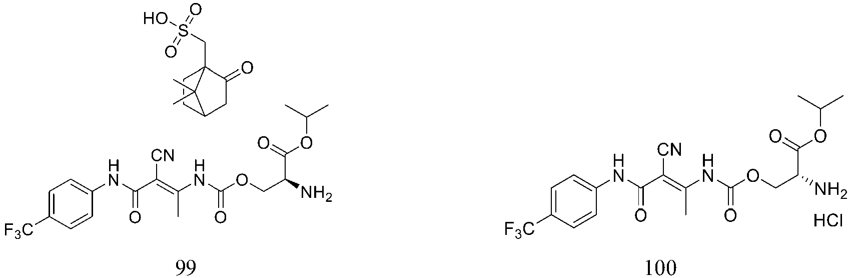

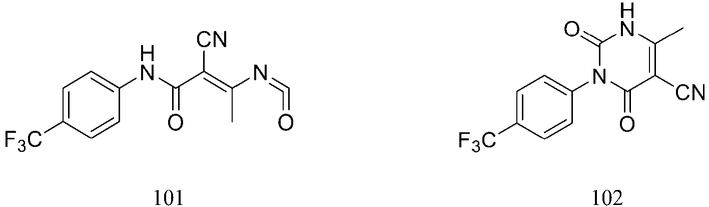

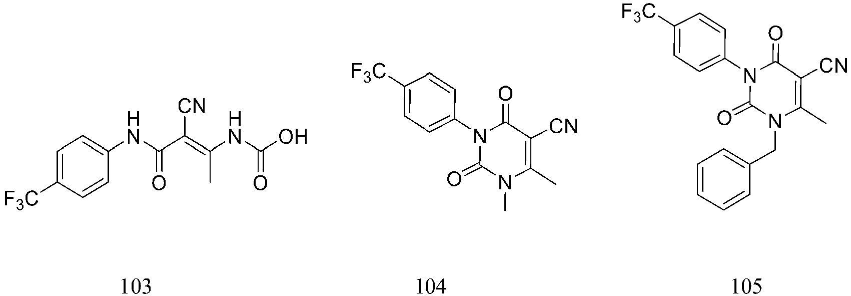

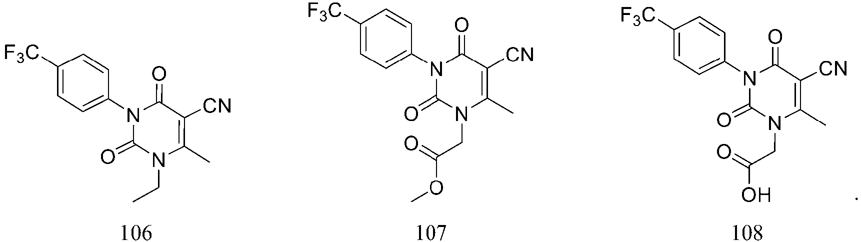

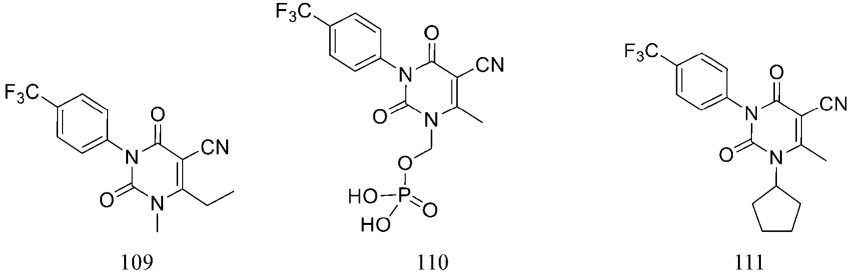

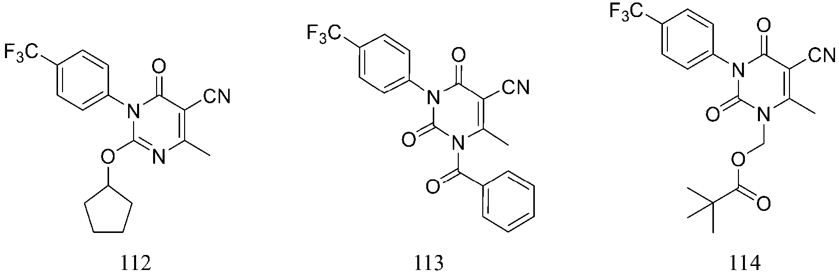

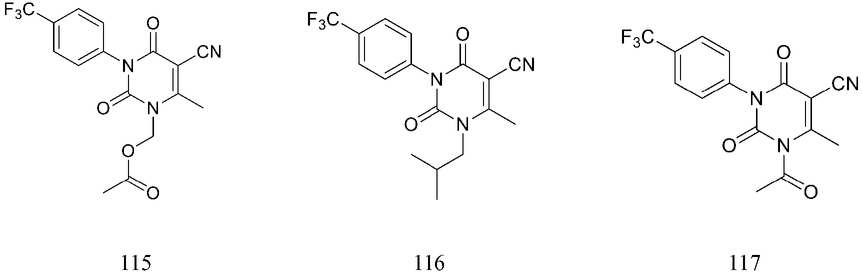

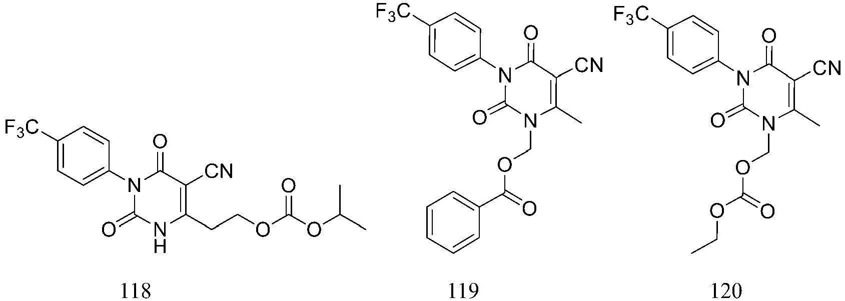

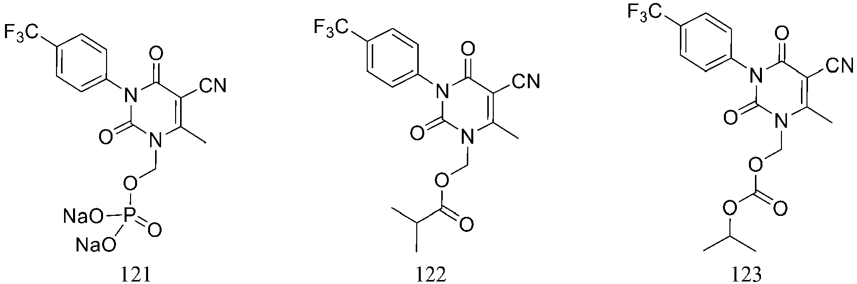

In another aspect, the present disclosure provides a method for preparing a compound of Formula (I), or an optical isomer thereof, or a pharmaceutically acceptable salt thereof, or a solvate thereof (e.g., a hydrate), or an inclusion complex thereof, or a racemate thereof, or a co-crystal thereof, or an isotope-labeled compound thereof, or nitrogen oxides thereof, or a prodrug thereof, or an active metabolite thereof;
the method comprises: subjecting a compound of Formula (A) and a compound of Formula (B) to Step A to afford a compound of Formula (I):

### Step A:

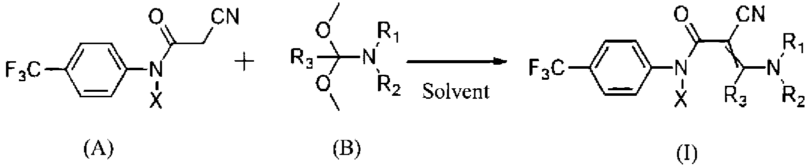

in Formula (A), X is as shown in Formula (I);
   in Formula (B), R₁, R₂ and R₃ are as shown in Formula (I);
optionally, the compound of Formula (I) is prepared from Formula (A) and Formula (B) via an addition-elimination reaction in a suitable solvent (such as xylene or toluene) at a temperature ranging from 50°C to 120°C.

Optionally, Formula (A) can be synthesized by, but not limited to, Method a;

### Method a:

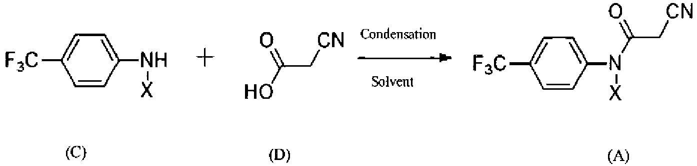

optionally, the compound of Formula (A) is prepared by condensation reaction of Formula (C) and Formula (D) catalyzed by a condensing agent (such as EDCI, HATU, HBTU, PyBOP, etc.), in the presence or absence of a base (such as pyridine, TEA, DIPEA, DBU, DBN, etc.), in a suitable solvent (such as THF, DCM, DCE, ACN, DMF, DMAC, etc.) at a temperature ranging from -10°C to 50°C.

Optionally, Formula (B) can be synthesized by, but not limited to, Method b;

### Method b:

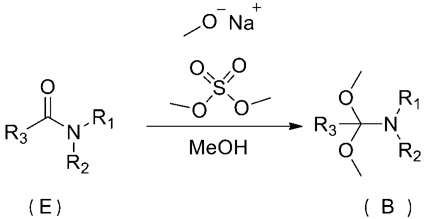

optionally, the compound of Formula (B) is prepared from Formula (E) via addition followed by methylation in the presence of sodium methoxide and dimethyl sulfate, with DMSO as solvent, at a temperature ranging from -10°C to 30°C.

Optionally, the method further comprises: obtaining the compound of Formula (I) by subjecting the compound of Formula (F) and the compound of Formula (G) to Step B when R₁ and R₂ in Formula (I) are the same or different, and are individually and independently H, alkyl, -alkylene-C(=O)O-alkyl, optionally substituted monosaccharide group, or when R₁ and R₂ are interconnected and form, together with nitrogen atoms to which they are attached, an optionally substituted aliphatic heterocyclyl group:

### Step B:

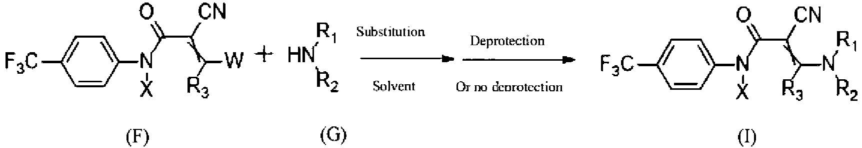

in Formula (F), X and R₃ are as defined in Formula (I), and W is dimethylamino, pyrrolyl, piperidinyl, or morpholinyl; in Formula (G), R₁ and R₂ are as defined in Formula (I).

Optionally, the compound of Formula (I) is prepared by substitution reaction of Formula (F) and Formula (G) catalyzed by a base (such as pyridine, TEA, DIPEA, DBU, DBN, etc.), with or without deprotection, in a suitable solvent (such as DMF, DMAC, DMSO, THF, DCM, DCE, MeOH, EtOH, dioxane, etc.) at a temperature ranging from 0°C to 100°C.

The method further comprises: obtaining the compound of Formula (I) by subjecting the compound of Formula (H) and the compound of Formula (J) to Step C when R₁ and R₂ in Formula (I) are different, and are individually and independently H, -S(=O)₂R₄₋₁, -C(=O)R₄₋₂, -C(=O)N(R₄₋₃)R₄₋₄, -A-Linker-R₄₋₆, -P(=O)(OR₄₋₇)OR₄₋₈:

### Step C:

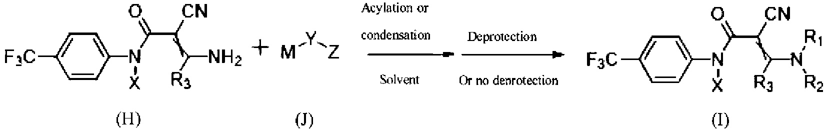

In Formula (H), X and R₃ are as defined in Formula (I); in Formula (J), M is halogen, hydroxy, imidazolyl, or p-nitrophenoxy, Y is alkyl, carbonyl, alkylene carbonyl, alkylene oxycarbonyl, alkylene oxyphosphoryl, sulfonyl, or phosphoryl; or M and Y form an isocyanate group; Z is optionally substituted alkyl, optionally substituted alkyloxy, optionally substituted aryl, optionally substituted aryloxy, optionally substituted amino, optionally substituted alkenyl, or optionally substituted alkylthio.

When M is halogen, and p-nitrophenoxy, imidazolyl, or M and Y form an isocyanate group, the compound of Formula (I) is optionally prepared by acylation reaction of Formula (H) and Formula (J) catalyzed by a base (such as pyridine, TEA, DIPEA, DBU, DBN, sodium hydride, etc.), with or without deprotection, in a suitable solvent (such as THF, DCM, DCE, ACN, DMF, DMAC, etc.) at a temperature ranging from -10°C to 30°C.

When M is hydroxy, the compound of Formula (I) is optionally prepared by condensation reaction of Formula (H) and Formula (J) catalyzed by a condensing agent (such as EDCI, HATU, HBTU, PyBOP, etc.), with or without deprotection, in a base (such as pyridine, TEA, DIPEA, DMAP, DBU, DBN, sodium hydride, etc.) and a suitable solvent (such as THF, DCM, DCE, ACN, DMF, DMAC, etc.) at a temperature ranging from -10°C to 50°C.

The method further comprises: when X and R₁ in Formula (I) jointly form the following cyclic amide structure

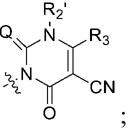

R₂' is as defined in Formula (I), and Formula (I) can be afforded by intramolecular cyclization of Formula (H) and reaction with the compound of Formula (J) via Step D:

### Step D:

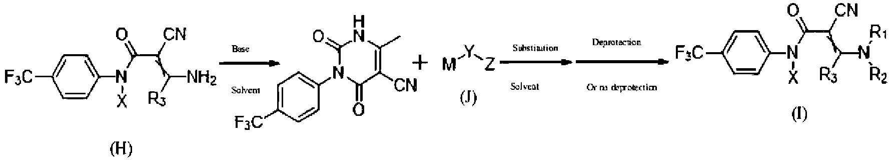

when R₂' is H, Q is O, and R₃ is CH₃; M is halogen (preferably, iodides or bromides), Y is alkyl, carbonyl, alkylene carbonyl, alkylene oxycarbonyl, alkylene oxyphosphoryl, or phosphoryl; Z is optionally substituted alkyl, C₃-C₇ monocyclic cycloalkyl, optionally substituted alkyloxy, optionally substituted aryl, or optionally substituted aryloxy.

Optionally, Formula (H) is capable of intramolecular cyclization by reacting with carbonyldiimidazole, or 4-nitrophenyl chloromethyl carbonate, or triphosgene under catalysis of a base (such as potassium carbonate, cesium carbonate, pyridine, TEA, DIPEA, DBU, DBN, NaHCO₃, sodium hydroxide, sodium hydride, etc.) in a suitable solvent (such as THF, DCM, DCE, ACN, DMF, acetone, DMAC, etc.) at a temperature ranging from -10°C to 60°C, and then the compound of Formula (I) is prepared by substitution reaction with Formula (J) under catalysis of a base (such as potassium carbonate, cesium carbonate, pyridine, TEA, DIPEA, DBU, DBN, sodium hydride, etc.), with or without deprotection, in a suitable solvent (such as THF, DCM, DCE, ACN, DMF, DMAC, etc.) at a temperature ranging from -10°C to 80°C.

The pharmaceutically acceptable salts are inorganic acid salts or organic acid salts, optionally selected from hydrochloric acid, sulfuric acid, phosphoric acid, nitric acid, trifluoroacetic acid, methanesulfonic acid, ethanesulfonic acid, tartaric acid, formic acid, acetic acid, salicylic acid, citric acid, succinic acid, fumaric acid, maleic acid, benzoic acid, hydrobromic acid, hydroiodic acid, benzenesulfonic acid, p-toluenesulfonic acid, and camphorsulfonic acid.

In another aspect, the present disclosure provides a pharmaceutical composition comprising a compound of the afore-mentioned Formula (I), or an optical isomer thereof, or a pharmaceutically acceptable salt thereof, or a solvate thereof (e.g., a hydrate), or an inclusion complex thereof, or a racemate thereof, or a co-crystal thereof, or an isotope-labeled compound thereof, or nitrogen oxides thereof, or a prodrug thereof, or an active metabolite thereof, and a pharmaceutically acceptable excipient.

Optionally, the pharmaceutically acceptable excipient is selected from: fillers, disintegrants, lubricants, glidants, effervescent agents, flavoring agents, preservatives, solubilizers, cosolvents, antioxidants, photodegradation inhibitors, pH regulators, emulsifiers, bacteriostatic preservatives, local anesthetics, complexing agents, non-aqueous solvents, coating materials, or other excipients.

Optionally, as for the pharmaceutically acceptable excipients: fillers thereof include a combination of one or more of lactose, mannitol, and calcium carbonate; the binders include a combination of one or more of sucrose, starch, povidone, and sodium carboxymethylcellulose; the disintegrants include a combination of one or more of starch, crospovidone, croscarmellose sodium, and effervescent disintegrants; non-aqueous solvents include a combination of one or more of soybean oil, castor oil, and peanut oil; solubilizers include a combination of one or more of Tween 80, Tween 60, and poloxamer 68; and cosolvents include a combination of one or more of sodium benzoate, sodium salicylate, and sodium p-aminobenzoate.

Optionally, administration routes of the pharmaceutical compositions include: oral administration (e.g., administration through mouth cavity), sublingual administration, parenteral administration (e.g., intramuscular, intravenous, or subcutaneous administration), rectal administration (e.g., via suppositories or lotions), transdermal administration (electroporation, transdermal formulations such as creams, gels, liniments, and transdermal patches), or administration by inhalation (e.g., aerosols), in solid, liquid, or gaseous dosage forms including tablets and suspensions. Administration may be carried out under continuous treatment, in a single unit dosage form, or as an optional single-dose treatment. The therapeutic composition may also be in the form of an oily emulsion or dispersion incorporating a lipophilic salt such as pamoate, or in the form of a biodegradable sustained-release composition for subcutaneous or intramuscular administration.

Optionally, the pharmaceutical composition can be formulated as a solid oral preparation, a liquid oral preparation, an injection, or a transdermal preparation. The solid and liquid oral preparations include: tablets, dispersible tablets, sugar-coated tablets, granules, dry powders, capsules, syrups, and solutions. The injection includes small-volume injections, large-volume infusions, lyophilized powder injections, etc. The transdermal preparation includes ointments, plasters, liniments, aerosols, conventional patches, adhesive dispersion-type patches, peripheral adhesive matrix-type patches, reservoir-type patches, cataplasms, etc.

In another aspect, the present disclosure provides the use of a compound of Formula (I), or an optical isomer thereof, or a pharmaceutically acceptable salt thereof, or a solvate thereof (e.g., a hydrate), or an inclusion complex thereof, or a racemate thereof, or a co-crystal thereof, or an isotope-labeled compound thereof, or nitrogen oxides thereof, or a prodrug thereof, or an active metabolite thereof, or the pharmaceutical composition, in the preparation of a medicament for the prophylaxis and/or treatment of inflammation, pain, multiple sclerosis, neuromyelitis optica, autoimmune diseases, or cancer.

Optionally, the inflammation is selected from rheumatoid arthritis, dermatitis, etc.; optionally, the pain is selected from mild to moderate pain, etc.; the multiple sclerosis (MS) is selected from clinically isolated syndrome (CIS) MS, relapsing-remitting MS, primary progressive MS, secondary progressive MS, etc.; the neuromyelitis optica (NMO) is selected from monophasic NMO, relapsing NMO, progressive NMO, etc.; the autoimmune disease is selected from systemic lupus erythematosus, psoriasis, anti-transplant rejection, etc.; the cancer is selected from renal cancer, prostate cancer, and lung cancer. Optionally, the cancer cells include human renal cancer cells 786-O, human prostate cancer cells LNCaP, PC3, DU145, human large cell lung cancer cells H460, and human non-small cell lung cancer A549.

Unless otherwise noted, the term "treatment" herein refers to alleviating or reducing the severity of symptoms associated with the disease or condition being treated, such as inflammation, pain, multiple sclerosis, neuromyelitis optica, etc. The term "prophylaxis" includes inhibiting symptoms of a specified disease or condition, such as inflammation, pain, multiple sclerosis, neuromyelitis optica, autoimmune diseases, etc.

The compounds of the present disclosure may exist in the form of isotope tracing or enrichment, and contain one or more atoms whose atomic weights or mass numbers differ from those of the most abundant atoms found in nature. Isotopes may be radioactive or non-radioactive. Isotopes of atoms such as hydrogen, carbon, phosphorus, sulfur, fluorine, chlorine, and iodine include, but are not limited to, ²H, ³H, ¹³C, ¹⁴C, ¹⁵N, ¹⁸O, ³²P, ³⁵S, ¹⁸F, ³⁶Cl and ¹²⁵I. Compounds containing these and/or other isotopes of other atoms are within the scope of the present disclosure.

The term "optical isomer" herein refers to substances having identical molecular structures and similar physicochemical properties but differing in optical rotation, including mixtures of optical isomers in any ratio. The compound of Formula (I) may contain one or more asymmetric carbon atoms, and may exist in a form of optically pure enantiomers, e.g., enantiomeric mixtures of racemates, optically pure diastereomers, diastereomeric mixtures, diastereomeric racemates, or mixtures of diastereomeric racemates. Optically active forms may be obtained, for example, by resolution of racemates, by asymmetric synthesis, or by asymmetric chromatography (chromatography using chiral adsorbents or eluents). The present disclosure includes all such forms.

The term "solvate" herein refers to a compound that further binds a stoichiometric or non-stoichiometric amount of solvent through non-covalent intermolecular forces. For example, when the solvent is water, the solvate is a hydrate.

### Abbreviations

EDCI: 1-ethyl-3-(3-dimethylaminopropyl)carbodiimide
HATU: 2-(7-azabenzotriazol-1-yl)-N,N,N',N'-tetramethyluronium hexafluorophosphate
HBTU: O-(benzotriazol-1-yl)-N,N,N',N'-tetramethyluronium hexafluorophosphate
TEA: triethylamine
DIPEA: N,N-diisopropylethylamine
DBU: 1,8-diazabicyclo[5.4.0]undec-7-ene
DBN: 1,5-diazabicyclo[4.3.0]non-5-ene
THF: tetrahydrofuran
TFA: trifluoroacetic acid
DCM: dichloromethane
DCE: dichloroethane
ACN: acetonitrile
DMF: N,N-dimethylformamide
DMAC: N,N-dimethylacetamide
DMSO: dimethyl sulfoxide
PyBOP: benzotriazol-1-yl-oxytripyrrolidinophosphonium hexafluorophosphate
MTBE: methyl tert-butyl ether
DMAP: 4-dimethylaminopyridine

### Description of Drawings

Fig. 1 is the score graph of neurological function of mice in Test Example 4;
Fig. 2 is a spinal cord section diagram of mice in Test Example 4;
Fig. 3 is a diagram showing the expression level of the inflammatory factor IL-17A of mice in Test Example 4;
Fig. 4 is a diagram showing the expression level of the inflammatory factor IFN-γ of mice in Test Example 4;
Fig. 5 is a diagram showing the survival rate of rat splenic lymphocytes in Test Example 6.

### Embodiments

Unless otherwise defined, all technical and scientific terms used herein have the same meaning as commonly understood by one of ordinary skill in the art. If a term used herein has multiple definitions, the definitions in this section shall prevail unless otherwise stated.

The examples of the present disclosure are described in detail below; however, the examples provided do not limit the present disclosure in any way.

### Examples

### General Synthetic Method 1:

The compound of Formula (F) (1.0 eq.) and the compound of Formula (G) (1.0 eq.) were dispersed in ethanol, with or without DIPEA (1.0 eq.), and the mixture was heated to reflux reaction; after the reaction, the mixture was cooled, a solid was precipitated and then was collected by filtration, and the compound of Formula (I) was thus prepared with or without deprotection via acid treatment, base treatment or palladium-carbon reduction.

### Example 1: Preparation of Compound 1

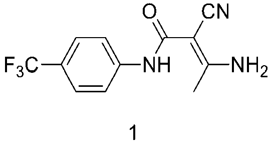

Compound 1 was prepared according to General Synthetic Method 1, wherein a white solid was afforded with a yield of 78.8%.

¹H NMR (400 MHz, DMSO-*d*₆) δ 9.58 (s, 1H), 9.35 (s, 1H), 8.71 (s, 1H), 7.81 (d, *J=* 8.6 Hz, 2H), 7.62 (d, *J =* 8.7 Hz, 2H), 2.19 (s, 3H).

### Example 2: Preparation of Compound 2

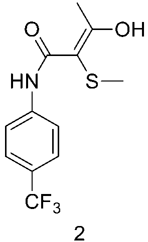

### Synthetic Method:

Compound TLFA-3 (25 mg, 0.08 mmol) and anhydrous potassium carbonate (5 mg, 0.04 mmol) were weighed and put into a reaction flask of 25 mL, and then methanol (2 mL) was added; the reaction was carried out at room temperature; after the reaction, the reaction mixture was washed twice with ethyl acetate (50 mL) and saturated NaCl (25 mL), and the organic phase was dried with anhydrous sodium; the solvent was removed via decompression and concentration, the residue was purified with petroleum ether/ethyl acetate=5/1, and 10 mg of a pale-yellow solid was thus afforded with a yield of 45%.

¹H NMR (400 MHz, DMSO-*d*₆) δ 10.18 (s, 1H), 7.97 (d, *J* = 8.5 Hz, 2H), 7.68 (d, *J=* 8.6 Hz, 2H), 6.91 (s, 1H), 3.68 (s, 3H), 1.56 (s, 3H).

### Example 3: Preparation of Compound 3

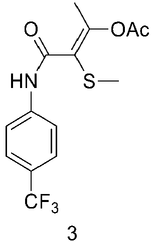

### Synthetic Method:

Compounds including p-(trifluoromethyl)aniline (161 mg, 1 mmol), 4,4-bis(methylthio)but-3-en-2-one (162 mg, 1 mmol) and 1,4-dioxane (3 mL) were weighed, and then the reaction mixture was slowly warmed from room temperature to 150°C and was stirred overnight; the reaction mixture was extracted with ethyl acetate (100 mL) and saturated ammonium chloride solution (50 mL); the organic phrase was washed with water (50 mL) once and with brine once, and was dried with anhydrous sodium sulfate; the solvent was removed via decompression and concentration, purification was performed using petroleum ether/ethyl acetate=10/1, and 132 mg of pale-yellow, oily product was thus afforded with a yield of 48%.

The product (60 mg, 0.2 mmol) and PhI(OAc)₂ (105 mg, 0.3 mmol) were weighed and added to DCE (3 mL) in a reaction flask of 25 mL for reaction at room temperature; after the reaction, the mixture was washed twice with ethyl acetate (100 mL) and saturated brine (50 mL); the organic phase was dried with anhydrous sodium sulfate, the solvent was removed via decompression and concentration, purification was performed using petroleum ether:ethyl acetate=7:1, and 25 mg of pale-yellow, oily product was thus afforded with a yield of 34%.

¹H NMR (400 MHz, DMSO-*d*₆) δ 10.44 (s, 1H), 7.85 (d, J = 8.5 Hz, 2H), 7.72 (d, J = 8.7 Hz, 2H), 2.35 (s, 3H), 2.26 (s, 3H), 2.08 (s, 3H).

### Example 4: Preparation of Compound 4

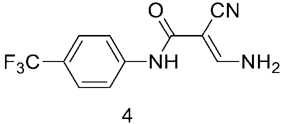

Compound 4 was prepared according to General Synthetic Method 1, wherein a yellow solid was afforded with a yield of 56.3%.

¹H NMR (400 MHz, DMSO-*d*₆) δ 9.47 (s, 1H), 9.03 (d, *J =* 15.2 Hz, 1H), 8.60 (s, 1H), 7.84 (d, *J =* 8.5 Hz, 2H), 7.65 (dd, *J =* 14.6, 8.7 Hz, 3H).

### Example 5: Preparation of Compound 5

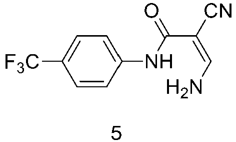

Compound 5 was prepared according to General Synthetic Method 1, wherein a white solid was afforded with a yield of 66.6%.

¹H NMR (400 MHz, DMSO-*d*₆) δ 9.61 (s, 1H), 8.30 (s, 1H), 8.03 (d, *J* = 7.3 Hz, 2H), 7.82 (d, *J=* 8.5 Hz, 2H), 7.62 (d, *J* = 8.6 Hz, 2H).

### General Synthetic Method 2:

The compound of Formula (C) (1.0 eq.) and the compound of Formula (D) (1.1 eq.) were dispersed in DMF, and the mixture was cooled to 0°C; after EDAC (1.2 eq.) was added slowly, the mixture was warmed to room temperature for reaction; after the reaction, water was added to precipitate a solid, which was collected by filtration to afford a white solid, i.e. the compound of Formula (A).

The compound of Formula (E) (1.0 eq.) reacted with dimethyl sulfate (1.0 eq.) at 90°C for 1 hour; then the mixture was cooled to -5°C, and sodium methoxide (1.5 eq.) in methanol was added dropwise slowly; after reacting for 1 hour, MTBE was added, the mixture was filtered, and the filtrate was concentrated to afford a yellow oily product, i.e. the compound of Formula (B).

The compound of Formula (A) (1.0 eq.) and the compound of Formula (B) (1.1 eq.) were dispersed in xylene, and the mixture was warmed to 100°C for reaction.; after the reaction, the mixture was cooled, petroleum ether was added, and a solid was precipitate, which was filtered and dried to afford a white or off-white solid. i.e. the compound of Formula (J).

### Example 6: Preparation of Compound 6

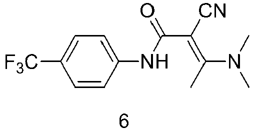

Compound 6 was prepared according to General Synthetic Method 2, wherein a white solid was afforded with a yield of 84.1%.

¹H NMR (400 MHz, DMSO-*d*₆) δ 9.94 (s, 1H), 7.78 (d, J= 8.5 Hz, 2H), 7.63 (d, J= 8.5 Hz, 2H), 3.04 (s, 6H), 2.32 (s, 3H).

### General Synthetic Method 3:

The compound of Formula (H) (1.0 eq.) was dispersed in DMAC, and then the mixture was cooled to 0°C; sodium hydride (1.5 eq.) or DBU (1.5 eq.) was added, and the mixture was stirred for half an hour; the solution of Formula (J) (1.5 eq.) in DMF was added slowly, and the mixture was warmed to room temperature for reaction; after the reaction, EA was added, and then the mixture was washed with saturated ammonium chloride solution three times and with saturated sodium chloride solution once, was dried with anhydrous sodium sulfate, and then was filtered and concentrated; a product obtained by column chromatography was then subjected to deprotection or no deprotection via acid treatment, base treatment or palladium-carbon reduction to afford the compound of Formula (I).

Alternatively, the compound of Formula (H) (1.0 eq.), the compound of Formula (J) (2 to 3 eq.) and HATU (2 to 3 eq.) were dispersed in DMF, DBU (2 to 3 eq.) was added, and then the mixture was warmed to 45°C for reaction; after the reaction, EA was added, and then the mixture was washed with saturated ammonium chloride solution three times and with saturated sodium chloride solution once, was dried with anhydrous sodium sulfate, and then was filtered and concentrated; a product obtained by column chromatography was then subjected to deprotection or no deprotection via acid treatment, base treatment or palladium-carbon reduction to afford the compound of Formula (I).

### Example 7: Preparation of Compound 7

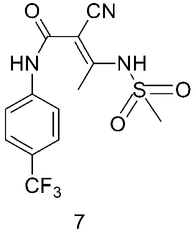

Compound 7 was prepared according to General Synthetic Method 3, wherein a white solid was afforded with a yield of 70.0%.

¹H NMR (400 MHz, DMSO-*d*₆) δ 12.33 (s, 1H), 7.71 (d, *J* = 8.5 Hz, 2H), 7.61 (d, *J=* 8.6 Hz, 2H), 3.03 (s, 3H), 2.44 (s, 3H).

### Example 8: Preparation of Compound 8

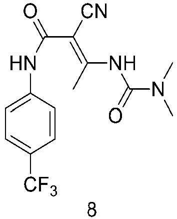

Compound 8 was prepared according to General Synthetic Method 3, wherein a yellow solid was afforded with a yield of 48.1%.

¹H NMR (400 MHz, DMSO-*d*₆) δ 12.55 (s, 1H), 10.01 (s, 1H), 7.80 (d, *J =* 8.6 Hz, 2H), 7.70 (d, *J=* 8.7 Hz, 2H), 2.96 (s, 6H), 2.66 (s, 3H).

### Example 9: Preparation of Compound 9

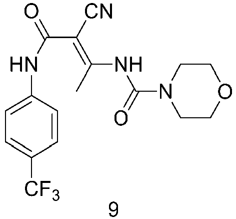

Compound 9 was prepared according to General Synthetic Method 3, wherein a white solid was afforded with a yield of 56.3%.

¹H NMR (400 MHz, DMSO-*d*₆) δ 12.49 (s, 1H), 10.06 (s, 1H), 7.79 (d, *J =* 8.6 Hz, 2H), 7.71 (d, *J=* 8.7 Hz, 2H), 3.71 - 3.55 (m, 4H), 3.47 - 3.38 (m, 4H), 2.65 (s, 3H).

### Example 10: Preparation of Compound 10

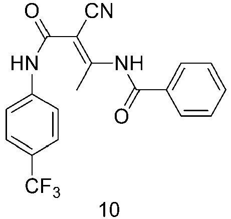

Compound 10 was prepared according to General Synthetic Method 3, wherein a white solid was afforded with a yield of 56.0%.

¹H NMR (400 MHz, DMSO-*d*₆) δ 13.43 (s, 1H), 10.31 (s, 1H), 7.92 (d, *J =* 7.3 Hz, 2H), 7.83 (d, *J =* 8.5 Hz, 2H), 7.72 (dd, *J =* 13.8, 8.0 Hz, 3H), 7.62 (t, *J =* 7.6 Hz, 2H), 2.83 (s, 3H).

### Example 11: Preparation of Compound 11

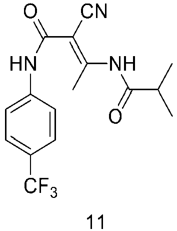

Compound 11 was prepared according to General Synthetic Method 3, wherein a white solid was afforded with a yield of 55.0%.

¹H NMR (400 MHz, DMSO-*d*₆) δ 12.40 (s, 1H), 10.21 (s, 1H), 7.82 (d, *J =* 8.6 Hz, 2H), 7.72 (d, *J =* 8.7 Hz, 2H), 2.66 (s, 3H), 2.65 - 2.59 (m, 1H), 1.12 (d, *J =* 6.9 Hz, 6H).

### Example 12: Preparation of Compound 12

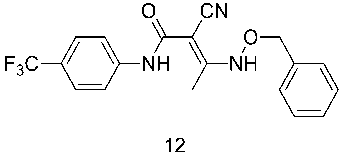

Compound 12 was prepared according to General Synthetic Method 1, wherein a white solid was afforded with a yield of 66.6%.

¹H NMR (400 MHz, DMSO-*d*₆) δ 11.14 (s, 1H), 10.77 (s, 1H), 7.83 - 7.71 (m, 4H), 7.49 - 7.22 (m, 5H), 5.00 (d, *J* = 10.1 Hz, 2H), 1.95 (s, 3H).

### Example 13: Preparation of Compound 13

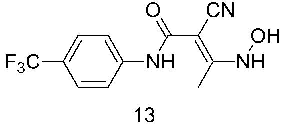

Compound 13 was prepared according to General Synthetic Method 1, wherein a white solid was afforded with a yield of 46.0%.

¹H NMR (400 MHz, DMSO-*d*₆) δ 9.17 (s, 1H), 7.82 (d, *J* = 8.5 Hz, 2H), 7.67 (d, *J* = 9.3 Hz, 4H), 2.34 (s, 3H).

### Example 14: Preparation of Compound 14

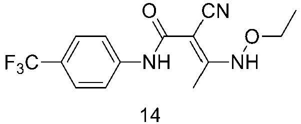

Compound 14 was prepared according to General Synthetic Method 1, wherein a white solid was afforded with a yield of 46.0%.

¹H NMR (400 MHz, DMSO-*d*₆) δ 11.16 (s, 1H), 7.80 *(d, J=* 8.8 Hz, 3H), 7.74 (d,*J* = 8.7 Hz, 2H), 4.16 - 4.07 (m, 2H), 1.92 (s, 3H), 1.22 - 1.19 (m, 3H).

### Example 15: Preparation of Compound 15

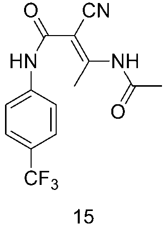

Compound 15 was prepared according to General Synthetic Method 3, wherein a white solid was afforded with a yield of 20.0%.

¹H NMR (400 MHz, DMSO-*d*₆) δ 12.19 (s, 1H), 10.22 (s, 1H), 7.82 (d, *J =* 8.6 Hz, 2H), 7.71 (d, *J =* 8.7 Hz, 2H), 2.64 (s, 3H), 2.17 (s, 3H).

### Example 16: Preparation of Compound 16

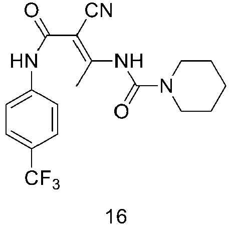

Compound 16 was prepared according to General Synthetic Method 3, wherein a white solid was afforded with a yield of 49.3%.

¹H NMR (400 MHz, DMSO-*d*₆) δ 12.52 (s, 1H), 10.02 (s, 1H), 7.79 (d, *J =* 8.5 Hz, 2H), 7.71 (d, *J=* 8.8 Hz, 2H), 3.47 - 3.39 (m, 4H), 2.63 (s, 3H), 1.54 (d, *J=* 22.8 Hz, 6H).

### Example 17: Preparation of Compound 17

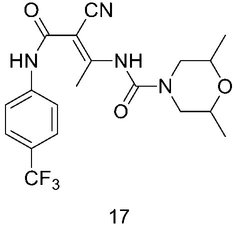

Compound 17 was prepared according to General Synthetic Method 3, wherein a white solid was afforded with a yield of 26.0%.

¹H NMR (400 MHz, DMSO-*d*₆) δ 12.40 (s, 1H), 10.04 (s, 1H), 7.75 (dd, *J =* 35.6, 8.6 Hz, 4H), 3.83 (s, 2H), 3.57 - 3.45 (m, 2H), 2.64 (d, *J* = 16.9 Hz, 4H), 1.10 (d, *J* = 6.2 Hz, 6H).

### Example 18: Preparation of Compound 18

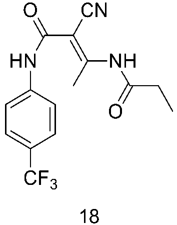

Compound 18 was prepared according to General Synthetic Method 3, wherein a white solid was afforded with a yield of 43.0%.

¹H NMR (400 MHz, DMSO-*d*₆) δ 12.28 (s, 1H), 10.20 (s, 1H), 7.82 (d, *J =* 8.6 Hz, 2H), 7.71 (d, *J =* 8.7 Hz, 2H), 2.66 (s, 3H), 2.46 (s, 2H), 1.04 (t, *J =* 7.4 Hz, 3H).

### Example 19: Preparation of Compound 19

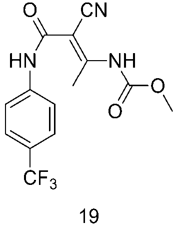

Compound 19 was prepared according to General Synthetic Method 3, wherein a white solid was afforded with a yield of 33.0%.

¹H NMR (400 MHz, DMSO-*d*₆) δ 12.20 (s, 1H), 10.16 (s, 1H), 7.81 (d, *J =* 8.5 Hz, 2H), 7.70 (d, *J =* 8.7 Hz, 2H), 3.73 (s, 3H), 2.65 (s, 3H).

### Example 20: Preparation of Compound 20

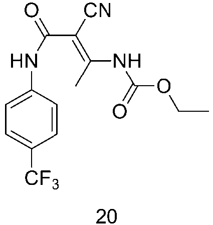

Compound 20 was prepared according to General Synthetic Method 3, wherein a white solid was afforded with a yield of 20.0%.

¹H NMR (400 MHz, DMSO-*d*₆) δ 12.18 (s, 1H), 10.15 (s, 1H), 7.82 (d, *J =* 8.5 Hz, 2H), 7.70 (d, *J =* 8.7 Hz, 2H), 4.17 (d, *J =* 7.1 Hz, 2H), 2.65 (s, 3H), 1.24 (t, *J=* 7.1 Hz, 3H).

### Example 21: Preparation of Compound 21

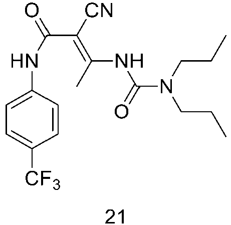

Compound 21 was prepared according to General Synthetic Method 3, wherein a white solid was afforded with a yield of 19.0%.

¹H NMR (400 MHz, DMSO-*d*₆) δ 12.63 (s, 1H), 10.00 (s, 1H), 7.82 (d, *J =* 8.6 Hz, 2H), 7.72 (t, *J =* 9.1 Hz, 2H), 3.30 - 3.20 (m, 4H), 2.66 (s, 3H), 1.55 (s, 4H), 0.87 (t, *J =* 6.8 Hz, 6H).

### Example 22: Preparation of Compound 22

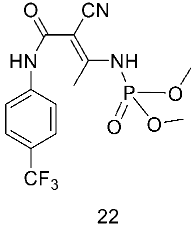

Compound 22 was prepared according to General Synthetic Method 3, wherein a pale-yellow solid was afforded with a yield of 36.0%.

¹H NMR (400 MHz, DMSO-*d*₆) δ 11.63 (d, *J =* 10.9 Hz, 1H), 10.07 (s, 1H), 7.82 (d, *J* = 8.5 Hz, 2H), 7.70 (d, *J =* 8.7 Hz, 2H), 3.79 (s, 3H), 3.76 (s, 3H), 2.45 (s, 3H).

### Example 23: Preparation of Compound 23

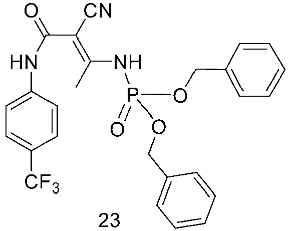

Compound 23 was prepared according to General Synthetic Method 3, wherein a pale-yellow solid was afforded with a yield of 83.0%.

¹H NMR (400 MHz, DMSO-*d*₆) δ 11.63 (d, *J =* 11.3 Hz, 1H), 10.01 (s, 1H), 7.81 (d, *J* = 8.6 Hz, 2H), 7.71 (d, *J =* 8.7 Hz, 2H), 7.47 - 7.32 (m, 10H), 5.15 (dd, *J =* 9.2, 1.7 Hz, 4H), 2.33 (s, 3H).

### Example 24: Preparation of Compound 24

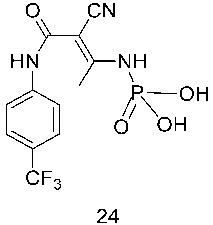

Compound 24 was prepared according to General Synthetic Method 3, wherein a pale-yellow solid was afforded with a yield of 50.0%.

¹H NMR (400 MHz, DMSO-*d*₆) δ 11.22 (d, *J= 9.9* Hz, 1H), 9.77 (s, 1H), 7.81 (d, *J* = 8.6 Hz, 2H), 7.67 (d, *J =* 8.7 Hz, 2H), 2.47 (s, 3H).

### Example 25: Preparation of Compound 25

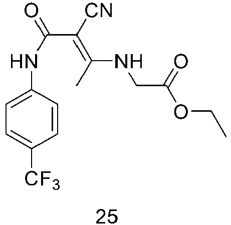

Compound 25 was prepared according to General Synthetic Method 1, wherein a white solid was afforded with a yield of 25.0%.

¹H NMR (400 MHz, DMSO-*d*₆) δ 10.75 (s, 1H), 9.46 (s, 1H), 7.81 (d, *J =* 8.6 Hz, 2H), 7.64 (d, *J =* 8.7 Hz, 2H), 4.37 (d, *J =* 5.6 Hz, 2H), 4.18 (d, *J =* 7.1 Hz, 2H), 2.24 (s, 3H), 1.23 (t, *J=* 7.1 Hz, 3H).

### Example 26: Preparation of Compound 26

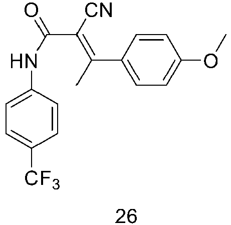

### Synthetic Method:

The compound of Formula (A) (1.0 eq.) and p-methoxyacetophenone (1.0 eq.) were dispersed in ethanol, and p-toluenesulfonic acid (1.0 eq.) was added; the mixture was heated to reflux; after the reaction, an off-white solid product was afforded with a yield of 7.8%.

¹H NMR (400 MHz, DMSO-*d*₆) δ 10.74 (s, 1H), 7.64 (d, *J =* 11.8 Hz, 4H), 7.40 (s, 2H), 6.94 (s, 2H), 3.76 (s, 3H).

### Example 27: Preparation of Compound 27

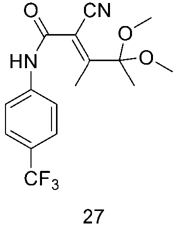

The synthetic method is the same as that in Example 26, wherein an off-white solid was afforded with a yield of 5.9%.

¹H NMR (400 MHz, DMSO-*d*₆) δ 11.12 (s, 1H), 8.03 - 7.54 (m, 4H), 3.18 (d, *J =* 7.3 Hz, 6H), 1.95 (s, 3H), 1.47 (s, 3H).

### Example 28: Preparation of Compound 28

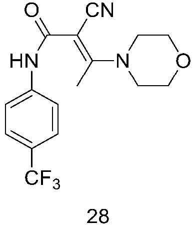

Compound 28 was prepared according to General Synthetic Method 1, wherein a white solid was afforded with a yield of 26.9%.

¹H NMR (400 MHz, DMSO-*d*₆) δ 10.03 (s, 1H), 7.78 (s, 2H), 7.65 (s, 2H), 3.66 (s, 4H), 3.34 (s, 4H), 2.34 (s, 3H).

### Example 29: Preparation of Compound 29

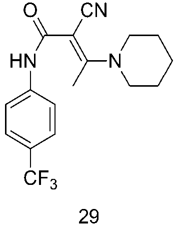

Compound 29 was prepared according to General Synthetic Method 1, wherein a white solid was afforded with a yield of 60.9%.

¹H NMR (400 MHz, DMSO-*d*₆) δ 9.89 (s, 1H), 7.78 (d, *J=* 8.6 Hz, 2H), 7.63 (d, *J =* 8.7 Hz, 2H), 3.38 (s, 4H), 2.32 (s, 3H), 1.60 (s, 6H).

### Example 30: Preparation of Compound 30

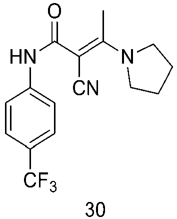

Compound 30 was prepared according to General Synthetic Method 1, wherein a yellow oily liquid was afforded with a yield of 63.3%.

¹H NMR (400 MHz, DMSO-*d*₆) δ 9.88 (d, *J* = 120.9 Hz, 1H), 7.79 (d, *J* = 8.5 Hz, 2H), 7.62 (d, *J =* 8.6 Hz, 2H), 3.64 (s, 4H), 2.33 (s, 3H), 1.87 (s, 4H).

### Example 31: Preparation of Compound 31

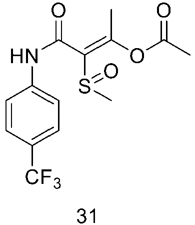

### Synthetic Method:

Compounds TLFA-3 (50 mg, 0.15 mmol) and meta-chloroperoxybenzoic acid (27 mg, 0.16 mmol) were weighed, and were added with dichloromethane (3 mL) for reaction at room temperature in a reaction flask of 25 mL; after the reaction, the reaction mixture was washed twice with ethyl acetate (50 mL) and with saturated brine (25 mL); the organic phase was dried with anhydrous sodium sulfate; the solvent was removed via decompression and concentration, purification was performed using petroleum ether:ethyl acetate=1:1, and 6 mg of pale-yellow solid was thus afforded with a yield of 11.5%.

¹H NMR (400 MHz, DMSO-*d*₆) δ 10.03 (s, 1H), 7.79 (d, *J=* 8.5 Hz, 2H), 7.62 (d, *J=* 8.6 Hz, 2H), 3.64 (s, 3H), 2.33 (s, 3H), 1.87 (s, 3H).

### Example 32: Preparation of Compound 32

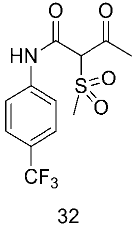

### Synthetic Method:

Compounds TLFA-3 (37 mg, 0.11 mmol) and potassium peroxymonosulfate (137 mg, 0.22 mmol) were weighed, and were added with acetone (0.6 mL) and water (0.2 mL) for reaction at room temperature in a reaction flask of 25 mL; after the reaction, the reaction mixture was washed twice with ethyl acetate (50 mL) and with saturated brine (25 mL); the organic phase was dried with anhydrous sodium sulfate; the solvent was removed via decompression and concentration, purification was performed using petroleum ether:ethyl acetate= 1:1, and 5 mg of pale-yellow solid was thus afforded with a yield of 12.5%.

¹H NMR (400 MHz, DMSO-*d*₆) δ 10.32 (s, 1H), 7.79 (d, *J* = 8.6 Hz, 2H), 7.66 (d, *J=* 8.7 Hz, 2H), 5.76 (s, 1H), 2.08 (s, 3H).

### Example 33: Preparation of Compound 33

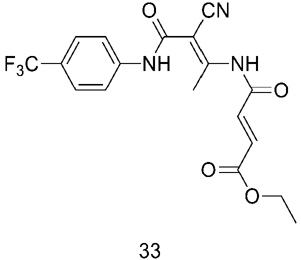

Compound 33 was prepared according to General Synthetic Method 3, wherein a white solid was afforded with a yield of 23.0%.

¹H NMR (400 MHz, DMSO-*d*₆) δ 12.21 (s, 1H), 10.40 (s, 1H), 7.82 (d, *J =* 8.5 Hz, 2H), 7.72 (d, *J =* 8.9 Hz, 2H), 7.04 (d, *J =* 15.5 Hz, 1H), 6.70 (d, *J* = 15.6 Hz, 1H), 4.27 - 4.18 (m, 2H), 2.65 (s, 3H), 1.25 (t, *J* = 7.1 Hz, 3H).

### Example 34: Preparation of Compound 34

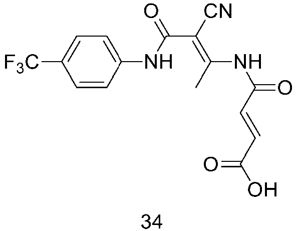

Compound 34 was prepared according to General Synthetic Method 3, wherein a white solid was afforded with a yield of 23.0%.

¹H NMR (400 MHz, DMSO-*d*₆) δ 8.52 (s, 1H), 8.01 (s, 1H), 6.95 (d, *J* = 10.3 Hz, 1H), 6.51 (d, *J* = 11.7 Hz, 1H), 6.32 (s, 1H), 6.20 (s, 1H), 6.00 (d, *J =* 8.1 Hz, 3H), 2.86 - 2.69 (m, 3H).

### Example 35: Preparation of Compound 35

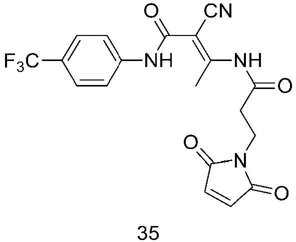

Compound 35 was prepared according to General Synthetic Method 3, wherein a white solid was afforded with a yield of 8.0%.

¹H NMR (400 MHz, DMSO-*d*₆) δ 12.14 (s, 1H), 10.23 (s, 1H), 7.79 (s, 2H), 7.69 (s, 2H), 7.01 (s, 2H), 3.67 (t, *J =* 6.9 Hz, 2H), 2.71 (t, *J =* 6.9 Hz, 2H), 2.59 (d, *J =* 12.1 Hz, 3H).

### Example 36: Preparation of Compound 36

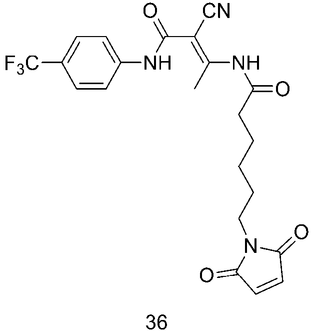

Compound 36 was prepared according to General Synthetic Method 3, wherein a white solid was afforded with a yield of 8.0%.

¹H NMR (400 MHz, DMSO-*d*₆) δ 12.22 (s, 1H), 10.20 (s, 1H), 7.82 (d, *J =* 8.6 Hz, 2H), 7.71 (d, *J =* 8.7 Hz, 2H), 7.00 (s, 2H), 2.65 (d, *J =* 6.5 Hz, 5H), 2.43 (d, *J =* 7.4 Hz, 2H), 1.59 - 1.44 (m, 4H), 1.23 (dd, *J =* 15.2, 8.7 Hz, 2H).

### Example 37: Preparation of Compound 37

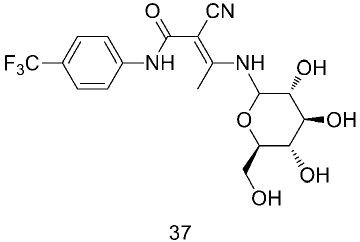

Compound 37 was prepared according to General Synthetic Method 1, wherein a yellow solid was afforded with a yield of 56.0%.

¹H NMR (400 MHz, DMSO-*d*₆) δ 10.85 (d, *J =* 7.5 Hz, 1H), 9.59 (s, 1H), 7.95 (s, 1H), 7.80 (d, *J =* 8.5 Hz, 2H), 7.65 (d, *J* = 8.7 Hz, 2H), 5.57 (d, *J* = 5.6 Hz, 1H), 5.12 (dd, *J* = 31.2, 5.3 Hz, 2H), 4.65 (dt, *J =* 12.1, 7.1 Hz, 2H), 3.71 - 3.54 (m, 2H), 3.50 - 3.41 (m, 1H), 3.26 (dd, *J=* 14.1, 5.7 Hz, 2H), 3.11 - 3.05 (m, 1H), 2.37 (d, *J* = 8.7 Hz, 3H).

### Example 38: Preparation of Compound 38

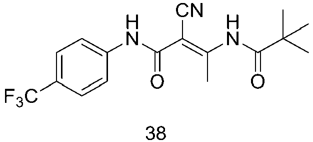

Compound 38 was prepared according to General Synthetic Method 3, wherein a white solid was afforded with a yield of 8.0%.

¹H NMR (400 MHz, DMSO-*d*₆) δ 12.83 (s, 1H), 10.20 (s, 1H), 7.81 (d, *J =* 8.6 Hz, 2H), 7.73 (d, *J* = 8.7 Hz, 2H), 2.70 (s, 3H), 1.21 (s, 9H).

### Example 39: Preparation of Compound 39

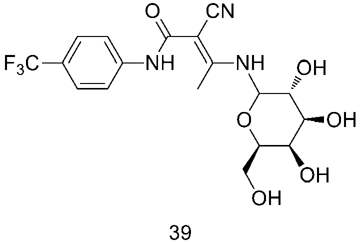

Compound 39 was prepared according to General Synthetic Method 1, wherein a pale-yellow solid was afforded with a yield of 62.0%.

¹H NMR (400 MHz, DMSO-*d*₆) δ 10.86 (d, *J* = 8.1 Hz, 1H), 9.58 (s, 1H), 7.81 (d, *J=* 8.4 Hz, 2H), 7.64 (d, *J =* 8.6 Hz, 2H), 5.38 (d, *J =* 5.3 Hz, 1H), 4.91 (d, *J =* 5.6 Hz, 1H), 4.71 - 4.60 (m, 2H), 4.56 (d, *J =* 5.0 Hz, 1H), 3.67 (s, 1H), 3.54 - 3.47 (m, 3H), 3.43 (d, *J =* 5.4 Hz, 1H), 3.39 (s, 1H), 2.37 (s, 3H).

### Example 40: Preparation of Compound 40

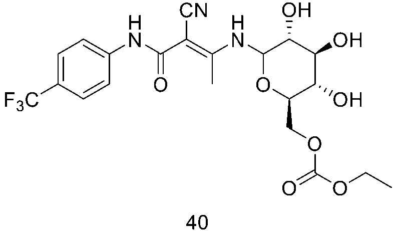

Compound 40 was prepared according to General Synthetic Method 1, wherein a pale-yellow solid was afforded with a yield of 33.0%.

¹H NMR (400 MHz, DMSO-*d*₆) δ 10.89 (s, 1H), 9.60 (s, 1H), 7.79 (d, *J* = 8.5 Hz, 2H), 7.63 (d, *J =* 8.4 Hz, 2H), 4.72 (s, 1H), 4.34 (d, *J =* 10.2 Hz, 1H), 4.16 - 4.08 (m, 2H), 3.61 - 3.52 (m, 2H), 3.26 (d, *J* = 8.8 Hz, 2H), 3.10 (dd, J = 8.9, 5.6 Hz, 2H), 2.33 (d, *J* = 1.7 Hz, 3H), 2.02 (s, 1H), 1.76 (s, 3H).

### Example 41: Preparation of Compound 41

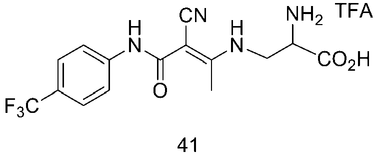

Compound 41 was prepared according to General Synthetic Method 1, wherein a white solid was afforded with a yield of 47.0%.

¹H NMR (400 MHz, DMSO-*d*₆) δ 10.70 (t, *J =* 6.4 Hz, 1H), 9.41 (s, 1H), 8.08 (s, 2H), 7.81 (d, *J= 8.5* Hz, 2H), 7.63 (d, *J* = 8.6 Hz, 2H), 3.83 - 3.60 (m, 3H), 2.30 (s, 3H).

### Example 42: Preparation of Compound 42

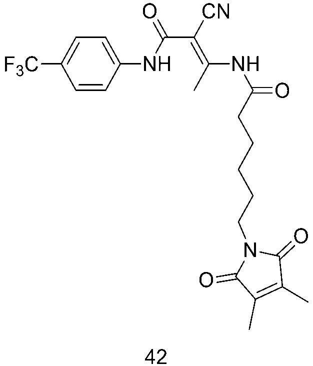

Compound 42 was prepared according to General Synthetic Method 3, wherein an off-white solid was afforded with a yield of 54.5%.

¹H NMR (400 MHz, DMSO-*d*₆) δ 12.23 (s, 1H), 10.20 (s, 1H), 7.82 (d, *J =* 8.5 Hz, 2H), 7.71 (d, *J =* 8.5 Hz, 2H), 2.64 (s, 3H), 2.42 (t, *J* = 7.3 Hz, 4H), 1.88 (s, 6H), 1.57 - 1.40 (m, 4H), 1.30 - 1.19 (m, 2H).

### Example 43: Preparation of Compound 43

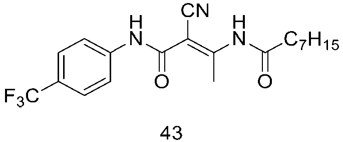

Compound 43 was prepared according to General Synthetic Method 3, wherein a white solid was afforded with a yield of 20.0%.

¹H NMR (400 MHz, DMSO-*d*₆) δ 12.22 (s, 1H), 10.20 (s, 1H), 7.81 (d, *J =* 8.6 Hz, 2H), 7.71 (d, *J* = 8.7 Hz, 2H), 2.65 (s, 3H), 2.42 (t, *J =* 7.4 Hz, 2H), 1.60 - 1.47 (m, 2H), 1.24 (s, 8H), 0.84 (t, *J* = 6.9 Hz, 3H).

### Example 44: Preparation of Compound 44

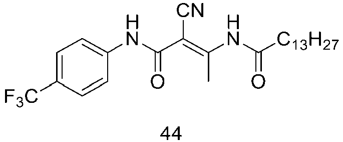

Compound 44 was prepared according to General Synthetic Method 3, wherein a white solid was afforded with a yield of 40.3%.

¹H NMR (400 MHz, DMSO-*d*₆) δ 12.23 (s, 1H), 10.20 (s, 1H), 7.82 (d, *J =* 8.6 Hz, 2H), 7.70 (d, *J =* 8.7 Hz, 2H), 2.65 (s, 3H), 2.42 (t, *J =* 7.3 Hz, 2H), 1.52 (d, *J =* 6.7 Hz, 2H), 1.22 (s, 20H), 0.84 (t, *J =* 6.8 Hz, 3H).

### Example 45: Preparation of Compound 45

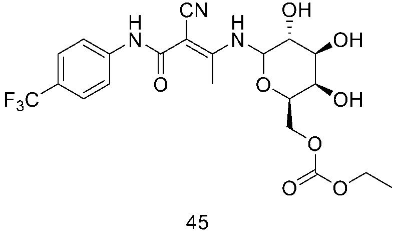

Compound 45 was prepared according to General Synthetic Method 1, wherein a yellow solid was afforded with a yield of 87.0%.

¹H NMR (600 MHz, DMSO-*d*₆) δ 10.87 (s, 1H), 9.57 (s, 1H), 7.80 (d, *J =* 8.5 Hz, 2H), 7.63 (d, *J =* 8.4 Hz, 2H), 4.67 (s, 1H), 4.19 (d, *J =* 8.1 Hz, 1H), 4.17 - 4.03 (m, 3H), 3.85 (d, *J =* 4.3 Hz, 1H), 3.68 (s, 1H), 3.43 (m, *J =* 10.9, 9.5, 5.5 Hz, 3H), 3.27 - 3.24 (m, 1H), 2.32 (s, 3H), 1.22 (q, *J* = 7.1, 6.5 Hz, 3H).

### Example 46: Preparation of Compound 46

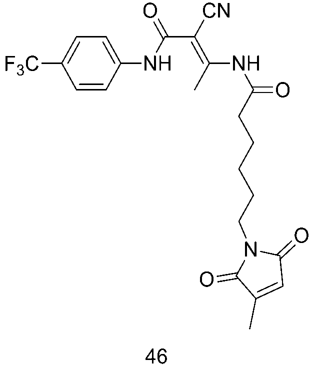

Compound 46 was prepared according to General Synthetic Method 3, wherein a white solid was afforded with a yield of 23.0%.

¹H NMR (400 MHz, DMSO-*d*₆) δ 12.23 (s, 1H), 10.20 (s, 1H), 7.82 (d, *J =* 8.5 Hz, 2H), 7.71 (d, *J =* 8.7 Hz, 2H), 6.60 (d, *J =* 1.9 Hz, 1H), 3.38 (d, *J =* 7.0 Hz, 2H), 2.66 (d, *J* = 11.0 Hz, 3H), 2.42 (t, *J =* 7.4 Hz, 2H), 1.97 (d, *J =* 1.8 Hz, 2H), 1.50 (m, *J =* 19.5, 15.0, 7.5 Hz, 4H), 1.28 - 1.21 (m, 2H).

### Example 47: Preparation of Compound 47

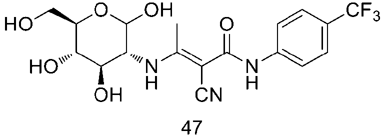

Compound 47 was prepared according to General Synthetic Method 1, wherein a yellow solid was afforded with a yield of 23.0%.

¹H NMR (400 MHz, DMSO-*d*₆) δ 10.75 (d, *J* = 9.7 Hz, 1H), 9.36 (s, 1H), 7.79 (d, *J* = 8.6 Hz, 2H), 7.62 (d, *J* = 8.7 Hz, 2H), 7.02 (s, 1H), 5.07 (s, 1H), 3.68 - 3.43 (m, 6H), 3.18 (d, *J* = 9.3 Hz, 2H), 2.33 (d, *J* = 6.8 Hz, 3H), 1.79 (s, 1H).

### Example 48: Preparation of Compound 48

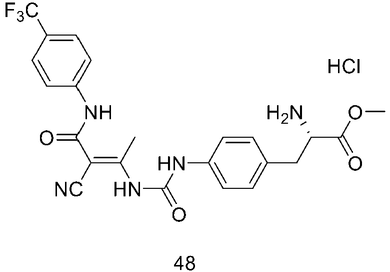

Compound 48 was prepared according to General Synthetic Method 3, wherein an off-white solid was afforded with a yield of 74.1%.

¹H NMR (400 MHz, DMSO-*d*₆) δ 12.06 (s, 1H), 10.44 (s, 1H), 10.07 (s, 1H), 8.52 (s, 3H), 7.85 (d, *J =* 8.6 Hz, 2H), 7.72 (d, *J =* 8.6 Hz, 2H), 7.51 - 7.44 (m, 2H), 7.21 - 7.14 (m, 2H), 4.27 (t, *J* = 6.6 Hz, 1H), 3.69 (s, 3H), 3.14 - 3.00 (m, 2H), 2.72 (s, 3H).

### Example 49: Preparation of Compound 49

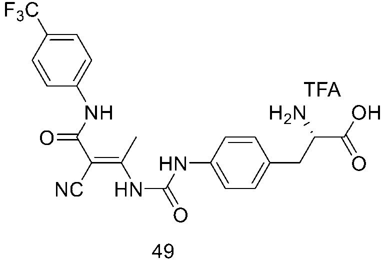

Compound 49 was prepared according to General Synthetic Method 3, wherein a white solid was afforded with a yield of 77.6%.

¹H NMR (400 MHz, DMSO-*d*₆) δ 12.08 (s, 1H), 10.43 (s, 1H), 10.07 (s, 1H), 8.24 (s, 3H), 7.85 (d, *J =* 8.5 Hz, 2H), 7.72 (d, *J =* 8.6 Hz, 2H), 7.47 (d, *J =* 8.3 Hz, 2H), 7.20 (d, *J =* 8.3 Hz, 2H), 4.17 (s, 1H), 3.11 - 2.98 (m, 2H), 2.73 (s, 3H).

### Example 50: Preparation of Compound 50

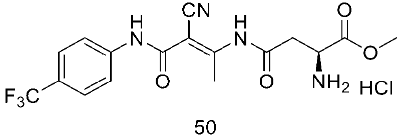

Compound 50 was prepared according to General Synthetic Method 3, wherein an off-white solid was afforded with a yield of 51.2%.

¹H NMR (400 MHz, DMSO-*d*₆) δ 10.36 (s, 1H), 8.54 (s, 3H), 7.83 (d, *J* = 8.6 Hz, 2H), 7.72 (d, *J =* 8.6 Hz, 2H), 4.37 (t, *J =* 5.2 Hz, 1H), 3.71 (s, 3H), 3.20 - 3.07 (m, 2H), 2.63 (s, 3H).

### Example 51: Preparation of Compound 51

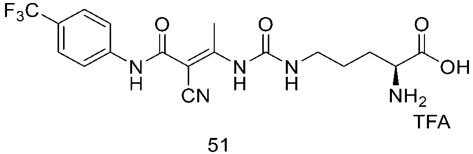

Compound 51 was prepared according to General Synthetic Method 3, wherein a white solid was afforded with a yield of 26.3%.

¹H NMR (400 MHz, DMSO-*d*₆) δ 11.89 (s, 1H), 10.03 - 9.91 (m, 1H), 8.34 (s, 1H), 7.82 (d, *J* = 8.5 Hz, 2H), 7.70 (d, *J* = 8.6 Hz, 2H), 3.55 - 3.42 (m, 1H), 3.36 (s, 2H), 3.06 (dd, *J* = 11.5, 5.3 Hz, 2H), 2.66 (s, 3H), 1.81 - 1.70 (m, 1H), 1.64 (td, *J* = 14.1, 12.8, 5.9 Hz, 1H), 1.51 (m, *J* = 12.1, 6.6 Hz, 2H), 1.11 (s, 1H).

### Example 52: Preparation of Compound 52

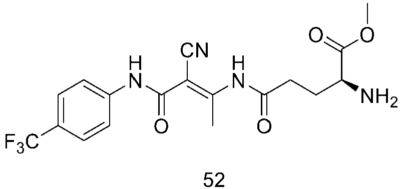

Compound 52 was prepared according to General Synthetic Method 3, wherein a white solid was afforded with a yield of 26.5%.

¹H NMR (400 MHz, DMSO-*d*₆) δ 12.31 (s, 1H), 10.26 (s, 1H), 8.55 (s, 3H), 7.77 (dd, *J* = 40.6, 8.4 Hz, 4H), 4.08 (s, 1H), 3.76 (d, *J* = 13.2 Hz, 3H), 2.66 (s, 3H), 2.06 (ddd, *J* = 29.5, 18.3, 11.5 Hz, 2H), 1.04 (d, *J =* 6.0 Hz, 1H).

### Example 53: Preparation of Compound 53

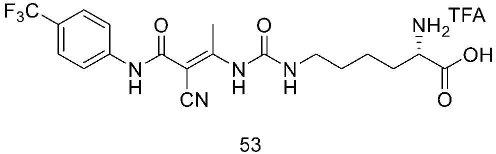

Compound 53 was prepared according to General Synthetic Method 3, wherein a white solid was afforded with a yield of 82.2%.

¹H NMR (400 MHz, DMSO-*d*₆) δ 11.89 (s, 1H), 9.95 (s, 1H), 8.44 - 8.04 (m, 3H), 7.82 (d, *J* = 8.4 Hz, 2H), 7.70 (d, *J* = 8.4 Hz, 2H), 3.83 (t, *J* = 6.2 Hz, 1H), 3.06 (q, *J* = 6.3 Hz, 2H), 2.66 (s, 3H), 1.76 (m, *J =* 7.2 Hz, 2H), 1.52 - 1.20 (m, 5H).

### Example 54: Preparation of Compound 54

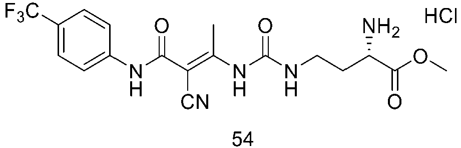

Compound 54 was prepared according to General Synthetic Method 3, wherein a white solid was afforded with a yield of 71.8%.

¹H NMR (400 MHz, DMSO-*d*₆) δ 11.91 (s, 1H), 9.98 (s, 1H), 8.63 (s, 3H), 8.37 (t, *J* = 5.4 Hz, 1H), 7.82 (d, *J =* 8.5 Hz, 2H), 7.70 (d, *J =* 8.7 Hz, 2H), 4.13 - 4.04 (m, 1H), 3.75 (s, 3H), 3.23 (m, *J =* 26.8, 6.8 Hz, 2H), 2.67 (s, 3H), 1.98 (m, *J =* 14.0, 7.1 Hz, 2H).

### Example 55: Preparation of Compound 55

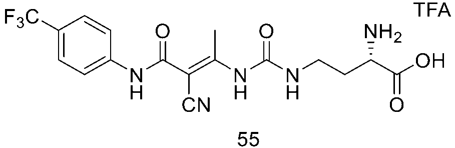

Compound 55 was prepared according to General Synthetic Method 3, wherein a white solid was afforded with a yield of 22.3%.

¹H NMR (400 MHz, DMSO-*d*₆) δ 11.00 (s, 1H), 10.65 (s, 1H), 8.33 (d, *J =* 8.3 Hz, 1H), 8.12 (d, *J =* 8.5 Hz, 2H), 8.08 - 7.98 (m, 7H), 4.75 (d, *J =* 8.3 Hz, 1H), 3.31 (t, *J =* 5.4 Hz, 2H), 2.94 (s, 5H).

### Example 56: Preparation of Compound 56

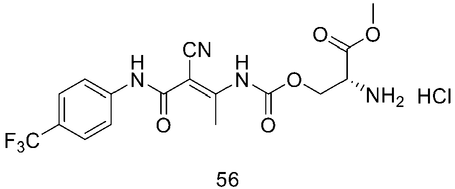

Compound 56 was prepared according to General Synthetic Method 3, wherein a white solid was afforded with a yield of 31.7%.

¹H NMR (400 MHz, DMSO-*d*₆) δ 12.11 (s, 1H), 10.24 (s, 1H), 8.80 (s, 3H), 7.81 (d, *J* = 8.6 Hz, 2H), 7.72 (d, *J =* 8.7 Hz, 2H), 4.64 - 4.48 (m, 3H), 3.86 - 3.68 (m, 3H), 2.75 - 2.60 (m, 3H).

### Example 57: Preparation of Compound 57

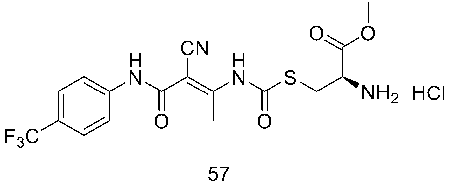

Compound 57 was prepared according to General Synthetic Method 3, wherein a white solid was afforded with a yield of 33.9%.

¹H NMR (400 MHz, DMSO-*d*₆) δ 12.76 (s, 1H), 10.36 (s, 1H), 8.62 (s, 3H), 7.77 (dd, *J =* 35.6, 8.5 Hz, 4H), 4.40 (s, 1H), 3.77 (d, *J =* 7.6 Hz, 3H), 3.46 (s, 2H), 2.65 (d, *J =* 16.7 Hz, 3H).

### Example 58: Preparation of Compound 58

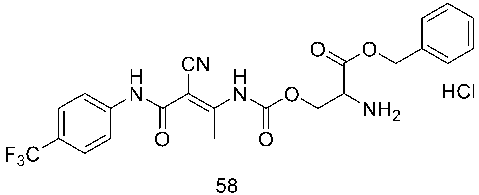

Compound 58 was prepared according to General Synthetic Method 3, wherein a white solid was afforded with a yield of 84.2%.

¹H NMR (400 MHz, DMSO-*d*₆) δ 12.05 (s, 1H), 10.25 (s, 1H), 8.71 (s, 3H), 7.81 (d, *J* = 8.6 Hz, 2H), 7.73 (d, *J =* 8.6 Hz, 2H), 7.47 - 7.22 (m, 5H), 5.35 - 5.21 (m, 2H), 4.64 (t, *J =* 3.8 Hz, 1H), 4.61 - 4.49 (m, 2H), 2.62 (s, 3H).

### Example 59: Preparation of Compound 59

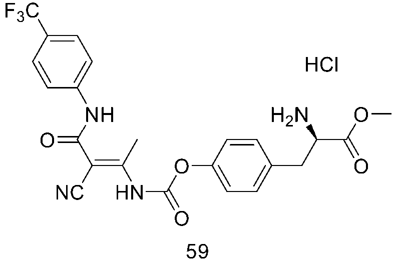

Compound 59 was prepared according to General Synthetic Method 3, wherein a white solid was afforded with a yield of 56.7%.

¹H NMR (400 MHz, DMSO-*d*₆) δ 12.46 (s, 1H), 10.31 (s, 1H), 8.62 (s, 3H), 7.96 - 7.60 (m, 4H), 7.27 (dd, *J =* 36.5, 8.6 Hz, 4H), 4.32 (t, *J* = 6.3 Hz, 1H), 3.70 (d, *J =* 8.0 Hz, 3H), 3.15 (qd, *J* = 14.2, 6.6 Hz, 2H), 2.68 (s, 3H).

### Example 60: Preparation of Compound 60

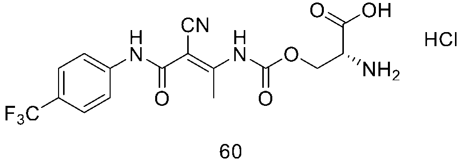

Compound 60 was prepared according to General Synthetic Method 3, wherein a white solid was afforded with a yield of 13.7%.

¹H NMR (400 MHz, DMSO-*d*₆) δ 12.14 (s, 1H), 10.23 (s, 1H), 8.61 (s, 3H), 7.77 (dd, *J* = 35.6, 8.6 Hz, 4H), 4.60 (dd, *J =* 12.0, 2.5 Hz, 1H), 4.56 - 4.35 (m, 2H), 2.66 (s, 3H).

### Example 61: Preparation of Compound 61

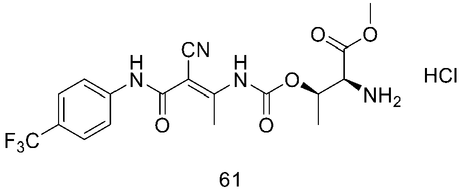

Compound 61 was prepared according to General Synthetic Method 3, wherein a white solid was afforded with a yield of 71.5%.

¹H NMR (400 MHz, DMSO-*d*₆) δ 12.04 (s, 1H), 10.24 (s, 1H), 8.72 (s, 3H), 7.81 (d, *J* = 8.5 Hz, 2H), 7.73 (d, *J =* 8.6 Hz, 2H), 5.29 - 5.22 (m, 1H), 4.44 (s, 1H), 3.77 (s, 3H), 2.65 (s, 3H), 1.39 (d, *J* = 6.5 Hz, 3H).

### Example 62: Preparation of Compound 62

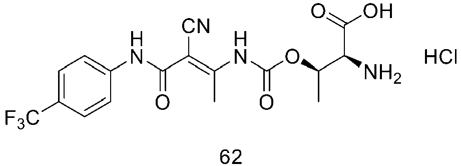

Compound 62 was prepared according to General Synthetic Method 3, wherein a white solid was afforded with a yield of 73.8%.

¹H NMR (400 MHz, DMSO-*d*₆) δ 12.05 (s, 1H), 10.23 (s, 1H), 8.57 (s, 3H), 7.77 (dd, *J* = 34.3, 8.7 Hz, 4H), 5.36 - 5.19 (m, 1H), 4.26 (s, 1H), 2.66 (s, 4H), 1.39 (d, *J* = 6.6 Hz, 3H).

### Example 63: Preparation of Compound 63

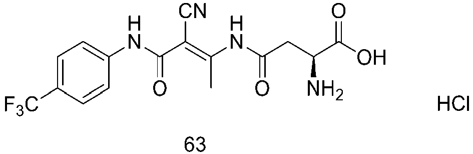

Compound 63 was prepared according to General Synthetic Method 3, wherein a white solid was afforded with a yield of 43.9%.

¹H NMR (400 MHz, DMSO-*d*₆) δ 10.69 (s, 1H), 7.88 - 7.63 (m, 7H), 4.53 (d, *J =* 8.2 Hz, 1H), 4.18 (t, *J* = 5.6 Hz, 1H), 2.71 - 2.59 (m, 3H), 2.16 (s, 3H).

### Example 64: Preparation of Compound 64

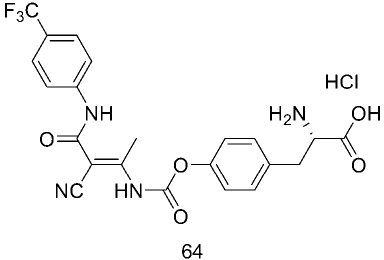

Compound 64 was prepared according to General Synthetic Method 3, wherein a white solid was afforded with a yield of 95.5%.

¹H NMR (400 MHz, DMSO-*d*₆) δ 12.46 (s, 1H), 10.31 (s, 1H), 8.39 (s, 3H), 7.93 - 7.67 (m, 4H), 7.41 - 7.16 (m, 4H), 4.20 (s, 1H), 3.21 - 3.05 (m, 2H), 2.68 (s, 3H).

### Example 65: Preparation of Compound 65

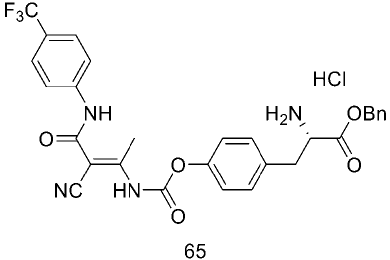

Compound 65 was prepared according to General Synthetic Method 3, wherein a white solid was afforded with a yield of 84.1%.

¹H NMR (400 MHz, DMSO-*d*₆) δ 12.46 (s, 1H), 10.31 (s, 1H), 8.64 (s, 3H), 7.93 - 7.65 (m, 4H), 7.37 (d, *J* = 7.1 Hz, 3H), 7.33 - 7.23 (m, 5H), 7.18 (d, *J* = 8.5 Hz, 2H), 5.16 (q, *J* = 12.3 Hz, 2H), 3.18 (ddd, *J* = 26.4, 15.2, 8.9 Hz, 2H), 2.68 (s, 3H).

### Example 66: Preparation of Compound 66

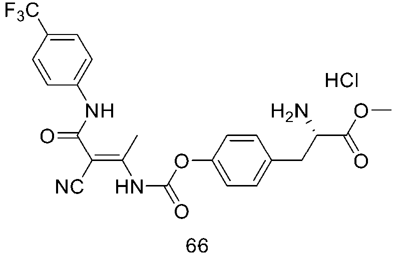

Compound 66 was prepared according to General Synthetic Method 3, wherein a white solid was afforded with a yield of 55.6%.

¹H NMR (400 MHz, DMSO) δ 12.46 (s, 1H), 10.31 (s, 1H), 8.64 (s, 3H), 7.92 - 7.64 (m, 4H), 7.27 (dd, *J* = 37.4, 8.6 Hz, 4H), 4.31 (t, *J* = 6.6 Hz, 1H), 3.69 (d, *J* = 8.1 Hz, 3H), 3.25 - 3.02 (m, 2H), 2.68 (s, 3H).

### Example 67: Preparation of Compound 67

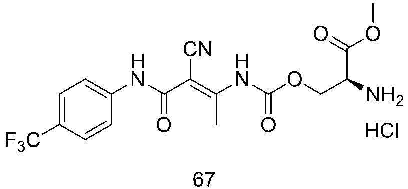

Compound 67 was prepared according to General Synthetic Method 3, wherein a white solid was afforded with a yield of 95.8%.

¹H NMR (400 MHz, DMSO-*d*₆) δ 12.11 (s, 1H), 10.24 (s, 1H), 8.81 (s, 3H), 7.81 (dt, *J* = 35.3, 8.6 Hz, 4H), 4.70 - 4.41 (m, 3H), 3.77 (d, *J =* 13.0 Hz, 3H), 2.66 (s, 3H).

### Example 68: Preparation of Compound 68

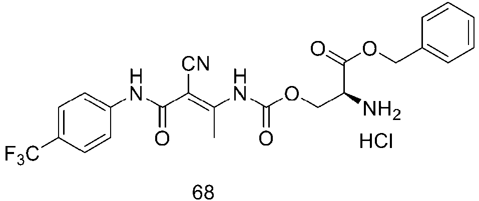

Compound 68 was prepared according to General Synthetic Method 3, wherein a white solid was afforded with a yield of 55.6%.

¹H NMR (400 MHz, DMSO-*d*₆) δ 12.05 (s, 1H), 10.25 (s, 1H), 8.73 (s, 3H), 7.91 - 7.67 (m, 4H), 7.49 - 7.25 (m, 5H), 5.28 (dd, *J =* 26.6, 12.4 Hz, 2H), 4.69 - 4.48 (m, 3H), 2.62 (s, 3H).

### Example 69: Preparation of Compound 69

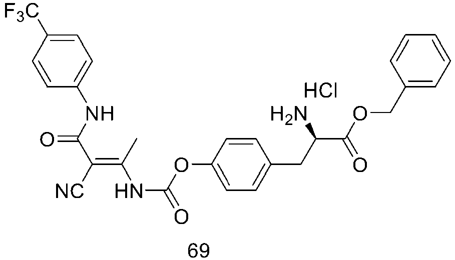

Compound 69 was prepared according to General Synthetic Method 3, wherein a white oily liquid was afforded with a yield of 44.7%.

¹H NMR (400 MHz, DMSO-*d*₆) δ 12.46 (s, 1H), 10.31 (s, 1H), 8.65 (s, 3H), 8.06 - 7.60 (m, 4H), 7.41 - 7.11 (m, 9H), 5.16 (q, *J* = 12.3 Hz, 2H), 4.38 (t, *J* = 6.5 Hz, 1H), 3.16 (ddd, *J* = 21.6, 14.2, 6.7 Hz, 2H), 2.68 (s, 3H).

### Example 70: Preparation of Compound 70

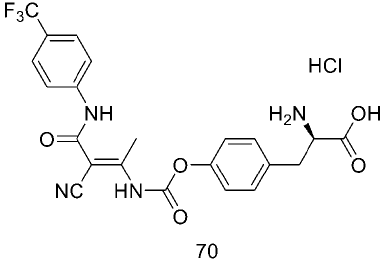

Compound 70 was prepared according to General Synthetic Method 3, wherein a white solid was afforded with a yield of 80.6%.

¹H NMR (400 MHz, DMSO-*d*₆) δ 13.89 (s, 1H), 12.46 (s, 1H), 10.30 (s, 1H), 8.44 (s, 3H), 7.78 (dd, *J* = 46.6, 8.6 Hz, 4H), 7.29 (dd, *J* = 50.4, 8.6 Hz, 4H), 4.19 (s, 1H), 3.15 (d, *J* = 6.3 Hz, 2H), 2.70 (d, *J =* 21.7 Hz, 3H).

### Example 71: Preparation of Compound 71

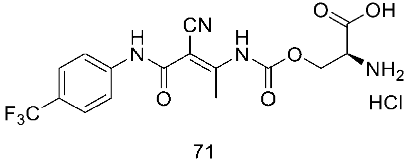

Compound 71 was prepared according to General Synthetic Method 3, wherein a white solid was afforded with a yield of 48.9%.

¹H NMR (400 MHz, DMSO-*d*₆) δ 12.14 (s, 1H), 10.23 (s, 1H), 8.58 (s, 3H), 7.77 (dd, *J* = 35.3, 8.7 Hz, 4H), 4.70 - 4.32 (m, 3H), 2.66 (s, 3H).

### Example 72: Preparation of Compound 72

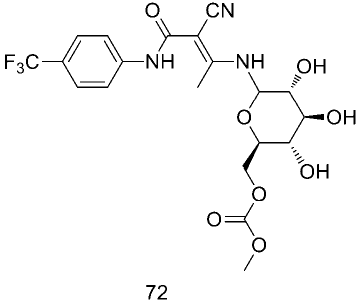

Compound 72 was prepared according to General Synthetic Method 1, wherein an off-white solid was afforded with a yield of 71.0%.

¹H NMR (600 MHz, DMSO-*d*₆) δ 10.85 (d, *J =* 7.5 Hz, 1H), 9.58 (s, 1H), 7.80 (d, *J =* 8.6 Hz, 2H), 7.64 (d, *J =* 8.7 Hz, 2H), 5.62 (d, *J =* 5.6 Hz, 1H), 5.35 (d, *J =* 5.5 Hz, 1H), 5.23 (d, *J* = 5.3 Hz, 1H), 4.76 (t, *J =* 8.1 Hz, 1H), 4.40 - 4.33 (m, 1H), 4.13 - 4.07 (m, 1H), 3.70 (s, 3H), 3.59 (s, 1H), 3.29 - 3.24 (m, 1H), 3.17 - 3.09 (m, 2H), 2.34 (s, 3H).

### Example 73: Preparation of Compound 73

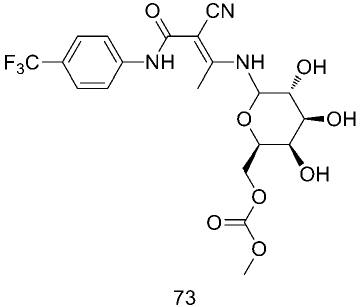

Compound 73 was prepared according to General Synthetic Method 1, wherein an off-white solid was afforded with a yield of 21.0%.

¹H NMR (600 MHz, DMSO-*d*₆) δ 10.87 (d, *J =* 7.7 Hz, 1H), 9.57 (s, 1H), 7.80 (d, *J =* 8.6 Hz, 2H), 7.64 (d, *J =* 8.8 Hz, 2H), 5.43 (d, *J* = 5.2 Hz, 1H), 4.99 (d, *J =* 5.4 Hz, 1H), 4.85 (d, *J* = 5.2 Hz, 1H), 4.69 (d, *J* = 7.7 Hz, 1H), 4.22 - 4.16 (m, 2H), 3.87 (d, *J* = 4.6 Hz, 1H), 3.69 (d, *J =* 13.8 Hz, 4H), 3.46 - 3.41 (m, 2H), 2.34 (s, 3H).

### Example 74: Preparation of Compound 74

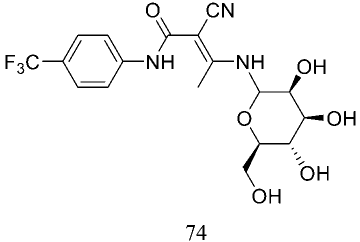

Compound 74 was prepared according to General Synthetic Method 1, wherein an off-white solid was afforded with a yield of 15.7%.

¹H NMR (400 MHz, DMSO-*d*₆) δ 11.13 (s, 1H), 9.60 (s, 1H), 7.80 (d, *J* = 8.7 Hz, 2H), 7.64 (d, *J* = 8.9 Hz, 2H), 5.28 (s, 1H), 5.18 (s, 2H), 5.13 - 5.06 (m, 1H), 4.96 (s, 2H), 4.86 (s, 1H), 4.55 (s, 2H), 3.72 (s, 2H), 2.43 - 2.35 (m, 3H).

### Example 75: Preparation of Compound 75

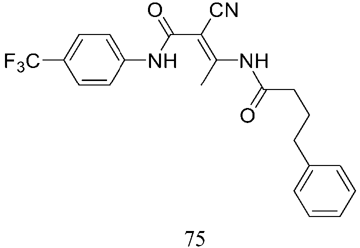

Compound 75 was prepared according to General Synthetic Method 3, wherein a white solid was afforded with a yield of 37.7%.

¹H NMR (600 MHz, DMSO-*d*₆) δ 12.20 (s, 1H), 10.17 (s, 1H), 7.81 (d, *J =* 8.5 Hz, 2H), 7.70 (d, *J* = 8.7 Hz, 2H), 7.32 - 7.24 (m, 2H), 7.18 (d, *J* = 7.6 Hz, 3H), 2.63 (s, 3H), 2.61 (d, *J* = 7.5 Hz, 2H), 2.44 (t, *J* = 7.3 Hz, 2H), 1.89 - 1.82 (m, 2H).

### Example 76: Preparation of Compound 76

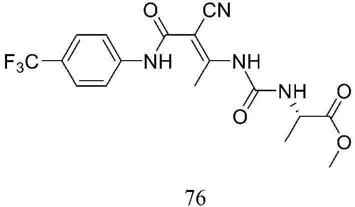

Compound 76 was prepared according to General Synthetic Method 3, wherein a white solid was afforded with a yield of 59.1%.

¹H NMR (600 MHz, DMSO-*d*₆) δ 11.88 (s, 1H), 9.96 (s, 1H), 8.72 (d, *J* = 6.7 Hz, 1H), 7.83 (d, *J =* 8.6 Hz, 2H), 7.70 (d, *J =* 8.7 Hz, 2H), 4.29 - 4.16 (m, 1H), 3.64 (s, 3H), 2.64 (s, 3H), 1.30 (d, *J* = 7.3 Hz, 3H).

### Example 77: Preparation of Compound 78

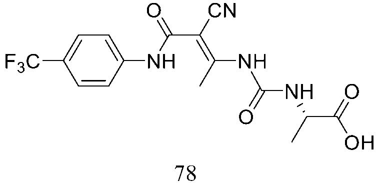

Compound 78 was prepared according to General Synthetic Method 3, wherein a white solid was afforded with a yield of 56.5%.

¹H NMR (600 MHz, DMSO-*d*₆) δ 11.84 (s, 1H), 9.95 (s, 1H), 8.61 (d, *J =* 6.9 Hz, 1H), 7.83 (d, *J =* 8.5 Hz, 2H), 7.70 (d, *J =* 8.6 Hz, 2H), 4.11 (p, *J =* 7.2 Hz, 1H), 2.63 (d, *J =* 21.5 Hz, 3H), 1.41 - 1.15 (m, 3H).

### Example 78: Preparation of Compound 79

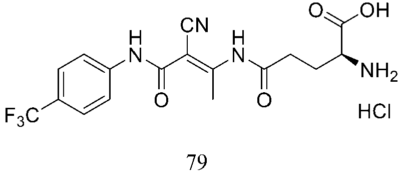

Compound 79 was prepared according to General Synthetic Method 3, wherein a white solid was afforded with a yield of 57.4%.

¹H NMR (600 MHz, DMSO-*d*₆) δ 12.32 (s, 1H), 10.22 (s, 1H), 8.38 (s, 3H), 7.86 - 7.79 (m, 2H), 7.71 (d, *J =* 8.7 Hz, 2H), 3.94 (s, 1H), 2.74 (ddd, *J =* 15.4, 10.3, 5.9 Hz, 1H), 2.68 - 2.60 (m, 4H), 2.15 - 2.08 (m, 1H), 2.03 (ddd, *J* = 17.9, 15.2, 7.7 Hz, 1H).

### Example 79: Preparation of Compound 80

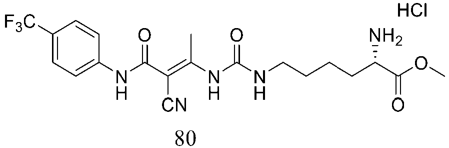

Compound 80 was prepared according to General Synthetic Method 3, wherein a yellow solid was afforded with a yield of 71.3%.

¹H NMR (600 MHz, DMSO-*d*₆) δ 11.86 (s, 1H), 9.92 (s, 1H), 8.50 (s, 3H), 8.22 (s, 1H), 7.82 (d, *J =* 8.5 Hz, 2H), 7.68 (dd, *J =* 15.0, 8.7 Hz, 2H), 4.07 - 3.95 (m, 1H), 3.75 (d, *J =* 5.7 Hz, 3H), 3.05 (d, *J* = 5.5 Hz, 2H), 2.71 - 2.61 (m, 3H), 1.85 - 1.72 (m, 2H), 1.48 - 1.36 (m, 4H).

### Example 80: Preparation of Compound 81

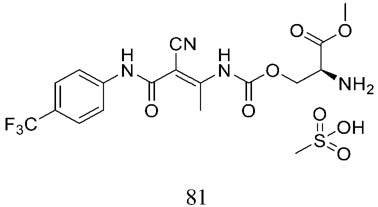

Compound 81 was prepared according to General Synthetic Method 3, wherein a white solid was afforded with a yield of 83.5%.

¹H NMR (600 MHz, DMSO-*d*₆) δ 12.11 (s, 1H), 10.20 (s, 1H), 8.64 (s, 3H), 7.81 (d, *J* = 8.6 Hz, 2H), 7.72 (d, *J =* 8.7 Hz, 2H), 4.61 - 4.47 (m, 3H), 3.80 (s, 3H), 2.64 (d, *J* = 25.8 Hz, 3H), 2.35 (s, 3H).

### Example 81: Preparation of Compound 82

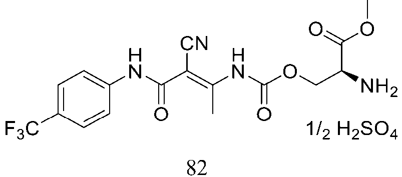

Compound 82 was prepared according to General Synthetic Method 3, wherein a white solid was afforded with a yield of 95.5%.

¹H NMR (600 MHz, DMSO-*d*₆) δ 12.12 (s, 1H), 10.21 (s, 1H), 8.61 (s, 3H), 7.80 (d, *J* = 8.6 Hz, 2H), 7.72 (d, *J =* 8.7 Hz, 2H), 4.63 - 4.47 (m, 3H), 3.80 (s, 3H), 2.66 (s, 3H).

### Example 82: Preparation of Compound 83

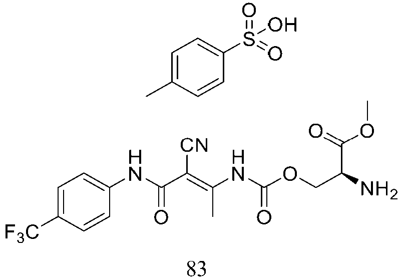

Compound 83 was prepared according to General Synthetic Method 3, wherein a white solid was afforded with a yield of 70.1%.

¹H NMR (600 MHz, DMSO-*d*₆) δ 12.10 (s, 1H), 10.20 (s, 1H), 8.64 (s, 3H), 7.85 - 7.76 (m, 2H), 7.72 (d, *J* = 8.7 Hz, 2H), 7.48 (d, *J* = 8.0 Hz, 2H), 7.11 (d, *J* = 7.9 Hz, 2H), 4.56 (dd, *J* = 9.0*,* 3.5 Hz, 2H), 4.50 (dd, *J =* 12.9, 5.4 Hz, 1H), 3.83 - 3.75 (m, 3H), 2.63 (d, *J =* 24.1 Hz, 3H), 2.29 (s, 3H).

### Example 83: Preparation of Compound 84

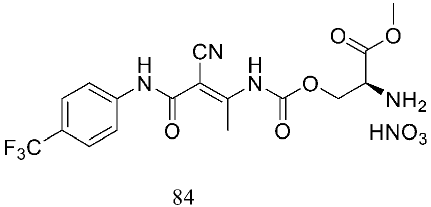

Compound 84 was prepared according to General Synthetic Method 3, wherein a white solid was afforded with a yield of 86.2%.

¹H NMR (600 MHz, DMSO-*d*₆) δ 12.11 (s, 1H), 10.20 (s, 1H), 8.61 (s, 3H), 7.80 (d, *J* = 8.5 Hz, 2H), 7.72 (d, *J =* 8.6 Hz, 2H), 4.61 - 4.42 (m, 3H), 3.80 (s, 3H), 2.64 (d, *J =* 27.0 Hz, 3H).

### Example 84: Preparation of Compound 85

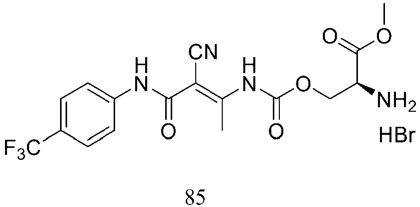

Compound 85 was prepared according to General Synthetic Method 3, wherein a white solid was afforded with a yield of 83.2%.

¹H NMR (600 MHz, DMSO-*d*₆) δ 12.11 (s, 1H), 10.20 (s, 1H), 8.66 (s, 3H), 7.81 (d, *J = 8.5* Hz, 2H), 7.72 (d, *J =* 8.5 Hz, 2H), 4.58 (d, *J =* 3.9 Hz, 1H), 4.57 - 4.48 (m, 2H), 3.80 (s, 3H), 2.66 (s, 3H).

### Example 85: Preparation of Compound 86

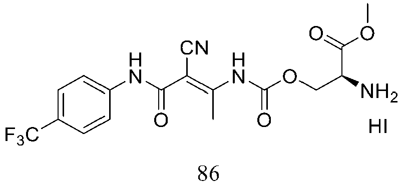

Compound 86 was prepared according to General Synthetic Method 3, wherein a pale-yellow solid was afforded with a yield of 70.2%.

¹H NMR (600 MHz, DMSO-*d*₆) δ 12.11 (s, 1H), 10.20 (s, 1H), 8.63 (s, 3H), 7.81 (d, *J* = 8.5 Hz, 2H), 7.73 (d, *J =* 8.6 Hz, 2H), 4.59 (d, *J =* 5.3 Hz, 1H), 4.58 - 4.48 (m, 2H), 3.80 (s, 3H), 2.66 (s, 3H).

### Example 86: Preparation of Compound 87

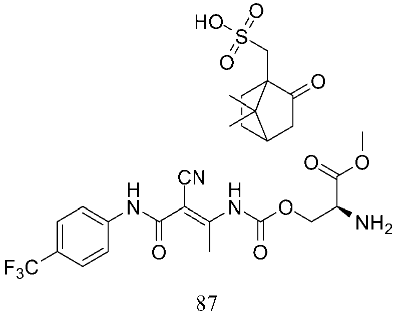

Compound 87 was prepared according to General Synthetic Method 3, wherein a white solid was afforded with a yield of 42.5%.

¹H NMR (600 MHz, DMSO-*d*₆) δ 12.12 (d, *J =* 16.9 Hz, 1H), 10.20 (s, 1H), 8.64 (s, 3H), 7.81 (d, *J =* 8.4 Hz, 2H), 7.72 (d, *J =* 8.5 Hz, 2H), 4.60 - 4.53 (m, 2H), 4.50 (dd, *J =* 12.6, 5.1 Hz, 1H), 2.67 (d, *J =* 17.8 Hz, 5H), 2.24 (dt, *J =* 18.0, 3.8 Hz, 2H), 1.94 (t, *J =* 4.6 Hz, 2H), 1.89 - 1.83 (m, 2H), 1.29 - 1.25 (m, 4H), 0.75 (s, 6H).

### Example 87: Preparation of Compound 88

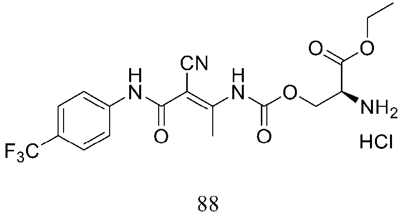

Compound 88 was prepared according to General Synthetic Method 3, wherein a colorless oily liquid was afforded with a yield of 56.9%.

¹H NMR (600 MHz, DMSO-*d*₆) δ 12.12 (s, 1H), 10.20 (s, 1H), 8.74 (s, 3H), 7.81 (d, *J* = 8.5 Hz, 2H), 7.72 (d, *J =* 8.5 Hz, 2H), 4.61 - 4.50 (m, 3H), 4.25 (qt, *J =* 7.8, 4.1 Hz, 2H), 2.66 (s, 3H), 1.24 (t, *J* = 7.1 Hz, 3H).

### Example 88: Preparation of Compound 89

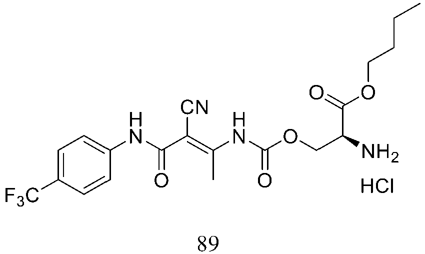

Compound 89 was prepared according to General Synthetic Method 3, wherein a white solid was afforded with a yield of 64.5%.

¹H NMR (600 MHz, DMSO-*d*₆) δ 12.13 (s, 1H), 10.20 (s, 1H), 8.72 (s, 3H), 7.80 (d, J = 8.5 Hz, 2H), 7.71 (d, J = 8.6 Hz, 2H), 4.61 - 4.51 (m, 3H), 4.24 (dt, J = 10.9, 6.5 Hz, 1H), 4.17 (dt, J = 10.8, 6.4 Hz, 1H), 2.66 (s, 3H), 1.65 - 1.57 (m, 2H), 1.34 (p, J = 7.5 Hz, 2H), 0.86 (t, J = 7.4 Hz, 3H).

### Example 89: Preparation of Compound 90

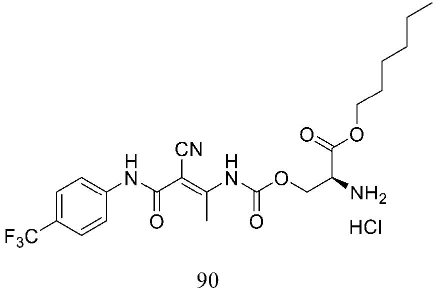

Compound 90 was prepared according to General Synthetic Method 3, wherein a white solid was afforded with a yield of 56.6%.

¹H NMR (600 MHz, DMSO-*d*₆) δ 12.15 (s, 1H), 10.21 (s, 1H), 8.68 (s, 3H), 7.80 (d, J = 8.5 Hz, 2H), 7.71 (d, J = 8.6 Hz, 2H), 4.54 (d, J = 3.4 Hz, 3H), 4.25 (dt, J = 12.5, 6.5 Hz, 1H), 4.15 (dt, J = 11.0, 6.6 Hz, 1H), 2.66 (s, 3H), 1.60 (t, J = 8.3 Hz, 2H), 1.30 (t, J = 7.4 Hz, 2H), 1.25 - 1.20 (m, 4H), 0.84 - 0.79 (m, 3H).

### Example 90: Preparation of Compound 91

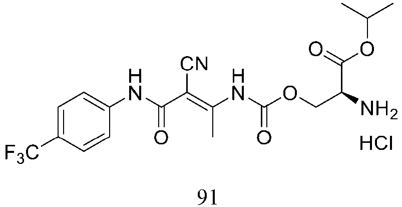

Compound 91 was prepared according to General Synthetic Method 3, wherein a white solid was afforded with a yield of 44.5 %.

¹H NMR (600 MHz, DMSO-*d*₆) δ 12.14 (s, 1H), 10.21 (s, 1H), 8.80 (s, 3H), 7.81 (d, J = 8.4 Hz, 2H), 7.72 (d, J = 8.5 Hz, 2H), 5.07 - 4.99 (m, 1H), 4.59 - 4.46 (m, 3H), 2.66 (s, 3H), 1.25 (dd, J = 10.4, 6.1 Hz, 6H).

### Example 91: Preparation of Compound 92

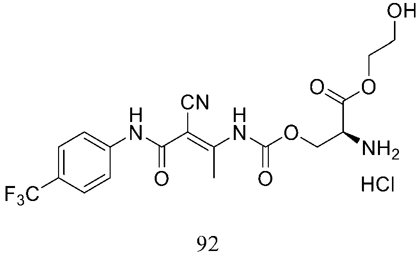

Compound 92 was prepared according to General Synthetic Method 3, wherein a white solid was afforded with a yield of 41.3%.

¹H NMR (600 MHz, DMSO-*d*₆) δ 12.11 (s, 1H), 10.19 (s, 1H), 8.74 (s, 3H), 7.81 (d, J = 8.4 Hz, 2H), 7.72 (d, J = 8.5 Hz, 2H), 4.89 (s, 1H), 4.60 - 4.52 (m, 3H), 4.22 (q, J = 4.3 Hz, 2H), 3.61 (t, J = 4.9 Hz, 2H), 2.66 (s, 3H), 2.41 (s, 1H).

### Example 92: Preparation of Compound 93

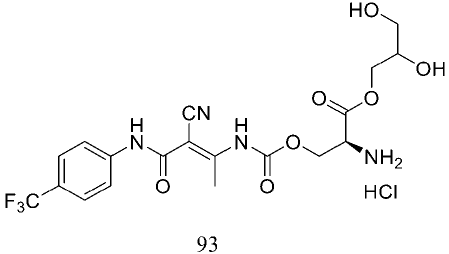

Compound 93 was prepared according to General Synthetic Method 3, wherein a white solid was afforded with a yield of 48.5%.

¹H NMR (600 MHz, DMSO-*d*₆) δ 12.11 (s, 1H), 10.19 (s, 1H), 8.78 (s, 3H), 7.82 (d, J = 8.5 Hz, 2H), 7.72 (d, J = 8.5 Hz, 2H), 5.01 (s, 1H), 4.70 (s, 1H), 4.63 - 4.52 (m, 3H), 4.27 (dd, J = 11.0, 3.8 Hz, 1H), 4.12 - 4.07 (m, 1H), 3.70 (t, J = 5.2 Hz, 1H), 3.45 - 3.34 (m, 2H), 2.66 (s, 3H).

### Example 93: Preparation of Compound 94

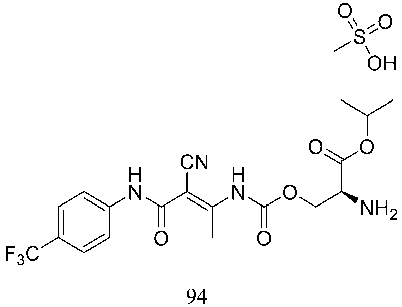

Compound 94 was prepared according to General Synthetic Method 3, wherein a white solid was afforded with a yield of 95.0%.

¹H NMR (400 MHz, DMSO-*d*₆) δ 12.15 (s, 1H), 10.21 (s, 1H), 8.60 (s, 3H), 7.80 (d, J = 8.6 Hz, 2H), 7.72 (d, J = 8.7 Hz, 2H), 5.05 (p, J = 6.2 Hz, 1H), 4.52 (s, 3H), 2.66 (s, 3H), 2.34 (s, 7H), 1.26 (dd, J = 10.0, 6.2 Hz, 6H).

### Example 94: Preparation of Compound 95

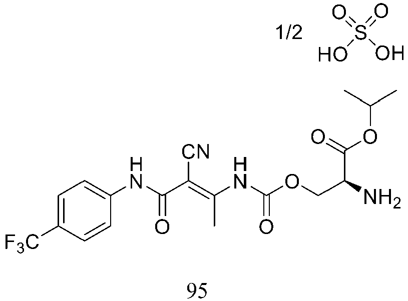

Compound 95 was prepared according to General Synthetic Method 3, wherein a white solid was afforded with a yield of 85.6%.

¹H NMR (400 MHz, DMSO-*d*₆) δ 12.16 (s, 1H), 10.21 (s, 1H), 8.58 (s, 3H), 7.80 (d, J = 8.6 Hz, 2H), 7.72 (d, J = 8.7 Hz, 2H), 5.06 (h, J = 6.2 Hz, 1H), 4.52 (s, 3H), 2.66 (s, 3H), 1.26 (dd, J = 9.9, 6.2 Hz, 6H).

### Example 95: Preparation of Compound 96

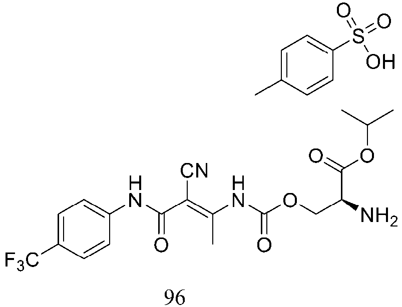

Compound 96 was prepared according to General Synthetic Method 3, wherein a white solid was afforded with a yield of 58.0%.

¹H NMR (400 MHz, DMSO-*d*₆) δ 12.15 (s, 1H), 10.21 (s, 1H), 8.65 - 8.52 (m, 3H), 7.80 (d, J = 8.6 Hz, 2H), 7.72 (d, J = 8.6 Hz, 2H), 7.50 - 7.45 (m, 2H), 7.11 (d, J = 7.9 Hz, 2H), 5.05 (p, J = 6.2 Hz, 1H), 4.52 (s, 3H), 2.66 (s, 3H), 2.29 (s, 3H), 1.26 (dd, J = 9.7, 6.2 Hz, 6H).

### Example 96: Preparation of Compound 97

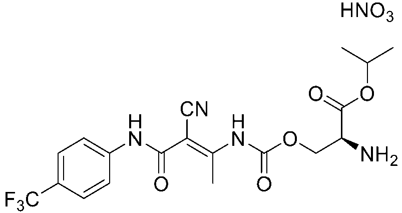

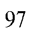

Compound 97 was prepared according to General Synthetic Method 3, wherein a white solid was afforded with a yield of 46.0%.

¹H NMR (400 MHz, DMSO-*d*₆) δ 12.16 (s, 1H), 10.21 (s, 1H), 8.57 (s, 3H), 7.80 (d, J = 8.6 Hz, 2H), 7.72 (d, J = 8.6 Hz, 2H), 5.05 (p, J = 6.2 Hz, 1H), 4.52 (s, 3H), 2.66 (s, 3H), 1.26 (dd, J = 9.9, 6.2 Hz, 6H).

### Example 97: Preparation of Compound 98

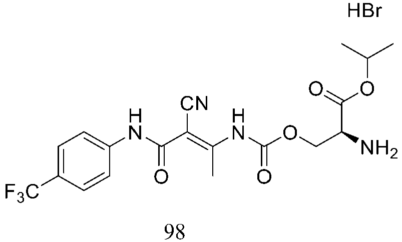

Compound 98 was prepared according to General Synthetic Method 3, wherein a white solid was afforded with a yield of 50.4%.

¹H NMR (400 MHz, DMSO-*d*₆) δ 12.15 (s, 1H), 10.21 (s, 1H), 8.61 (s, 3H), 7.81 (d, J = 8.6 Hz, 2H), 7.72 (d, J = 8.6 Hz, 2H), 5.06 (h, J = 6.2 Hz, 1H), 4.53 (s, 3H), 2.66 (s, 3H), 1.26 (dd, J = 10.1, 6.3 Hz, 6H).

### Example 98: Preparation of Compound 99

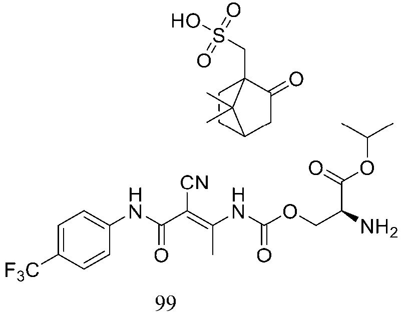

Compound 99 was prepared according to General Synthetic Method 3, wherein a white solid was afforded with a yield of 50.4%.

¹H NMR (600 MHz, DMSO-*d*₆) δ 12.12 (d, *J =* 16.9 Hz, 1H), 10.20 (s, 1H), 8.64 (s, 3H), 7.81 (d, *J =* 8.4 Hz, 2H), 7.72 (d, *J =* 8.5 Hz, 2H), 4.60 - 4.53 (m, 2H), 4.50 (dd, *J =* 12.6, 5.1 Hz, 1H), 2.67 (d, *J =* 17.8 Hz, 5H), 2.24 (dt, *J =* 18.0, 3.8 Hz, 2H), 1.29 - 1.25 (m, 4H), 1.26 (dd, J = 10.1, 6.3 Hz, 6H), 0.75(s, 6H).

### Example 99: Preparation of Compound 100

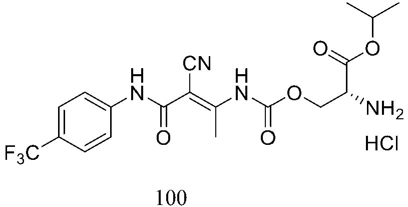

Compound 100 was prepared according to General Synthetic Method 3, wherein a white solid was afforded with a yield of 47.5 %.

¹H NMR (600 MHz, DMSO-*d*₆) δ 12.13 (s, 1H), 10.20 (s, 1H), 8.80 (s, 3H), 7.81 (d, J = 8.4 Hz, 2H), 7.72 (d, J = 8.5 Hz, 2H), 5.06 - 5.02 (m, 1H), 4.59 - 4.52 (m, 2H), 4.48 (s, 1H),2.66 (s, 3H), 1.25 (dd, J = 15.5, 6.2 Hz, 6H).

### Example 100: Preparation of Compound 102

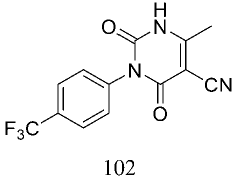

Compound 102 was prepared according to General Synthetic Method 3 or General Synthetic Method 4, wherein a yellowish-white solid was afforded with a yield of 48.5%.

¹H NMR (600 MHz, DMSO-*d*₆) δ 12.48 (s, 1H), 7.88 - 7.87 (m, 2H), 7.57 - 7.55 (m, 2H), 2.40 (s, 3H).

### General Synthetic Method 4:

The compound of Formula (H) (1.0 eq.) was dispersed in DMAC or DMF, and was stirred until dissolved; the mixture was cooled to -10°C, and then was added with 60% NaH (1.5 eq.); the reaction solution was stirred at -10°C for 30 minutes, and 4-nitrophenyl chloromethyl carbonate (1.3 eq.) was added slowly; the reaction mixture was warmed slowly from -10°C to room temperature; after the reaction, EA was added, and the mixture was washed with saturated ammonium chloride solution three times and with saturated sodium chloride solution once, was dried with anhydrous sodium sulfate, was filtered and concentrated, and then was subject to column chromatography purification, thereby affording the intramolecular cyclization product of Formula (H).

The intramolecular cyclization product of Formula (H) (1.0 eq.) was dispersed in ACN and was cooled to 0°C; cesium carbonate (1.5 eq.) or potassium carbonate (1.5 to 2.0 eq.) was added, and the mixture was stirred for 30 minutes; a solution of Formula (J) (1.5 eq.) in ACN was added slowly, and the reaction was warmed slowly from 0°C to reflux; after the reaction, EA was added, and then the mixture was washed with saturated ammonium chloride solution three times and with saturated sodium chloride solution once, was dried with anhydrous sodium sulfate, and then was filtered and concentrated; a product obtained by column chromatography purification was then subjected to deprotection or no deprotection via acid treatment, base treatment or palladium-carbon reduction to afford the compound of Formula (I).

Alternatively, the compound of Formula (K) (1.0 eq.) was dispersed in DMAC or DMF and was cooled to 0°C; NaH (1.5 to 2.0 eq.) was added, and the mixture was stirred for 30 minutes; the solution of Formula (J) (1.5 eq.) in DMF was added slowly, and the reaction mixture was warmed slowly from 0°C to 60°C; after the reaction, EA was added, and then the mixture was washed with saturated ammonium chloride solution three times and with saturated sodium chloride solution once, was dried with anhydrous sodium sulfate, and then was filtered and concentrated; a product obtained by column chromatography purification was then subjected to deprotection or no deprotection via acid treatment, base treatment or palladium-carbon reduction to afford the compound of Formula (I).

### Example 101: Preparation of Compound 104

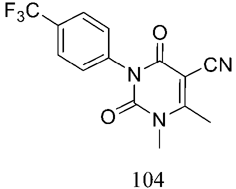

Compound 104 was prepared according to General Synthetic Method 4, wherein a yellow solid was afforded with a yield of 32.0%.

¹H NMR (400 MHz, DMSO-*d*₆) δ 7.89 (d, *J =* 8.3 Hz, 2H), 7.54 (d, *J =* 8.2 Hz, 2H), 3.44 (s, 3H), 2.63 (s, 3H).

### Example 102: Preparation of Compound 105

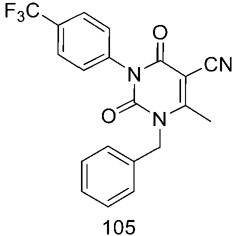

Compound 105 was prepared according to General Synthetic Method 4, wherein a white solid was afforded with a yield of 9.0%.

¹H NMR (600 MHz, DMSO-*d*₆) δ 7.90 (dd, *J =* 7.6, 1.3 Hz, 2H), 7.64 - 7.59 (m, 2H), 7.41 - 7.35 (m, 4H), 7.32 (ddt, *J =* 8.4, 5.8, 2.0 Hz, 1H), 5.25 (s, 2H), 2.53 (s, 3H).

### Example 103: Preparation of Compound 106

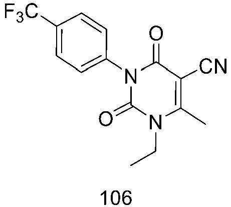

Compound 106 was prepared according to General Synthetic Method 4, wherein a yellow solid was afforded with a yield of 64.0%.

¹H NMR (400 MHz, CDCl3) δ 7.77 (d, *J =* 8.4 Hz, 2H), 7.36 (d, *J =* 8.3 Hz, 2H), 4.06 (q, *J =* 7.1 Hz, 2H), 2.70 (s, 3H), 1.38 (t, *J =* 7.1 Hz, 3H).

### Example 104: Preparation of Compound 107

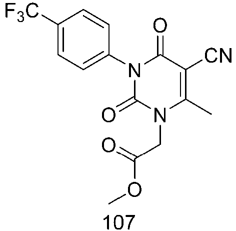

Compound 107 was prepared according to General Synthetic Method 4, wherein a white solid was afforded with a yield of 37.0%.

¹H NMR (400 MHz, DMSO-*d*₆) δ 7.90 (d, *J =* 8.4 Hz, 2H), 7.57 (d, *J =* 8.2 Hz, 2H), 4.85 (s, 2H), 3.73 (s, 3H), 2.61 (s, 3H).

### Example 105: Preparation of Compound 108

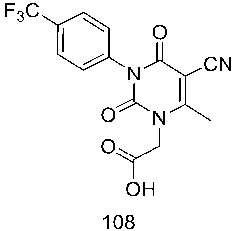

Compound 108 was prepared according to General Synthetic Method 4, wherein a pink solid was afforded with a yield of 46.0%.

¹H NMR (400 MHz, DMSO-*d*₆) δ 13.54 (s, 1H), 7.90 (d, *J =* 8.3 Hz, 2H), 7.57 (d, *J=* 8.2 Hz, 2H), 4.76 (s, 2H), 2.60 (s, 3H).

### Example 106: Preparation of Compound 109

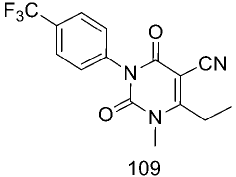

Compound 109 was prepared according to General Synthetic Method 4, wherein a white solid was afforded with a yield of 22.0%.

¹H NMR (600 MHz, DMSO-*d*₆) δ 7.89 (d, *J =* 8.4 Hz, 2H), 7.58 - 7.41 (m, 2H), 3.46 (s, 3H), 2.92 (q, *J* = 7.6 Hz, 2H), 1.30 (t, *J =* 7.6 Hz, 3H).

### Example 107: Preparation of Compound 110

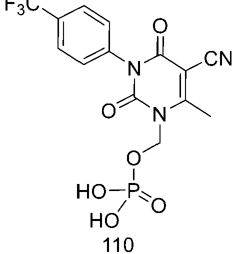

Compound 110 was prepared according to General Synthetic Method 4, wherein a white solid was afforded with a yield of 47.0%.

¹H NMR (400 MHz, DMSO-*d*₆) δ 7.90 (d, *J =* 8.4 Hz, 2H), 7.57 (d, *J =* 8.2 Hz, 2H), 5.67 (d, *J* = 7.8 Hz, 2H), 2.75 (s, 3H).

### Example 108: Preparation of Compound 111

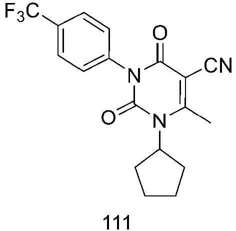

Compound 111 was prepared according to General Synthetic Method 4, wherein a white solid was afforded with a yield of 8.1%.

¹H NMR (400 MHz, DMSO-*d*₆) δ 7.88 (d, *J =* 8.4 Hz, 2H), 7.55 (s, 2H), 4.73 (p, *J =* 8.4 Hz, 1H), 2.70 (s, 3H), 2.07 - 1.86 (m, 4H), 1.78 (s, 2H), 1.49 (q, *J =* 5.6, 4.8 Hz, 2H).

### Example 109: Preparation of Compound 112

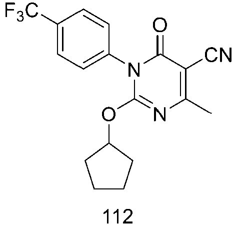

Compound 112 was prepared according to General Synthetic Method 4, wherein a white solid was afforded with a yield of 29.2%.

¹H NMR (400 MHz, DMSO-*d*₆) δ 8.04 - 7.87 (m, 2H), 7.73 - 7.52 (m, 2H), 5.51 (tt, *J* = 5.2, 2.3 Hz, 1H), 2.48 (s, 3H), 1.78 (dtd, *J =* 14.2, 8.1, 5.4 Hz, 2H), 1.70 - 1.54 (m, 2H), 1.47 (qq, *J =* 10.2, 4.6, 4.2 Hz, 2H), 1.34 (ddd, *J =* 12.5, 8.3, 4.7 Hz, 2H).

### Example 110: Preparation of Compound 113

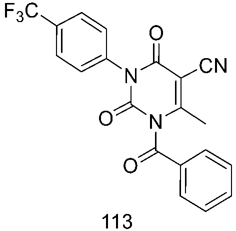

Compound 113 was prepared according to General Synthetic Method 4, wherein a white solid was afforded with a yield of 74.0%.

¹H NMR (400 MHz, DMSO-*d*₆) δ 8.31 - 8.22 (m, 2H), 7.88 (dd, *J =* 20.8, 7.9 Hz, 3H), 7.66 (t, *J =* 7.6 Hz, 4H), 2.42 (s, 3H).

### Example 111: Preparation of Compound 114

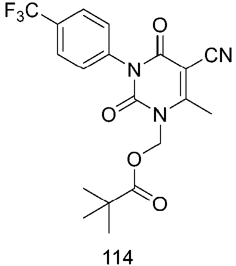

Compound 114 was prepared according to General Synthetic Method 4, wherein a white solid was afforded with a yield of 6.0%.

¹H NMR (400 MHz, DMSO-*d*₆) δ 7.91 (d, *J =* 8.4 Hz, 2H), 7.66 - 7.51 (m, 2H), 5.93 (s, 2H), 2.70 (s, 3H), 1.17 (s, 9H).

### Example 112: Preparation of Compound 115

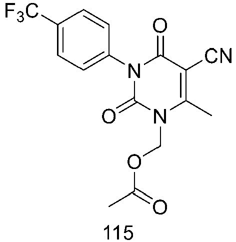

Compound 115 was prepared according to General Synthetic Method 4, wherein a white solid was afforded with a yield of 32.0%.

¹H NMR (400 MHz, DMSO-*d*₆) δ 7.90 (d, *J =* 8.4 Hz, 2H), 7.63 - 7.50 (m, 2H), 5.91 (s, 2H), 2.71 (s, 3H), 2.09 (s, 3H).

### Example 113: Preparation of Compound 116

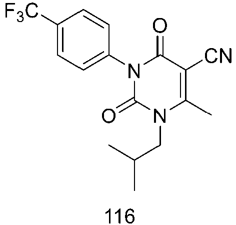

Compound 116 was prepared according to General Synthetic Method 4, wherein a yellow solid was afforded with a yield of 1.7%.

¹H NMR (400 MHz, DMSO-*d*₆) δ 7.89 (d, *J =* 8.3 Hz, 2H), 7.56 (d, *J =* 8.2 Hz, 2H), 3.79 (d, *J* = 7.5 Hz, 2H), 2.65 (s, 3H), 2.22-1.99 (m, 1H), 0.92 (d, *J =* 6.7 Hz, 6H).

### Example 114: Preparation of Compound 117

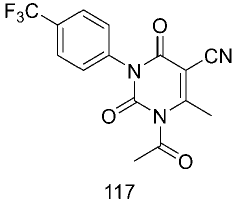

Compound 117 was prepared according to General Synthetic Method 4, wherein a white solid was afforded with a yield of 56.0%.

¹H NMR (400 MHz, CDCl₃) δ 7.78 (d, *J =* 8.3 Hz, 2H), 7.38 (d, *J =* 8.2 Hz, 2H), 2.71 (s, 3H), 2.56 (s, 3H).

### Example 115: Preparation of Compound 118

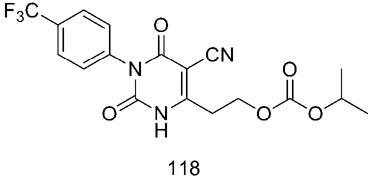

Compound 118 was prepared according to General Synthetic Method 4, wherein a white solid was afforded with a yield of 8.6%.

¹H NMR (400 MHz, DMSO-*d*₆) δ7.89 (d, *J =* 8.4 Hz, 2H), 7.58 (d, *J =* 8.2 Hz, 2H), 6.20 (s, 1H), 4.83 - 4.76 (m, 1H), 4.42 (t, *J =* 6.0 Hz, 2H), 3.01 (t, *J =* 6.0 Hz, 2H), 1.29 - 1.14 (m, 6H).

### Example 116: Preparation of Compound 119

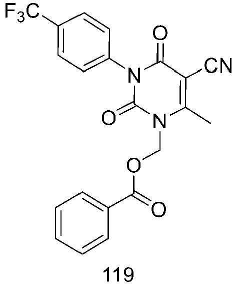

Compound 119 was prepared according to General Synthetic Method 4, wherein a white solid was afforded with a yield of 55.0%.

¹H NMR (400 MHz, DMSO-*d*₆) δ 8.03 (d, *J =* 7.8 Hz, 2H), 7.90 (d, *J =* 8.3 Hz, 2H), 7.71 (t, *J* = 7.4 Hz, 1H), 7.62 - 7.50 (m, 4H), 6.21 (s, 2H), 2.81 (s, 3H).

### Example 117: Preparation of Compound 120

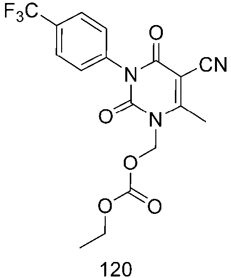

Compound 120 was prepared according to General Synthetic Method 4, wherein a yellow liquid was afforded with a yield of 97.0%.

¹H NMR (400 MHz, DMSO-*d*₆) δ 7.91 (d, *J =* 8.3 Hz, 2H), 7.58 (d, *J =* 8.2 Hz, 2H), 5.96 (s, 2H), 4.23 - 4.16 (m, 2H), 2.71 (s, 3H), 1.23 (t, *J* = 7.1 Hz, 3H).

### Example 118: Preparation of Compound 121

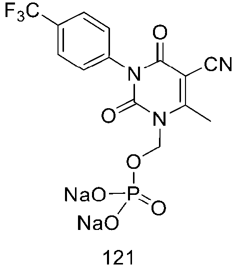

Compound 121 was prepared according to General Synthetic Method 4, wherein a white solid was afforded with a yield of 47.4%.

¹H NMR (400 MHz, CD₃OD) δ 7.80 (d, *J =* 8.4 Hz, 2H), 7.53 (d, *J =* 8.2 Hz, 2H), 3.28 (s, 3H), 5.70 (d, *J =* 8.9 Hz, 2H).

### Example 119: Preparation of Compound 122

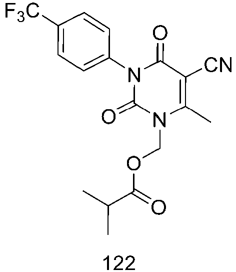

Compound 122 was prepared according to General Synthetic Method 4, wherein a white solid was afforded with a yield of 40.0%.

¹H NMR (400 MHz, DMSO-*d*₆) δ 7.91 (d, *J =* 8.4 Hz, 2H), 7.58 (d, *J =* 8.3 Hz, 2H), 5.93 (s, 2H), 2.70 (s, 3H), 2.66 - 2.57 (m, 1H), 1.11 (d, *J =* 7.0 Hz, 6H).

### Example 120: Preparation of Compound 123

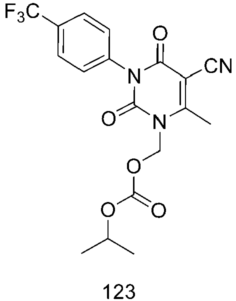

Compound 123 was prepared according to General Synthetic Method 4, wherein a yellow solid was afforded with a yield of 64.0%.

¹H NMR (400 MHz, DMSO-*d*₆) δ 7.91 (d, *J =* 8.4 Hz, 2H), 7.58 (d, *J =* 8.2 Hz, 2H), 5.96 (s, 2H), 4.81 (p, *J* = 6.2 Hz, 1H), 2.70 (s, 3H), 1.24 (d, *J* = 6.2 Hz, 6H).

### Test Examples

### Test Example 1: Distribution of test drugs in the brain and spinal cord of mice

Methods: Adult male ICR mice aged 6~8 weeks and weighing 30±20 g were randomly divided into groups with 3 mice per group. The mice were fasted for 12 h before the experiment while having free access to water. All groups were administered intragastrically at a dose of 50 mg/kg for Teriflunomide and other example groups; blood samples (0.5 mL) were collected via eyeball enucleation respectively at 0.5 h, 1 h, 2 h, and 4 h after administration and were put into centrifuge tube; after mice were sacrificed by cervical dislocation, they were dissected and their hearts, livers, spleens, lungs, kidneys, brains, stomachs, small intestines, thymuses, pancreases, and spinal cords were collected. These samples were rinsed with physiological saline to remove the surface blood and then were blotted, and approximately 50 mg of each sample was weighed and put into a homogenizing tube; 500 µL physiological saline was added, and homogenization parameters were set as 40 s*3 times with intervals of 1 min at a speed of 6.00 m/s and at a temperature of 4°C; after vortex homogeneity, a certain volume of the samples was taken and was diluted by one time with methanol, and then was diluted by four times with a mixed extractant of acetonitrile:methanol=7:3 containing 0.2% formic acid; after vortex oscillation for 3 min, the mixture was centrifuged at 13000 rpm for 10 min at 4°C, and 5 µL of the supernatant was taken for sample injection and analysis, wherein pharmacokinetic parameters of each example were calculated using Winnonlin software. The results are shown in Table 1.

Results: the exposure level of active metabolites of compounds in the present disclosure in mouse brain and spinal cord was significantly higher than that of Teriflunomide in brain and spinal cord. The mass spectrum fragment structure of "other active metabolites" in Table 1 is Compound 102:

**Table 1 Distribution Parameters of Test Drugs in Mouse Brain and Spinal Cord**

| **Test drugs** | **Time** | **Prototype drug** | | **Teriflunomide** | | **Other active metabolites** | |
|---|---|---|---|---|---|---|---|
| | | **Brain** | **Spinal cord** | **Brain** | **Spinal cord** | **Brain** | **Spinal cord** |
| **Teriflunomide** | 0.5 h | - | - | 3688.9 | | | |
| | 1 h | - | - | 3631.7 | | | |
| | 2 h | - | - | 3821.1 | | | |
| | 4 h | - | - | 5134.4 | | | |
| **Compound 6** | 0.5 h | 937.2 | 1465.5 | 16.7 | 238.6 | 12198. 3 | 20739.4 |
| | 1 h | 481.8 | 783.9 | 15.5 | 92.7 | 20236.5 | 32563.2 |
| | 2 h | 163.2 | 85.6 | 41.6 | 333.8 | 11703.1 | 14199.9 |
| | 4 h | 0.0 | 0.0 | 95. 3 | 449.5 | 4817.1 | 4960.9 |
| **Compound 28** | 0.5 h | 6251.1 | - | 145.9 | - | - | - |
| | 1 h | 5791.6 | - | 270.1 | - | - | - |
| | 2 h | 2256.3 | - | 819.7 | - | - | - |
| | 4 h | 147.2 | - | 974.7 | - | - | - |
| **Compound 56** | 0.5 h | - | - | - | - | 17832.7 | 129832.2 |
| | 1 h | - | - | - | - | 20741.4 | 117063.6 |
| | 2 h | - | - | - | - | 21177.7 | 78769.3 |
| | 4 h | - | - | - | - | 2223.6 | 16331.1 |
| **Compound 59** | 0.5 h | - | - | - | - | 31167.9 | 90437.1 |
| | 1 h | - | - | - | - | 9404.9 | 18772.2 |
| | 2 h | - | - | - | - | 13626.1 | 32229.6 |
| | 4 h | - | - | - | - | 2511.8 | 7196.3 |
| **Compound 61** | 0.5 h | - | - | - | - | 17804.6 | 51996.8 |
| | 1 h | - | - | - | - | 8213.9 | 24849.1 |
| | 2 h | - | - | - | - | 3123.6 | 3818.7 |
| | 4 h | - | - | - | - | 230.8 | 1271.4 |
| **Compound 62** | 0.5 h | - | - | - | - | 9364.0 | 59524.2 |
| | 1 h | - | - | - | - | 6342.3 | 19321.6 |
| | 2 h | - | - | - | - | 12073.5 | 30020.7 |
| | 4 h | - | - | - | - | 2217.7 | 4963.4 |
| **Compound 64** | 0.5 h | - | - | - | - | 12659.5 | 42765.3 |
| | 1 h | - | - | - | - | 9440.7 | 19060.7 |
| | 2 h | - | - | - | - | 2792.6 | 10532.6 |
| | 4 h | - | - | - | - | 1603.4 | 5489.9 |
| **Compound 65** | 0.5 h | - | - | - | - | 19588.4 | 35588.9 |
| | 1 h | - | - | - | - | 13845.9 | 35008.1 |
| | 2 h | - | - | - | - | 2919.8 | 10342.3 |
| | 4 h | - | - | - | - | 1253.6 | 5741.5 |
| **Compound 67** | 0.5 h | - | - | - | - | 30852.2 | 55585.9 |
| | 1 h | - | - | - | - | 15921.4 | 39118.5 |
| | 2 h | - | - | - | - | 13822.5 | 30402.8 |
| | 4 h | - | - | - | - | 5487.6 | 7914.7 |
| **Compound 68** | 0.5 h | | | | | 18670.2 | 37758.1 |
| | 1 h | | | | | 16850.0 | 35909.6 |
| | 2 h | | | | | 12172.9 | 12396.9 |
| | 4 h | | | | | 1811.8 | 3291.7 |
| **Compound 69** | 0.5 h | | | | | 6785.5 | 26197.9 |
| | 1 h | | | | | 6264.8 | 11888.2 |
| | 2 h | | | | | 2638.1 | 4084.8 |
| | 4 h | | | | | 2484.7 | 1131.1 |
| **Compound 70** | 0.5 h | | | | | 10756.5 | 34110.7 |
| | 1 h | | | | | 17996.8 | 82104.3 |
| | 2 h | | | | | 9122.4 | 31976.3 |
| | 4 h | | | | | 4753.2 | 17462.9 |

### Test Example 2: Tolerance Study of Test Drugs in Mice

Method: SPF-grade male ICR mice weighing 30±2 g were randomly divided into groups, with 6 mice per group, half males and half females. The control drug Teriflunomide and compounds of the present disclosure were administered respectively and intraperitoneally once daily for 4 consecutive days at a dosing volume of 10 µL/g. Status and mortality of mice were monitored within 4 hours post-administration, and prolonged observation was conducted for 7 consecutive days following drug discontinuation. Body weight, motor coordination, urination and defecation, mental state, eyelid signs, respiration, fur condition and other statuses were observed and recorded over the 4 days.

**Table 2 Tolerance Experiments of Test Drugs**

| **Test drugs** | **Administration dosage** | **Clinical observation** | **Tolerance dose** |
|---|---|---|---|
| **Teriflunomide** | 70 mg/kg | All died on the third day after administration. | < 70 mg/kg |
| Compound 16 | 200 mg/kg | No adverse reactions. | > 200 mg/kg |
| Compound 24 | 200 mg/kg | No adverse reactions. | > 200 mg/kg |
| Compound 37 | 200 mg/kg | No adverse reactions. | > 200 mg/kg |
| Compound 44 | 200 mg/kg | No adverse reactions. | > 200 mg/kg |
| Compound 54 | 200 mg/kg | No adverse reactions. | > 200 mg/kg |
| Compound 59 | 200 mg/kg | No adverse reactions. | > 200 mg/kg |
| Compound 67 | 200 mg/kg | No adverse reactions. | > 200 mg/kg |
| Compound 102 | 200 mg/kg | No adverse reactions. | > 200 mg/kg |
| Compound 104 | 200 mg/kg | No adverse reactions. | > 200 mg/kg |
| Compound 105 | 200 mg/kg | No adverse reactions. | > 200 mg/kg |
| Compound 106 | 200 mg/kg | No adverse reactions. | > 200 mg/kg |
| Compound 107 | 200 mg/kg | No adverse reactions. | > 200 mg/kg |
| Compound 109 | 200 mg/kg | No adverse reactions. | > 200 mg/kg |
| Compound 110 | 200 mg/kg | No adverse reactions. | > 200 mg/kg |
| Compound 112 | 200 mg/kg | No adverse reactions. | > 200 mg/kg |
| Compound 113 | 200 mg/kg | No adverse reactions. | > 200 mg/kg |
| Compound 114 | 200 mg/kg | No adverse reactions. | > 200 mg/kg |
| Compound 115 | 200 mg/kg | No adverse reactions. | > 200 mg/kg |
| Compound 116 | 200 mg/kg | No adverse reactions. | > 200 mg/kg |
| Compound 117 | 200 mg/kg | No adverse reactions. | > 200 mg/kg |
| Compound 118 | 200 mg/kg | No adverse reactions. | > 200 mg/kg |
| Compound 119 | 200 mg/kg | No adverse reactions. | > 200 mg/kg |
| Compound 120 | 200 mg/kg | No adverse reactions. | > 200 mg/kg |
| Compound 121 | 200 mg/kg | No adverse reactions. | > 200 mg/kg |

### Test Example 3: Effects on Acetic Acid-induced Writhing Response in Mice

Method: Kunming mice, aged 6~8 weeks, weighing 20±2 g, with equal numbers of males and females, 8 mice per group. The mice were respectively divided into a normal saline group by oral administration (the model group), a compound group of the present disclosure, and a Teriflunomide control group, all at a dose of 40 mg/kg and a dosing volume of 10 µL/g. Thirty minutes after administration, each mouse was injected intraperitoneally with 0.2 mL of 0.6% acetic acid solution. The number of writhing responses (positive writhing was defined as abdominal concave accompanied by trunk twisting, hip elevation and hind limb extension) within 20 min was observed and recorded, and the writhing inhibition rate was calculated.

Writhing inhibition rate (%)=(Mean writhing number of control group-Mean writhing number of administration group)/Mean writhing number of control group×100%.

Results: all compounds of the present disclosure in Table 3 exhibited better analgesic effects than Teriflunomide.

**Table 3. Comparison of Acetic Acid-induced Writhing Responses in Mice of Each Group (x̅±s)**

| Groups | Number of animals (head) | Writhing times within 20 min | Writhing inhibition rate (%) |
|---|---|---|---|
| Blank control group | 8 | 34.4±9.4 | - |
| Teriflunomide | 8 | 15.1±11.2** | 56.0 |
| Compound 1 | 8 | 8.9±9.2*** | 74.2 |
| Compound 2 | 8 | 9.4±6.3*** | 72.7 |
| Compound 3 | 8 | 9.3±6.3** | 73.1 |
| Compound 4 | 8 | 8.3±10.1*** | 76.0 |
| Compound 6 | 8 | 4.0±4.5*** | 88.4 |
| Compound 12 | 8 | 7.7±5.5*** | 77.6 |
| Compound 13 | 8 | 8.0±5.4*** | 76.7 |
| Compound 19 | 8 | 6.6±7.6*** | 80.9 |
| Compound 24 | 8 | 8.8±7.0*** | 74.5 |
| Compound 28 | 8 | 10.3±3.5*** | 70.1 |
| Compound 30 | 8 | 9.1±8.5*** | 73.5 |
| Compound 39 | 8 | 8.1±7.1*** | 76.4 |
| Compound 40 | 8 | 6.4±6.6*** | 81.3 |
| Compound 41 | 8 | 8.1±5.1*** | 76.4 |
| Compound 48 | 8 | 7.3±5.6*** | 78.8 |
| Compound 49 | 8 | 6.0±5.8*** | 82.5 |
| Compound 54 | 8 | 9.3±8.0*** | 73.1 |
| Compound 61 | 8 | 6.9±9.1*** | 80.0 |
| Compound 109 | 8 | 8.8±5.7*** | 74.4 |
| Compound 110 | 8 | 8.3±5.2*** | 75.9 |
| Compound 112 | 8 | 10.8+3.2*** | 68.6 |

### Test Example 4: Effects on Experimental Autoimmune Encephalomyelitis (EAE) Mice

Method: The antigen MOG35-55 was diluted to 2 mg/mL with phosphate-buffered saline, and was mixed with an equal volume (1:1) of complete Freund's adjuvant (the final concentration of H37Ra being 5 mg/mL) to form an emulsion. After emulsification, female C57BL/6 mice, aged 8 to 10 weeks and weighing 18 to 20 g, were anesthetized and fixed, and 0.2 mL of the emulsion was subcutaneously injected at two sites on both sides of the spine per mouse; and, on the day of immunization and 48 h later, 0.25 mL of PTX (250 ng per mouse) was intraperitoneally injected twice, thereby establishing the mouse EAE model.

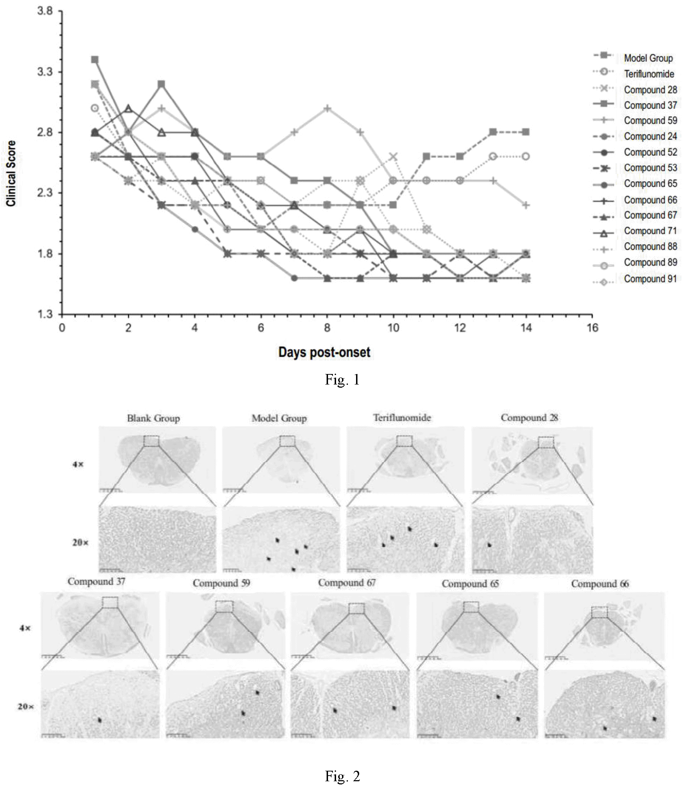

After disease onset, the mice were divided into groups, which were respectively a normal saline intragastric administration group (once daily), a compound group of the present disclosure (Compound 24, Compound 28, Compound 37, Compound 52, Compound 53, Compound 59, Compound 65, Compound 66, Compound 67, Compound 71, Compound 88, Compound 89, Compound 91), and a Teriflunomide control group, all at a dose of 5 mg/kg and a dosing volume of 10 µL/g for 14 consecutive days; mouse behaviors (such as fur conditions, activities, diet, etc.) were observed at the same time each day, body weights were measured and recorded every other day, and neurological functions were scored according to the Kono 5-point scale (0: no disease; 1: tail weakness; 2: mild hindlimb weakness; 3: severe hindlimb paralysis; 4: tetraplegia; 5: moribund state or death). The neurological function scores of mice are shown in Fig. 1.

After 14 days of administration, serum, brain tissue and spinal cord were collected for pathological examination and detection of inflammatory factors IL-17A and IFN-γ.

As shown in Fig. 2, the results of LFB staining revealed that the myelin structure of spinal cord tissue in the normal group was clear and intact without demyelination. In the model group, demyelinated areas of varying sizes were observed in the spinal cord tissue, presenting as lightly stained blue regions or unstained white regions, accompanied by myelin sheath loosening of varying degrees. Compared with the model group, the lightly stained blue regions or unstained white regions in the spinal cord tissue of the test drug groups (Compound 28, Compound 65, Compound 66) were significantly reduced, and the demyelinated areas in Compound 28, 37, 59, 65, 66 and 67 groups were significantly smaller than those in the Teriflunomide (approved drug) group.

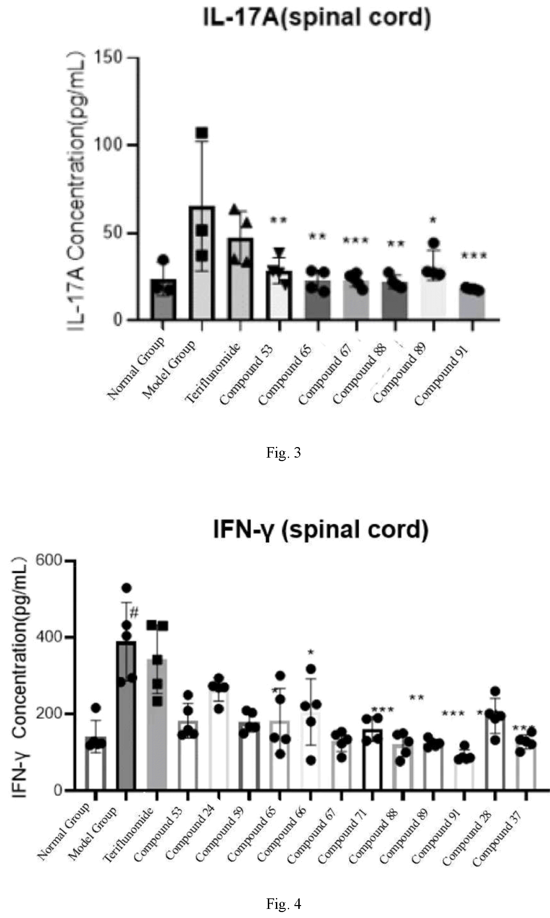

As shown in Fig. 3, the detection results of the inflammatory factor IL-17A indicated that the IL-17A level in the model group was significantly higher than that of the normal group, Teriflunomide reduced the expression of IL-17A in mice; Compounds 53, 65, 67, 88, 89 and 91 of the present disclosure significantly decreased the IL-17A level in spinal compared with the model group, and showed obvious advantages over Teriflunomide.

As shown in Fig. 4, the detection result of the inflammatory factor IFN-γ indicated that the inflammatory level of IFN-γ in the model group was remarkably higher than that of the normal group, Teriflunomide decreased the expression of IFN-γ in mouse spinal cord; Compounds 24, 28, 37, 53, 59, 65, 66, 67, 71, 88, 89 and 91 of the present disclosure significantly reduced the IFN-γ level in spinal compared with the model group, and showed obvious advantages over Teriflunomide.

Results: compared with Teriflunomide, the compounds of the present disclosure can significantly alleviate clinical symptoms in EAE model mice with multiple sclerosis and neuromyelitis optica, reduce the levels of inflammatory factors IL-17A and IFN-y, and decrease demyelinated areas in the spinal cord. Therefore, the compounds of the present disclosure exert more remarkable therapeutic effects on central nervous system diseases such as multiple sclerosis and neuromyelitis optica.

### Test example 5: anticancer activity

Method: The CellTiter-Glo^{®} Luminescent Cell Viability Assay was adopted. Tumor cells in logarithmic phase were collected, the cell suspension concentration was adjusted, and cells were seeded in black clear-bottom 96-well plates (786-O cells: 1200 cells/well; A549 cells: 1500 cells/well; A498, PC3 and DU145 cells: 3000 cells/well; H460 cells: 1500 cells/well; LNCaP cells: 3500 cells/well); after overnight culture, test compounds were added to the wells, and the plates were incubated in a 37°C incubator for 72 h; then the culture medium was removed, cell viability reagent was added, and the mixture was shaken at 150 rpm/min for 2 min; and, after incubation at room temperature (25°C) for 10 min, chemiluminescence intensity was immediately detected using a microplate reader. Cell viability was calculated based on the chemiluminescence intensity measured by the microplate reader, using the following formula:$Cell viability \left(%\right) = \left(Treatment group-Blank group\right) / \left(Control group-Blank group\right) \times 100 %$

Results: the compounds of the present disclosure listed in Table 4 exhibited superior anticancer efficacy compared with Teriflunomide.

**Table 4. Anticancer Activity of Compounds of the Present Disclosure**

| Compounds | IC₅₀ (µM) | | | | | |
|---|---|---|---|---|---|---|
| | 786-O | LNCaP | A549 | PC3 | DU145 | H460 |
| Leflunomide | 6.67 | 5.43 | 7.86 | 9.16 | 4.65 | 5.43 |
| Teriflunomide | 2.65 | 4.60 | 6.92 | 4.81 | 3.54 | 2.79 |
| Compound 12 | 1.22 | 2.93 | 4.03 | 2.33 | 2.68 | 1.59 |
| Compound 33 | 1.25 | 3.01 | 4.96 | 4.61 | 3.32 | 1.07 |
| Compound 35 | 1.09 | 1.99 | 5.02 | 3.79 | 2.89 | 1.23 |
| Compound 36 | 1.49 | 0.37 | 5.51 | 2.57 | 2.58 | 1.17 |
| Compound 46 | 1.99 | 0.84 | 2.66 | 1.95 | 2.64 | 1.12 |

### Test Example 6: Inhibition of proliferation in rat splenocytes stimulated by Lipopolysaccharide (LPS)

Method: Splenocytes were taken from 11-week-old female SD rats and were seeded in 96-well plates at a density of 2.5×10⁶ cells/mL, followed by overnight culture. DMSO, Teriflunomide, and 50 µL of test drugs (Compound 6, Compound 20, Compound 24, Compound 52, Compound 53, Compound 56, Compound 59, Compound 66, Compound 67, Compound 88, Compound 91, Compound 113, Compound 114, Compound 115, Compound 118) (at concentrations of 0.1, 1, 10, and 100 µM) were added to the wells. After incubation in a 37°C incubator for 2 h, the cells were treated with 10 µg/mL LPS for 24 h, followed by MTT assay to calculate cell viability. Cell viability was calculated according to the values measured by the microplate reader, using the following formula: $Cell viability \left(%\right) = \left({OD}_{Treatment group}-{OD}_{Blank group}\right) / \left({OD}_{Control group}-{OD}_{Blank group}\right) \times 100 %$

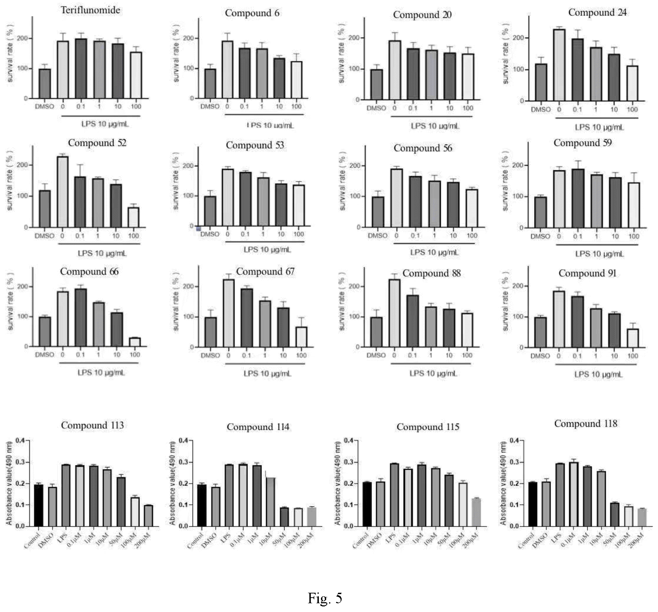

As shown in Fig. 5, the results showed that compounds of the present disclosure were more effective than Teriflunomide for inhibiting lymphocyte proliferation.

## Claims

1. A compound represented by Formula (I), or an optical isomer thereof, or a pharmaceutically acceptable salt thereof, or a solvate thereof (optionally, the solvate is a hydrate), or an inclusion complex thereof, or a racemate thereof, or a co-crystal thereof, or an isotope-labeled compound thereof, or nitrogen oxides thereof, or a prodrug thereof, or an active metabolite thereof, 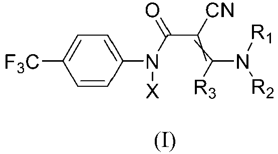 wherein:
R₁ is selected from: -H, alkyl, -alkylene-C(=O)O-alkyl, -S(=O)₂R₄₋₁, optionally substituted monosaccharide group, -C(=O)R₄₋₂, -C(=O)N(R₄₋₃)R₄₋₄, -O-R₄₋₅, -A-Linker-R₄₋₆, and -P(=O)(OR₄₋₇)OR₄₋₈;
R₂ is selected from: -H, alkyl, aryl, cycloalkyl, -alkylene-aryl, -alkylene-C(=O)OH, -alkylene-C(=O)O-alkyl, -S(=O)₂R₄₋₁, optionally substituted monosaccharide group, -C(=O)R₄₋₂, -C(=O)N(R₄₋₃)R₄₋₄, -O-R₄₋₅, -A-Linker-R₄₋₆, -P(=O)(OR₄₋₇)OR₄₋₈, -alkylene-O-C(=O)R₄₋₁₁, -alkylene-O-P(=O) (OR₄₋₇)OR₄₋₈, and -alkylene-O-C(=O)-OR₄₋₁₀;
alternatively, R₁ and R₂ are interconnected and, together with nitrogen atoms to which they are attached, jointly form an optionally substituted aliphatic heterocyclyl group;
alternatively, R₁ and R₂, together with nitrogen atoms to which they are attached, are represented as: 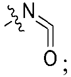
X is selected from -H, or X and R₁ jointly form a cyclic amide structure; 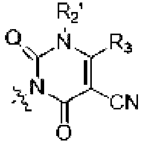
R₂' is selected from: -H, alkyl, aryl, cycloalkyl, -alkylene-aryl, -alkylene-C(=O)OH, -alkylene-C(=O)O-alkyl, -S(=O)₂R₄₋₁, optionally substituted monosaccharide group, -C(=O)R₄₋₂, -C(=O)N(R₄₋₃)R₄₋₄, -O-R₄₋₅, -A-Linker-R₄₋₆, -P(=O)(OR₄₋₇)OR₄₋₈, -alkylene-O-C(=O)R₄₋₁₁, -alkylene-O-P(=O) (OR₄₋₇)OR₄₋₈, and -alkylene-O-C(=O)-OR₄₋₁₀;
Q is selected from O, and -O-cycloalkyl;
R₃ is individually and independently selected from: -H, and alkyl or -alkylene-O-C(=O)-OR₄₋₂;
A is selected from: -C(=O)-, and alkylene;
R₄₋₁ is selected from: alkyl, hydroxy, amino, carboxy, halogen, nitro, and cyano; preferably, R₄₋₁ is alkyl;
R₄₋₂ is selected from: -OH, aryl, alkyl, alkoxy, -alkenyl-carboxy, -alkenyl-alkoxycarbonyl, and -alkylene-R₄₋₉;
R₄₋₃ and R₄₋₄ are the same or different, and are individually and independently selected from H, and alkyl;
alternatively, R₄₋₃ and R₄₋₄ are interconnected and, together with nitrogen atoms to which they are attached, jointly form an optionally substituted aliphatic heterocyclyl group;
R₄₋₅ is selected from: H, alkyl, and -alkylene-aryl;
Linker is an optionally present group, and, when present, is selected from: alkylene, -B-alkylene-, -B-alkylene-arylene-, and -B-arylene-alkylene-;
each B is individually and independently selected from -O-, -S-, and -NH-;
R4-6 is 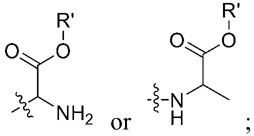
each R' is individually and independently selected from: -H, alkyl, -alkylene-aryl, and hydroxy-substituted alkyl;
each R₄₋₇ and R₄₋₈ are the same or different, and are individually and independently selected from H, alkyl, and -alkylene-aryl;
R₄₋₉ is selected from: 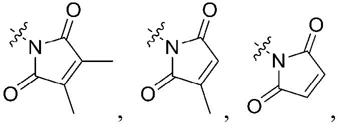 aryl, and -C(=O)O-alkyl;
the "optionally substituted monosaccharide group" means that one or more hydrogen atoms on a monosaccharide group are unsubstituted or are substituted with a substituent selected from: -C(=O)OR₄₋₁₀;
the monosaccharide group in the optionally substituted monosaccharide group is selected from the following monosaccharide groups, wherein 1-position substitution of the monosaccharide is α-substitution, or β-substitution, or both: 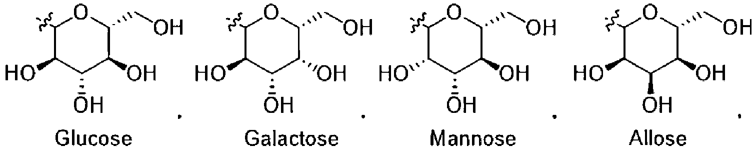 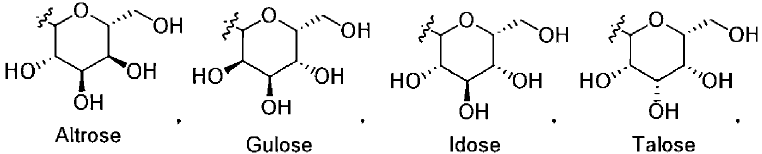 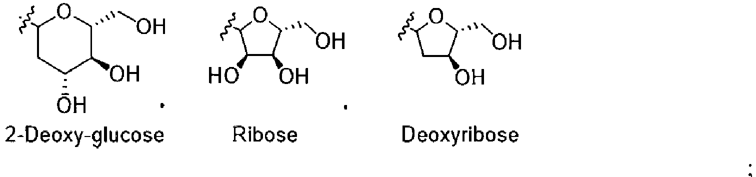
2-position substitution of the monosaccharidei is α-configuration, β-configuration, or both: 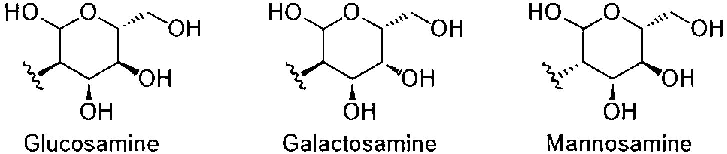
each R₄₋₁₀ is selected from: H, and alkyl;
R₄₋₁₁ is selected from: alkyl, and aryl;
"optionally substituted aliphatic heterocyclyl group" means that one or more hydrogen atoms on the aliphatic heterocyclyl group are unsubstituted or are substituted with substituents, and the substituents in the "optionally substituted aliphatic heterocyclyl group" are individually and independently selected from: hydroxy, amino, carboxy, halogen, nitro, cyano, alkyl, alkylthio, alkanoyl, and hydroxy-substituted aryl; and
the "hydroxy-substituted alkyl" means that one or more hydrogen atoms on the alkyl are unsubstituted or are substituted with hydroxy.

2. The compound represented by Formula (I) according to claim 1, or an optical isomer thereof, or a pharmaceutically acceptable salt thereof, or a solvate thereof (optionally, the solvate is a hydrate), or an inclusion complex thereof, or a racemate thereof, or a co-crystal thereof, or an isotope-labeled compound thereof, or nitrogen oxides thereof, or a prodrug thereof, or an active metabolite thereof, 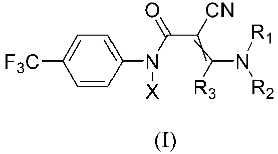 wherein:
R₁ and R₂ are the same or different, and are individually and independently selected from: -H, alkyl, -alkylene-C(=O)O-alkyl, -S(=O)₂R₄₋₁, optionally substituted monosaccharide group, -C(=O)R₄₋₂, -C(=O)N(R₄₋₃)R₄₋₄, -O-R₄₋₅, -A-Linker-R₄₋₆, and -P(=O)(OR₄₋₇)OR₄₋₈;
alternatively, R₁ and R₂ are interconnected and, together with nitrogen atoms to which they are attached, jointly form an optionally substituted aliphatic heterocyclyl group;
alternatively, R₁ and R₂, together with nitrogen atoms to which they are attached, are 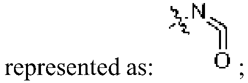
X is selected from -H, or X and R₁ jointly form a cyclic amide structure 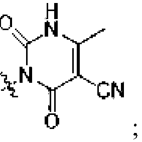
R₃ is selected from: -H, and alkyl;
A is selected from: -C(=O)-, and alkylene;
R₄₋₁ is selected from: alkyl, hydroxy, amino, carboxy, halogen, nitro, and cyano; preferably, R₄₋₁ is alkyl;
R₄₋₂ is selected from: -OH, aryl, alkyl, alkoxy, -alkenyl-carboxy, -alkenyl-alkoxycarbonyl, and -alkylene-R₄₋₉;
R₄₋₃ and R₄₋₄ are the same or different, and are individually and independently selected from H, and alkyl;
alternatively, R₄₋₃ and R₄₋₄ are interconnected and, together with nitrogen atoms to which they are attached, jointly form an optionally substituted aliphatic heterocyclyl group;
R₄₋₅ is selected from: H, alkyl, and aryl;
Linker is an optionally present group, and, when present, is selected from: alkylene, -B-alkylene-, -B-alkylene-arylene-, and -B-arylene-alkylene-;
each B is individually and independently selected from -O-, -S-, and -NH-;
R4-6 is 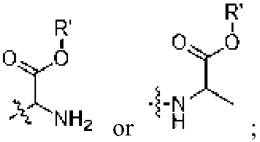
each R' is individually and independently selected from: -H, alkyl, -alkylene-aryl, and hydroxy-substituted alkyl;
R₄₋₇ and R₄₋₈ are the same or different, and are individually and independently selected from H, alkyl, and -alkylene-aryl;
R₄₋₉ is selected from: 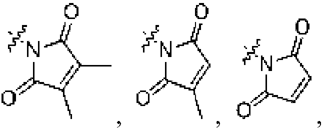 aryl, and -C(=O)O-alkyl;
the "optionally substituted monosaccharide group" means that one or more hydrogen atoms on a monosaccharide group are unsubstituted or are substituted with a substituent selected from: -C(=O)OR₄₋₁₀;
the monosaccharide group in the optionally substituted monosaccharide group is selected from the following monosaccharide groups, wherein 1-position substitution of the monosaccharide is α-substitution, or β-substitution, or both: 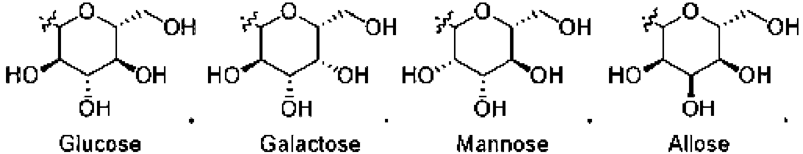 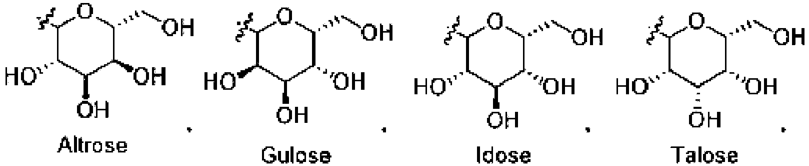 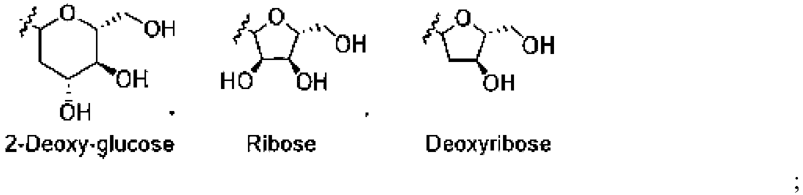
2-position substitution of the monosaccharidei is α-configuration, β-configuration, or both: 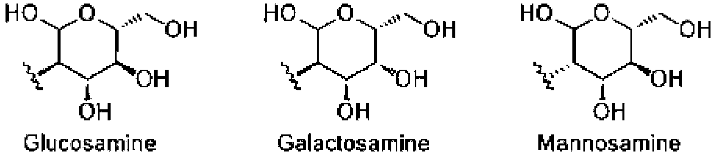
R₄₋₁₀ is selected from: H, and alkyl;
substituents in the "optionally substituted aliphatic heterocyclyl group" are individually and independently selected from: hydroxy, amino, carboxy, halogen, nitro, cyano, alkyl, alkylthio, alkanoyl, and hydroxy-substituted aryl; and
the "hydroxy-substituted alkyl" means that one or more hydrogen atoms on the alkyl are unsubstituted or are substituted with hydroxy.

3. The compound represented by Formula (I) according to claim 1 or 2, or an optical isomer thereof, or a pharmaceutically acceptable salt thereof, or a solvate thereof (optionally, the solvate is a hydrate), or an inclusion complex thereof, or a racemate thereof, or a co-crystal thereof, or an isotope-labeled compound thereof, or nitrogen oxides thereof, or a prodrug thereof, or an active metabolite thereof, wherein:
optionally, the halogen is selected from F, Cl, Br, and I;
optionally, the alkyl moieties in "alkyl", "alkanoyl", "hydroxy-substituted alkyl", "alkoxy", "-C(=O)O-alkyl", and "alkoxycarbonyl" are individually and independently a C₁₋₂₀ straight or branched alkyl, optionally a C₁₋₁₇ straight or branched alkyl, optionally a C₁₋₁₃ straight or branched alkyl, optionally a C₁₋₁₀ straight or branched alkyl, optionally a C₁₋₇ straight or branched alkyl, optionally a C₁₋₅ straight or branched alkyl, optionally methyl, ethyl, n-propyl, isopropyl, n-butyl, isobutyl, tert-butyl, sec-butyl, n-pentyl, isopentyl, neopentyl, tert-pentyl, n-hexyl, isohexyl, heptyl, n-octyl, n-nonyl, n-decyl, undecyl, dodecyl, tridecyl, tetradecyl, pentadecyl, hexadecyl, or heptadecyl; and optionally methyl, ethyl, n-propyl, isopropyl, n-butyl, isobutyl, tert-butyl, sec-butyl;
optionally, the alkyl moieties in the "-alkylene-C(=O)O-alkyl" are individually and independently a C₁₋₂₀ straight or branched alkyl, optionally a C₁₋₁₇ straight or branched alkyl, optionally a C₁₋₁₃ straight or branched alkyl, optionally a C₁₋₁₀ straight or branched alkyl, optionally a C₁₋₇ straight or branched alkyl, optionally a C₁₋₅ straight or branched alkyl, optionally methyl, ethyl, n-propyl, isopropyl, n-butyl, isobutyl, tert-butyl, sec-butyl, n-pentyl, isopentyl, neopentyl, tert-pentyl, n-hexyl, isohexyl, heptyl, n-octyl, n-nonyl, n-decyl, undecyl, dodecyl, tridecyl, tetradecyl, pentadecyl, hexadecyl, or heptadecyl; and optionally methyl, ethyl, n-propyl, isopropyl, n-butyl, isobutyl, tert-butyl, sec-butyl;
optionally, the "alkylene" in "-alkylene-", "-alkylene-aryl", "-B-alkylene-", "-B-alkylene-arylene-", "-B-arylene-alkylene-", "-O-alkylene-R₄₋₅", and "-alkylene-R₄₋₉" is a C₁₋₂₀ straight or branched alkylene, optionally a C₁₋₁₇ straight or branched alkylene, optionally a C₁₋₁₀ straight or branched alkylene, optionally a C₁₋₈ straight or branched alkylene, optionally a C₁₋₅ straight or branched alkylene, optionally a C₁₋₃ straight or branched alkylene, and optionally methylene, ethylene, isopropylene, n-propylene, isopropylene, n-butylene, isobutylene, tert-butylene, sec-butylene, n-pentylene, isopentylene, neopentylene, tert-pentylene, n-hexylene, isohexylene, heptylene, n-octylene, n-nonylene, n-decylene;
optionally, the "alkylene" in the "-alkylene-C(=O)O-alkyl", "-alkylene-O-C(=O)R₄₋₁₁", "-alkylene-O-P(=O)(OR₄₋₇)OR₄₋₈", "-alkylene-O-C(=O)-OR₄₋₁₀", "-alkylene-O-C(=O)-OR₄₋₂", and "-alkylene-C(=O)OH" is a C₁₋₂₀ straight or branched alkylene, optionally a C₁₋₁₇ straight or branched alkylene, optionally a C₁₋₁₀ straight or branched alkylene, optionally a C₁₋₅ straight or branched alkylene, optionally a C₁₋₅ straight or branched alkylene, optionally a C₁₋₃ straight or branched alkylene, and optionally methylene, ethylene, isopropylene, n-propylene, isopropylene, n-butylene, isobutylene, tert-butylene, sec-butylene, n-pentylene, isopentylene, neopentylene, tert-pentylene, n-hexylene, isohexylene, heptylene, n-octylene, n-nonylene, n-decylene;
optionally, the aliphatic heterocycle in the "aliphatic heterocyclyl group" is a C₃₋₈ (preferably C₄₋₆) aliphatic heterocycle containing 1 to 3 heteroatoms selected from O, N, and S on the ring, and optionally dioxolane, aziridinyl, azetidinyl, pyrrolidinyl, morpholinyl, piperidinyl, or piperazinyl;
optionally, the aryl in the "aryl", "hydroxy-substituted aryl", and "-alkylene-aryl" is a 6- to 10-membered monocyclic or bicyclic fused aromatic ring group; and optionally phenyl or naphthyl;
optionally, the arylene in the "-B-alkylene-arylene-", "-B-arylene-alkylene-", and "-alkylene-arylene-" is a 6- to 10-membered monocyclic or bicyclic fused aromatic ring group; and optionally phenylene or naphthylene;
optionally, the alkenyl moieties in the "alkenyl-carboxy" and "alkenyl-alkoxycarbonyl" are individually and independently a C₂-C₈ straight or branched alkenyl containing one or more double bonds, optionally a C₂-C₆ straight or branched alkenyl, optionally a C₂-C₄ straight or branched alkenyl; and optionally ethenyl, propenyl, butenyl, pentenyl, ethynyl, and hexynyl;
optionally, the cycloalkyl in the "cycloalkyl" and "-O-cycloalkyl" is a 3- to 7-membered monocyclic cycloalkyl, and optionally selected from: cyclopropyl, cyclobutyl, cyclopentyl, and cyclohexyl; and
optionally, the N-(4-trifluoromethyl)-2-cyanocrotonamide moiety is of Z-configuration or E-configuration.

4. The compound represented by Formula (I) according to any one of claims 1 to 3, or an optical isomer thereof, or a pharmaceutically acceptable salt thereof, or a solvate thereof (optionally, the solvate is a hydrate), or an inclusion complex thereof, or a racemate thereof, or a co-crystal thereof, or an isotope-labeled compound thereof, or nitrogen oxides thereof, or a prodrug thereof, or an active metabolite thereof, wherein:
optionally, when R₁ and R₂ are interconnected and, together with nitrogen atoms to which they are attached, jointly form an optionally substituted aliphatic heterocyclyl group, the optionally substituted aliphatic heterocyclyl group is selected from: 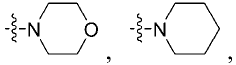 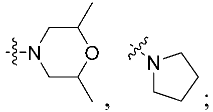
optionally, R₄₋₁ is C₁₋₅ straight or branched alkyl;
optionally, R₄₋₂ is selected from: -OH, phenyl, C₁₋₁₃ straight or branched alkyl, C₁₋₅ straight or branched alkoxy, -C=C-COOH, -C=C-C₁₋₅ straight or branched alkoxycarbonyl, -C₁₋₅ straight or branched alkylene-R₄₋₉;
optionally, R₄₋₃ and R₄₋₄ are the same or different, and are individually and independently selected from H, and alkyl; alternatively, R₄₋₃ and R₄₋₄ are interconnected and, together with nitrogen atoms to which they are attached, jointly form 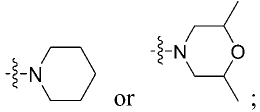
optionally, R₄₋₅ is selected from: H, C₁₋₅ straight or branched alkyl, -C₁₋₃ straight or branched alkylene-phenyl;
optionally, Linker is an optionally present group, and, when present, is selected from: C₁₋₅ straight or branched alkylene, -B-C₁₋₅ straight or branched alkylene-, -B-C₁₋₅ straight or branched alkylene-phenylene-, -B-phenylene-C₁₋₅ straight or branched alkylene-;
optionally, R' is individually and independently selected from: -H, C₁₋₅ straight or branched alkyl, -C₁₋₅ straight or branched alkylene-phenyl, hydroxy-substituted C₁₋₅ straight or branched alkyl;
optionally, the amino acid moiety of R₄₋₆ is of D-configuration or L-configuration;
optionally, R₄₋₇ and R₄₋₈ are the same or different, and are individually and independently selected from: H, C₁₋₅ straight or branched alkyl, -C₁₋₅ straight or branched alkylene-phenyl;
optionally, R₄₋₉ is selected from: 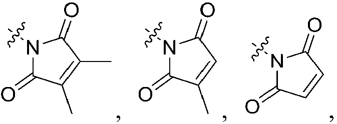 phenyl;
optionally, R₄₋₁₀ is selected from: H, C₁₋₅ straight or branched alkyl;
optionally, R₃ is selected from: -H, C₁₋₅ straight or branched alkyl;
optionally, A is selected from: -C(=O)-, C₁₋₅ straight or branched alkylene;
optionally, R₄₋₁₁ is selected from: H, C₁₋₅ straight or branched alkyl, phenyl; and
optionally, Q is O or -O-cyclopentyl.

5. The compound represented by Formula (I) according to any one of claims 2 to 4, or an optical isomer thereof, or a pharmaceutically acceptable salt thereof, or a solvate thereof (optionally, the solvate is a hydrate), or an inclusion complex thereof, or a racemate thereof, or a co-crystal thereof, or an isotope-labeled compound thereof, or nitrogen oxides thereof, or a prodrug thereof, or an active metabolite thereof, wherein:
R₁ and R₂ are the same or different, and are individually and independently selected from: -H, C₁₋₅ straight or branched alkyl, -C₁₋₅ straight or branched alkylene-C(=O)O-C₁₋₅ straight or branched alkyl, -S(=O)₂-C₁₋₅ straight or branched alkyl, -C(=O) R₄₋₂, -C(=O)N(R₄₋₃)R₄₋₄, -O-R₄₋₅, -A-Linker-R₄₋₆, -P(=O)(OR₄₋₇)OR₄₋₈, and the following monosaccharide group, wherein 1-position substitution of the monosaccharide is α-substitution or β-substitution: 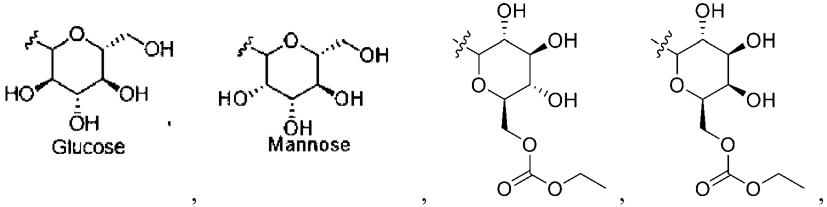 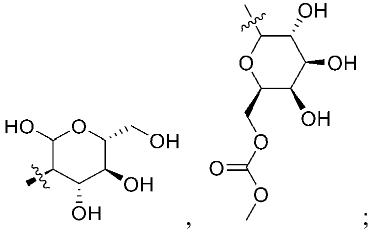
wherein:
R₄₋₂ is selected from: -OH, phenyl, C₁₋₁₃ straight or branched alkyl, C₁₋₅ straight or branched alkoxy, -C=C-COOH, -C=C-C₁₋₅ straight or branched alkoxycarbonyl, -C₁₋₅ straight or branched alkylene-R₄₋₉;
R₄₋₃ and R₄₋₄ are the same or different, and are individually and independently selected from: H, C₁₋₅ straight or branched alkyl;
alternatively, R₄₋₃ and R₄₋₄ are are interconnected and, together with nitrogen atoms to which they are attached, jointly form 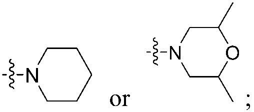
R₄₋₅ is selected from: **H,** C₁₋₅ straight or branched alkyl, -C₁₋₅ straight or branched alkylene-phenyl;
Linker is an optionally present group and, when present, is selected from: C₁₋₅ straight or branched alkylene, -B-C₁₋₅ straight or branched alkylene-, -B-C₁₋₅ straight or branched alkylene-phenylene-, -B-phenylene-C₁₋₅ straight or branched alkylene-;
A is selected from: -C(=O)-, C₁₋₅ straight or branched alkylene;
R₃ is selected from: -H, C₁₋₅ straight or branched alkyl;
R₄₋₇ and R₄₋₈ are the same or different, and are individually and independently selected from: H, C₁₋₅ straight or branched alkyl, -C₁₋₅ straight or branched alkylene-phenyl;
R₄₋₉ is selected from: 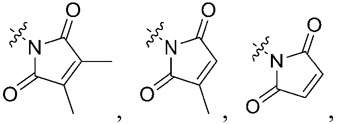 and phenyl;
R₄₋₁₀ is selected from H, C₁₋₅ straight or branched alkyl;
alternatively, R₁ and R₂ are are interconnected and, together with nitrogen atoms to which they are attached, jointly form: 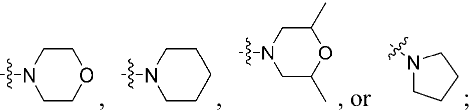
X is selected from -H; and
each B is individually and independently selected from -O-, -S-, -NH-.

6. The compound represented by Formula (I) according to claim 1, or an optical isomer thereof, or a pharmaceutically acceptable salt thereof, or a solvate thereof (optionally, the solvate is a hydrate), or an inclusion complex thereof, or a racemate thereof, or a co-crystal thereof, or an isotope-labeled compound thereof, or nitrogen oxides thereof, or a prodrug thereof, or an active metabolite thereof, wherein:
R₁ and R₂ are the same or different, and are individually and independently selected from: -H, C₁₋₅ straight or branched alkyl, -C₁₋₅ straight or branched alkylene-C(=O)O-C₁₋₅ straight or branched alkyl, -S(=O)₂-C₁₋₅ straight or branched alkyl, -C(=O) R₄₋₂, -C(=O)N(R₄₋₃)R₄₋₄, -O-R₄₋₅, -A-Linker-R₄₋₆, -P(=O)(OR₄₋₇)OR₄₋₈, and the following monosaccharide group, wherein 1-position substitution of the monosaccharide is α-substitution or β-substitution: 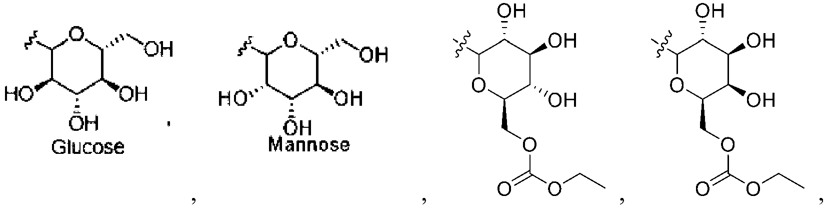 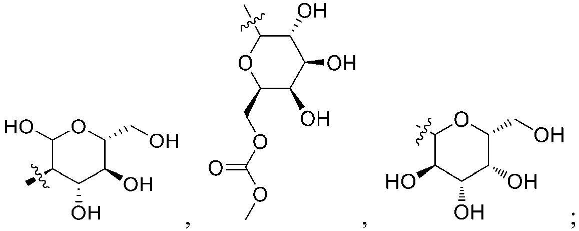
wherein:
R₄₋₂ is selected from: -OH, phenyl, C₁₋₁₃ straight or branched alkyl, C₁₋₅ straight or branched alkoxy, -C=C-COOH, -C=C-C₁₋₅ straight or branched alkoxycarbonyl, -C₁₋₅ straight or branched alkylene-R₄₋₉;
R₄₋₃ and R₄₋₄ are the same or different, and are individually and independently selected from: H, C₁₋₅ straight or branched alkyl;
alternatively, R₄₋₃ and R₄₋₄ are are interconnected and, together with nitrogen atoms to which they are attached, jointly form 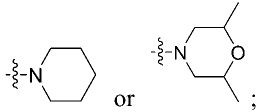
R₄₋₅ is selected from: H, C₁₋₅ straight or branched alkyl, -C₁₋₅ straight or branched alkylene-phenyl;
Linker is an optionally present group and, when present, is selected from: C₁₋₅ straight or branched alkylene, -B-C₁₋₅ straight or branched alkylene-, -B-C₁₋₅ straight or branched alkylene-phenylene-, -B-phenylene-C₁₋₅ straight or branched alkylene-;
A is selected from: -C(=O)-, C₁₋₅ straight or branched alkylene;
R₃ is selected from: -H, C₁₋₅ straight or branched alkyl;
R₄₋₇ and R₄₋₈ are the same or different, and are individually and independently selected from: H, C₁₋₅ straight or branched alkyl, -C₁₋₅ straight or branched alkylene-phenyl;
R₄₋₉ is selected from: 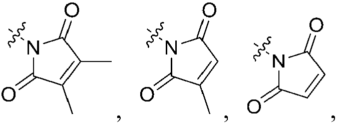 and phenyl;
R₄₋₁₀ is selected from H, C₁₋₅ straight or branched alkyl;
alternatively, R₁ and R₂ are are interconnected and, together with nitrogen atoms to which they are attached, jointly form: 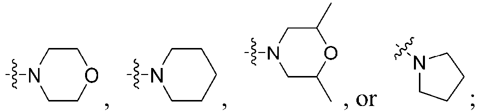
X is selected from -H; alternatively, X and R₁ jointly form a cyclic amide structure 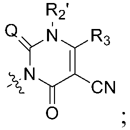
R₂' is selected from: -H, C₁₋₅ straight or branched alkyl, phenyl, cyclopentyl, -C₁₋₅ straight or branched alkylene-phenyl, -C₁₋₅ straight or branched alkylene-C(=O)OH, -C₁₋₅ straight or branched alkylene-C(=O)O-C₁₋₅ straight or branched alkyl, -C(=O)-phenyl, -alkylene-O-C(=O)R₄₋₁₁, -alkylene-O-P(=O)(OR₄₋₇)OR₄₋₈, -alkylene-O-C(=O)-OR₄₋₁₀;
R₄₋₁₀ is C₁₋₅ straight or branched alkyl;
R₄₋₁₁ is C₁₋₅ straight or branched alkyl, phenyl;
each B is individually and independently selected from -O-, -S-, -NH-.;
Q is O or -O-cyclopentyl; and
R₃ is selected from: C₁₋₅ straight or branched alkyl, -C₁₋₅ straight or branched alkylene-O-C(=O)-O-C₁₋₅ straight or branched alkyl.

7. The compound represented by Formula (I) according to any one of claims 1 to 6, or an optical isomer thereof, or a pharmaceutically acceptable salt thereof, or a solvate thereof (optionally, the solvate is a hydrate), or an inclusion complex thereof, or a racemate thereof, or a co-crystal thereof, or an isotope-labeled compound thereof, or nitrogen oxides thereof, or a prodrug thereof, or an active metabolite thereof, being selected from the following compounds:

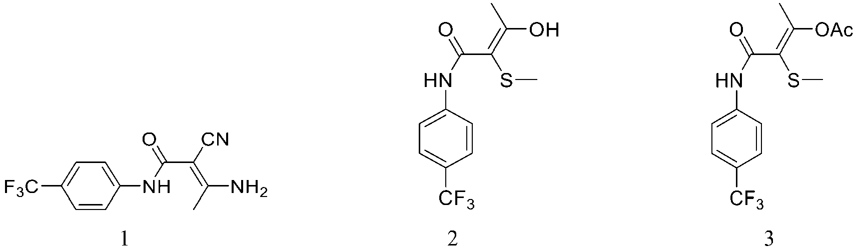

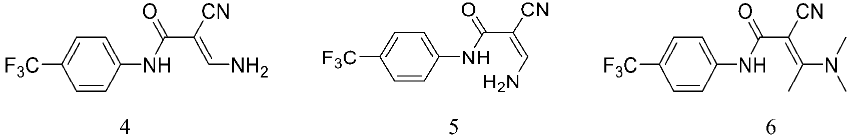

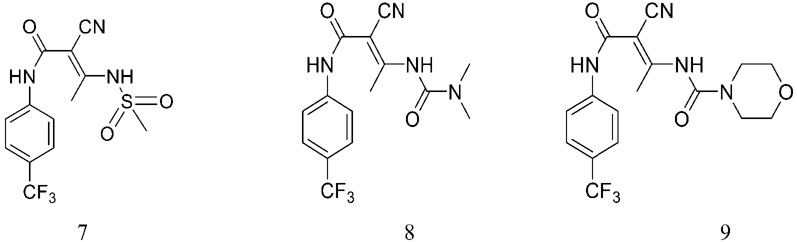

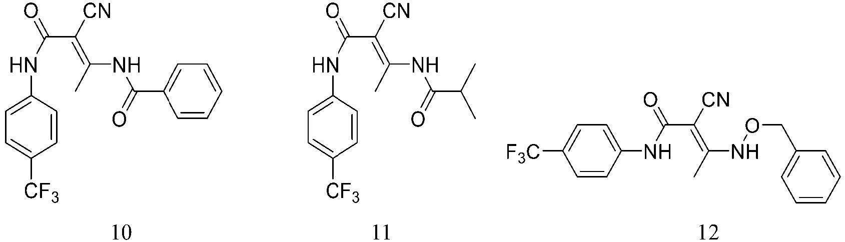

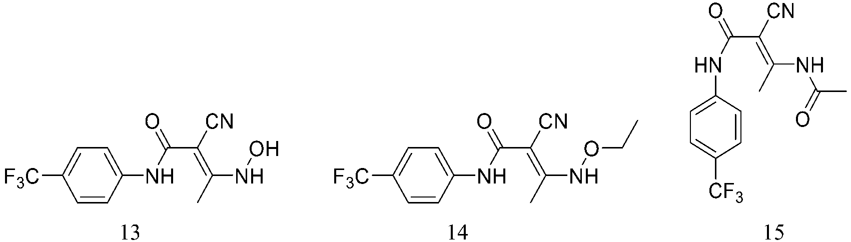

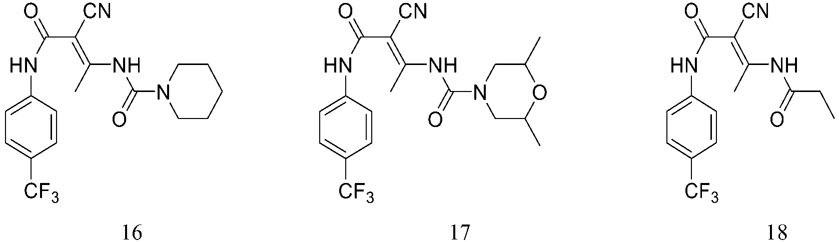

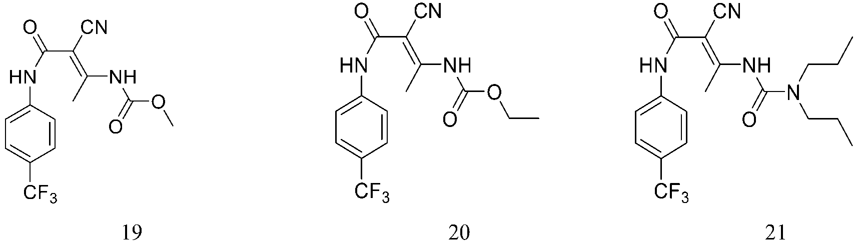

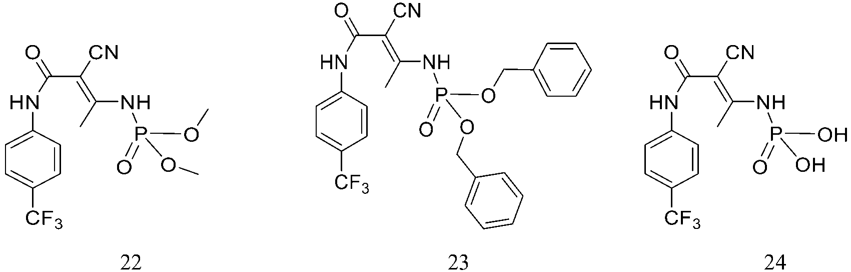

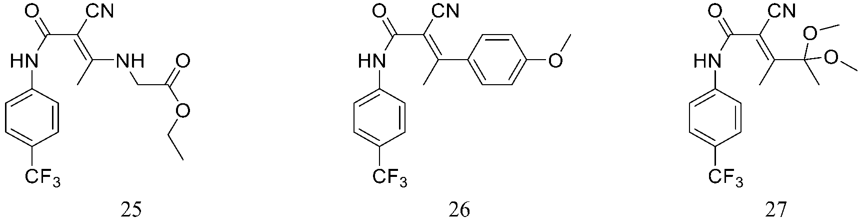

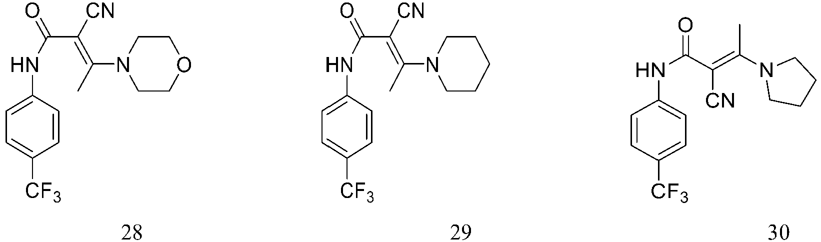

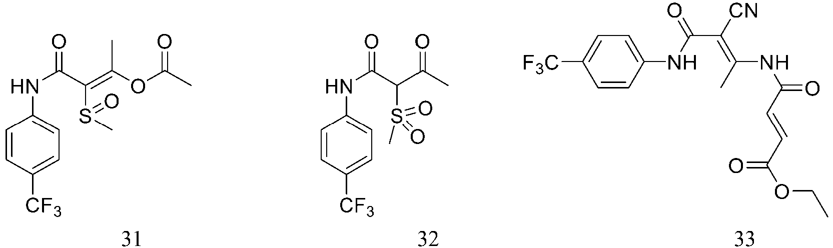

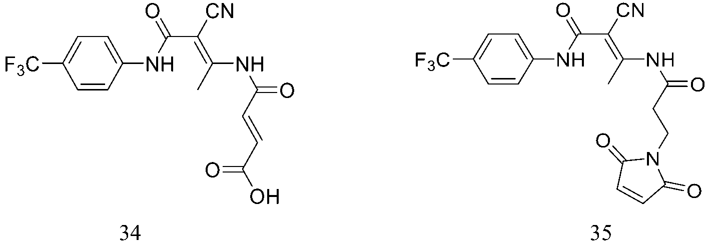

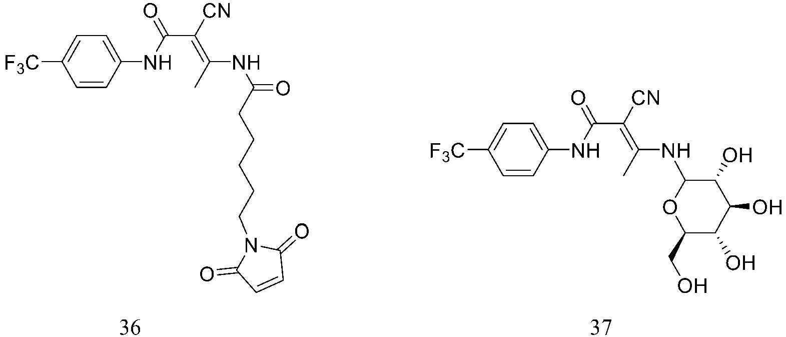

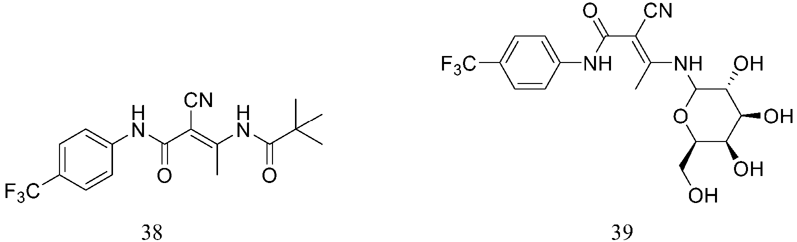

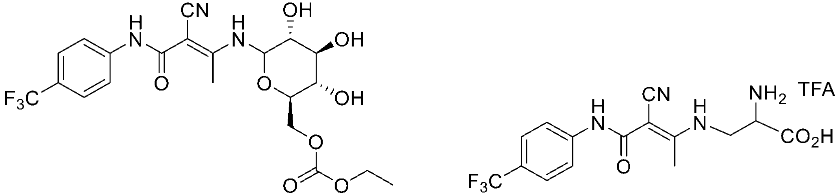

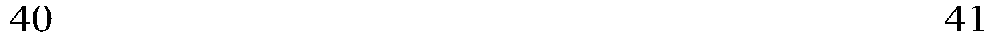

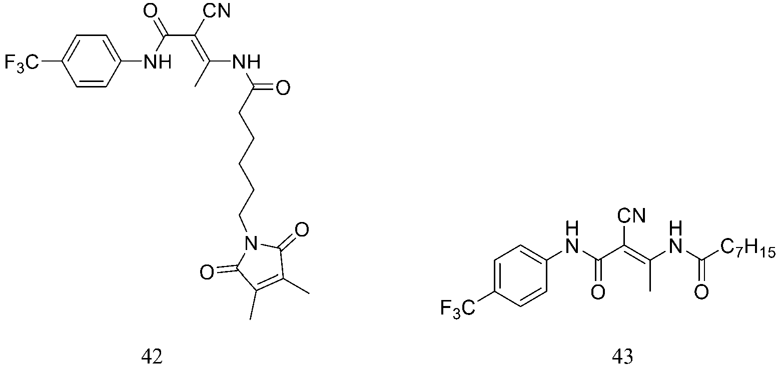

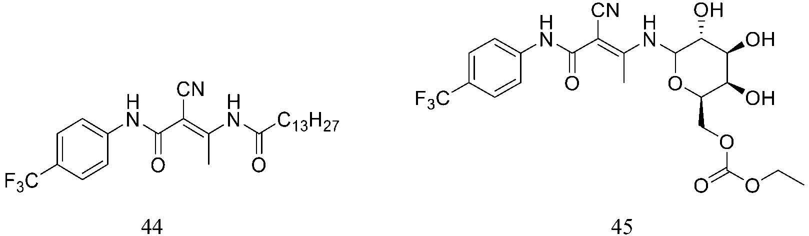

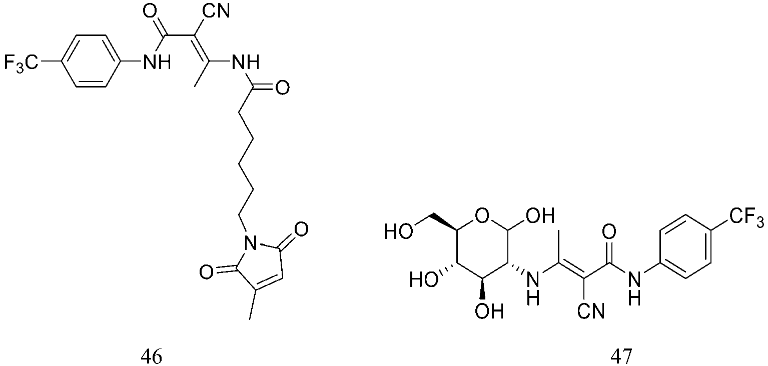

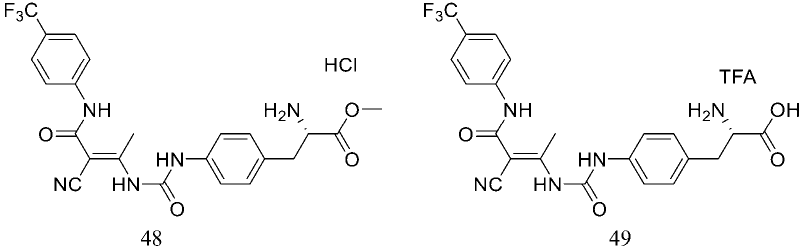

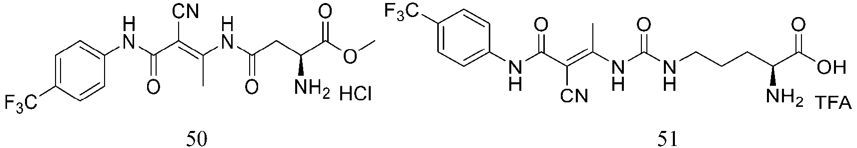

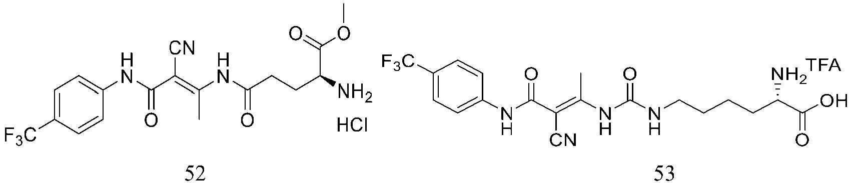

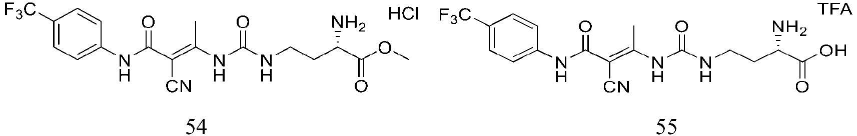

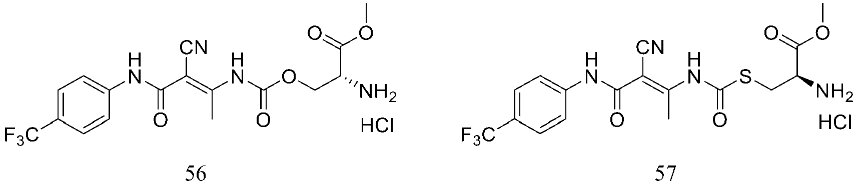

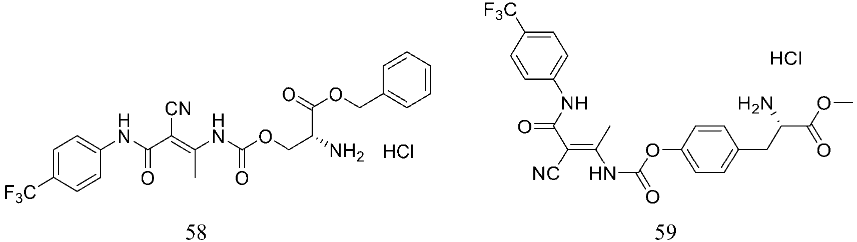

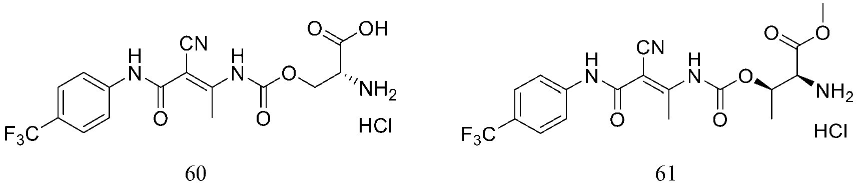

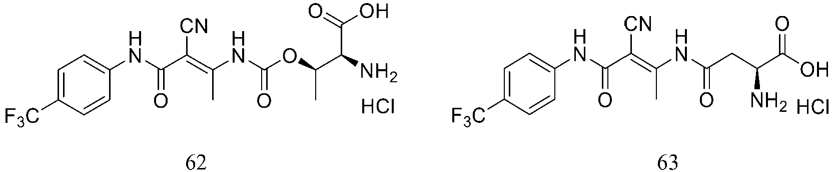

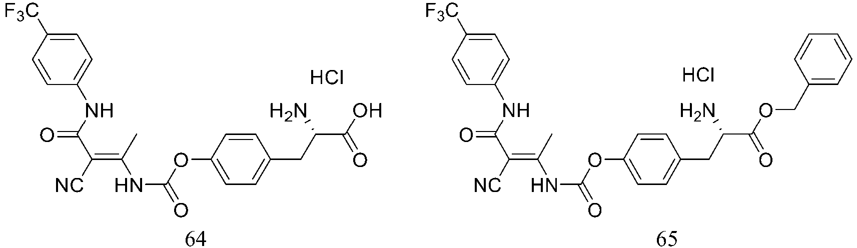

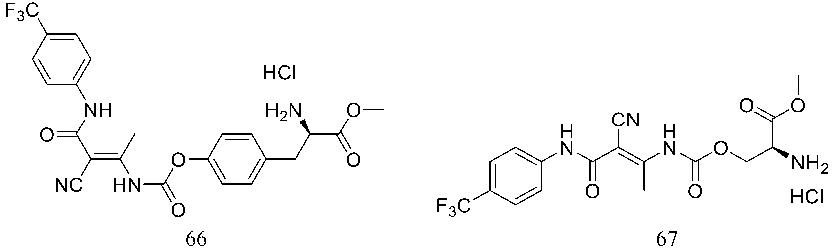

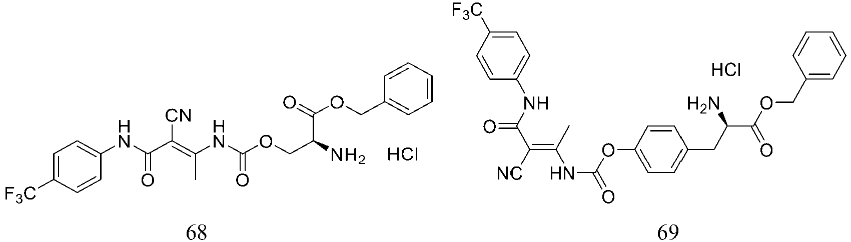

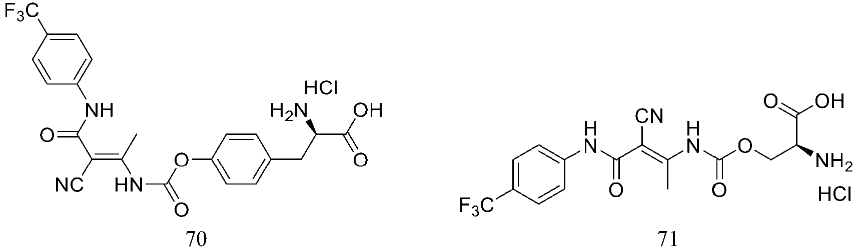

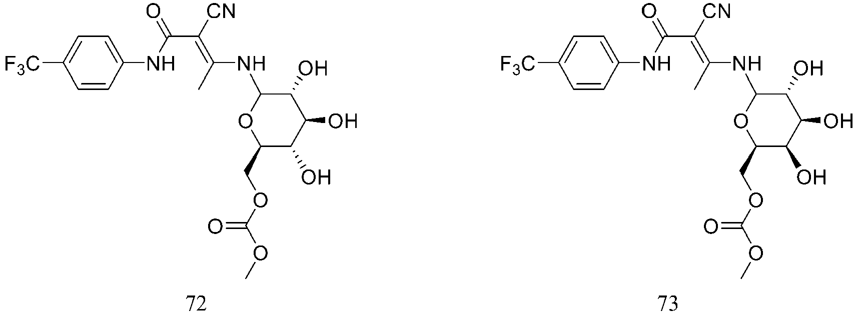

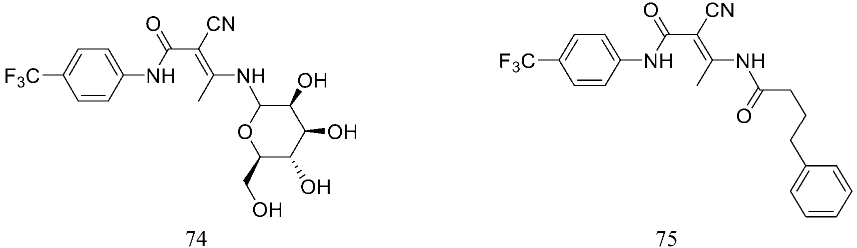

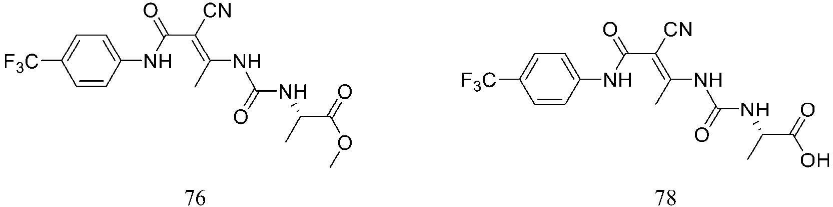

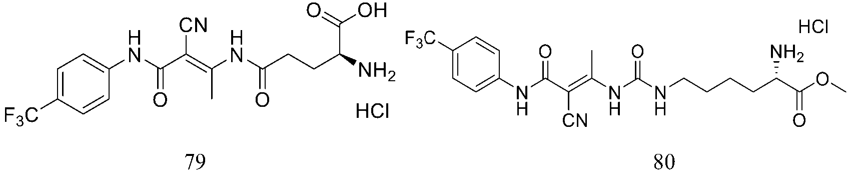

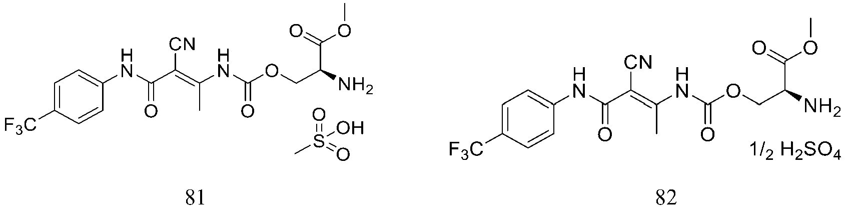

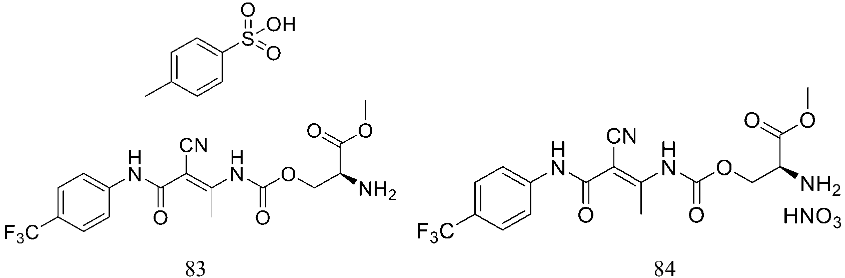

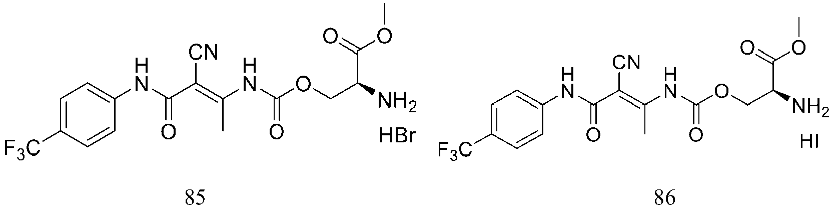

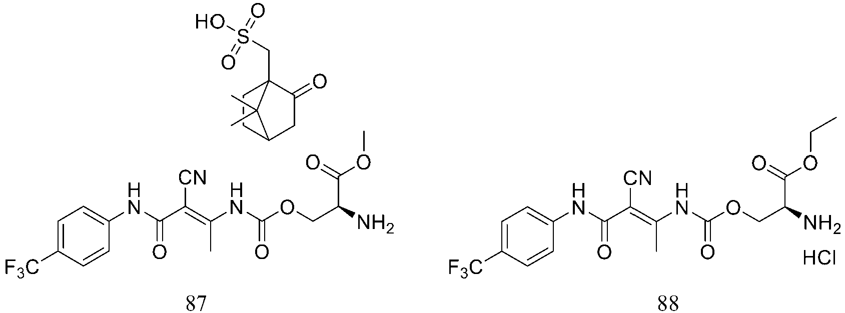

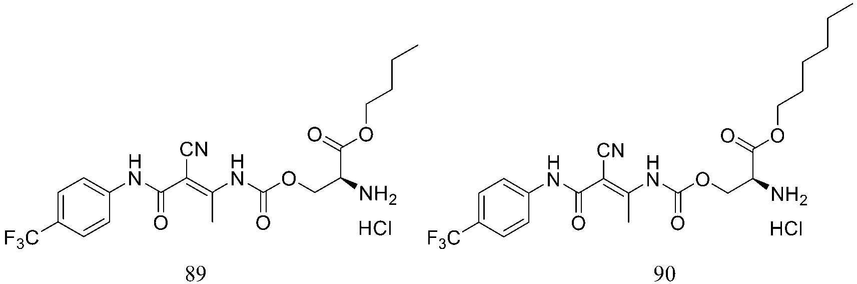

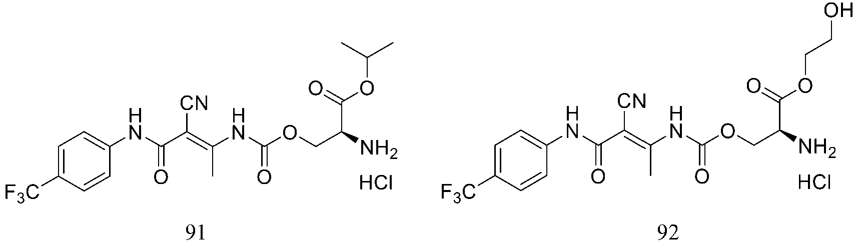

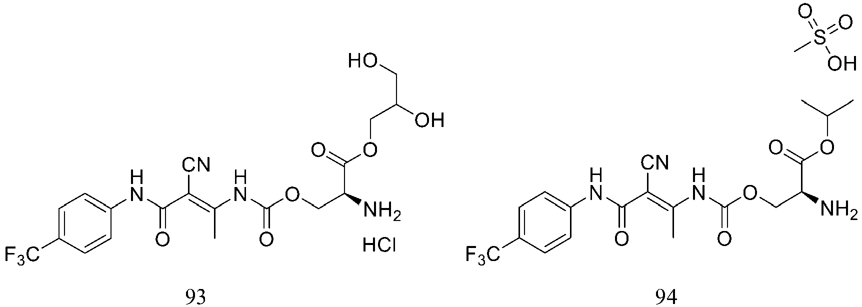

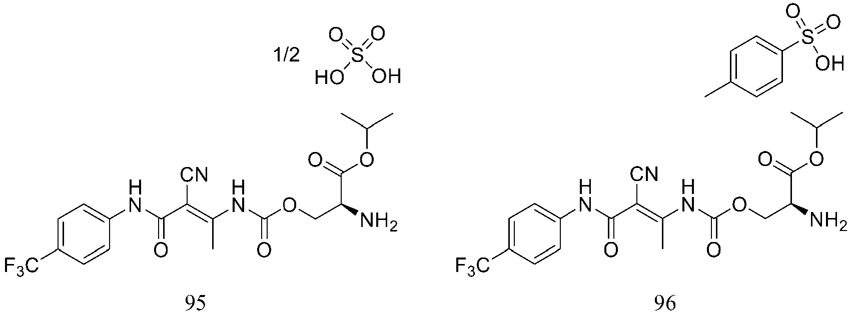

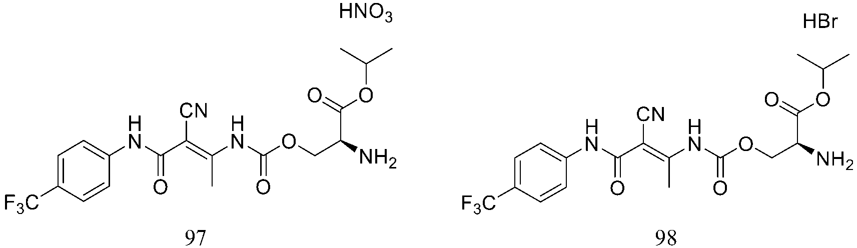

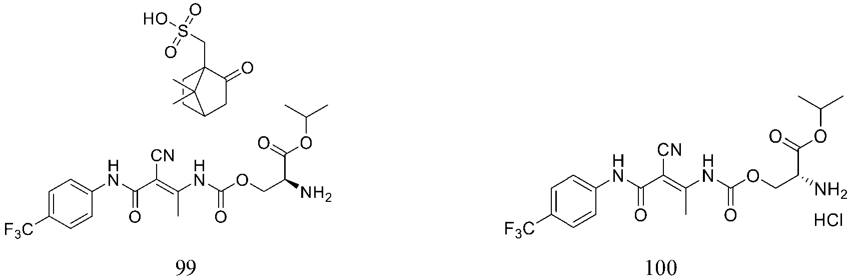

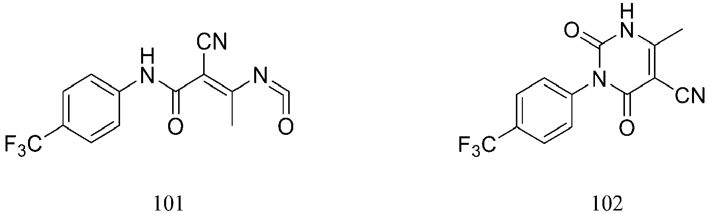

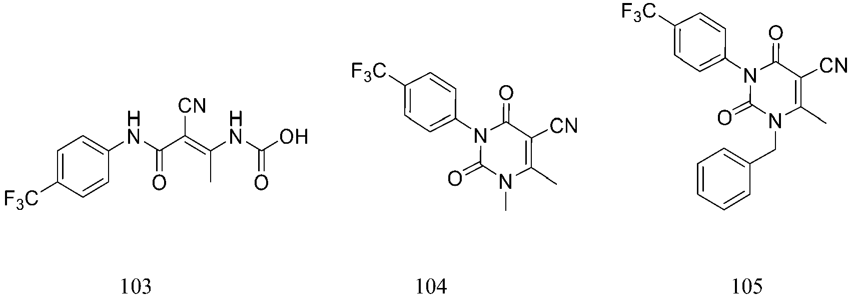

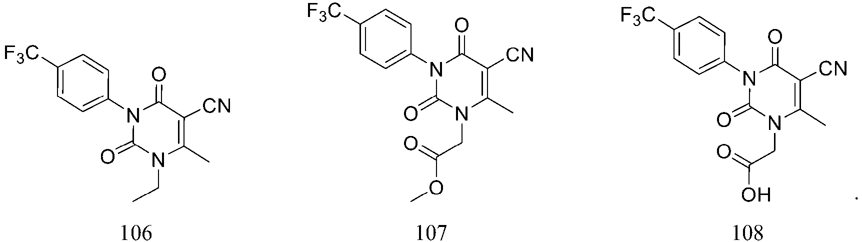

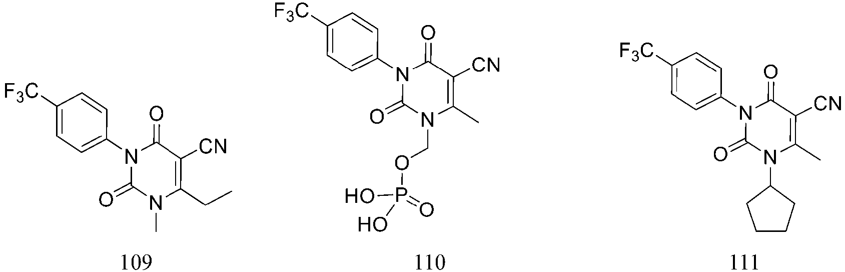

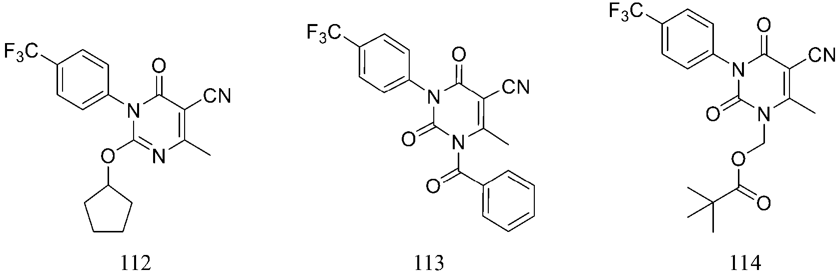

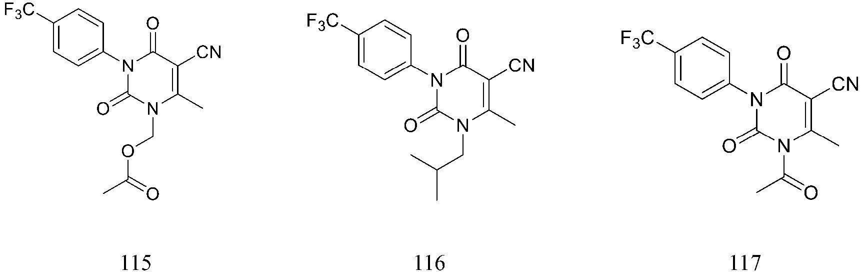

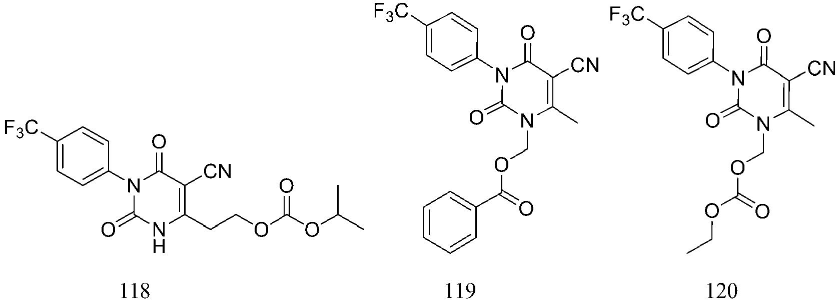

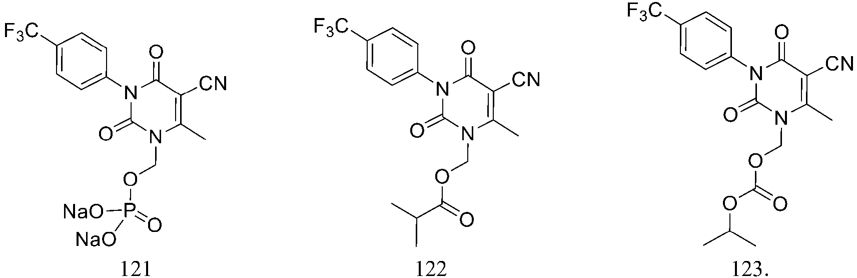

8. The compound represented by Formula (I) according to any one of claims 1 to 7, or an optical isomer thereof, or a pharmaceutically acceptable salt thereof, or a solvate thereof (optionally, the solvate is a hydrate), or an inclusion complex thereof, or a racemate thereof, or a co-crystal thereof, or an isotope-labeled compound thereof, or nitrogen oxides thereof, or a prodrug thereof, or an active metabolite thereof, wherein:
the pharmaceutically acceptable salts are inorganic acid salts or organic acid salts, and
optionally selected from hydrochloric acid, sulfuric acid, phosphoric acid, nitric acid, trifluoroacetic acid, methanesulfonic acid, ethanesulfonic acid, tartaric acid, formic acid, acetic acid, salicylic acid, citric acid, succinic acid, fumaric acid, maleic acid, benzoic acid, hydrobromic acid, hydroiodic acid, benzenesulfonic acid, p-toluenesulfonic acid, and camphorsulfonic acid.

9. A method for preparing a compound of Formula (I) according to any one of claims 1 to 7, or an optical isomer thereof, or a pharmaceutically acceptable salt thereof, or a solvate thereof (e.g., a hydrate), or an inclusion complex thereof, or a racemate thereof, or a co-crystal thereof, or an isotope-labeled compound thereof, or nitrogen oxides thereof, or a prodrug thereof, or an active metabolite thereof;
comprising: subjecting a compound of Formula (A) and a compound of Formula (B) to Step A to afford a compound of Formula (I):
Step A: 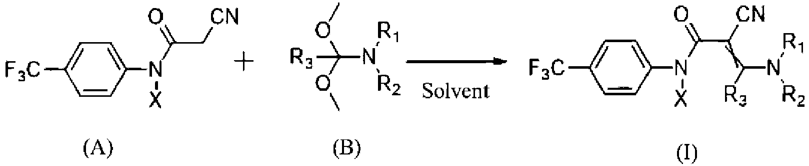
in Formula (A), X is as shown in Formula (I);
in Formula (B), R₁, R₂ and R₃ are as shown in Formula (I);
optionally, the compound of Formula (I) is prepared from Formula (A) and Formula (B) via an addition-elimination reaction in a suitable solvent (such as xylene or toluene) at a temperature ranging from 50°C to 120°C;
optionally, Formula (A) can be synthesized by, but not limited to, Method a;
Method a: 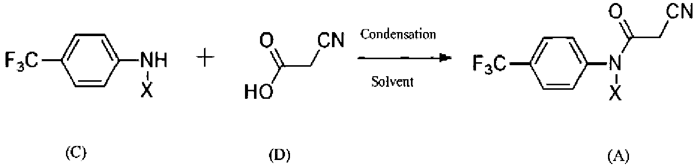
optionally, the compound of Formula (A) is prepared by condensation reaction of Formula (C) and Formula (D) catalyzed by a condensing agent (such as EDCI, HATU, HBTU, PyBOP, etc.), in the presence or absence of a base (such as pyridine, TEA, DIPEA, DBU, DBN, etc.), in a suitable solvent (such as THF, DCM, DCE, ACN, DMF, DMAC) at a temperature ranging from -10°C to 50°C;
optionally, Formula (B) can be synthesized by, but not limited to, Method b;
Method b: 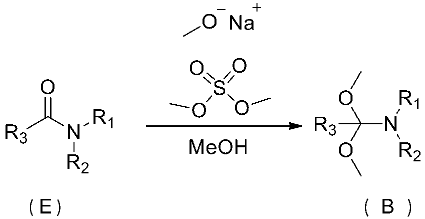
optionally, the compound of Formula (B) is prepared from Formula (E) via addition followed by methylation in the presence of sodium methoxide and dimethyl sulfate, with DMSO as solvent, at a temperature ranging from -10°C to 30°C;
optionally, the method further comprises: obtaining the compound of Formula (I) by subjecting the compound of Formula (F) and the compound of Formula (G) to Step B when R₁ and R₂ in Formula (I) are the same or different, and are individually and independently H, alkyl, -alkylene-C(=O)O-alkyl, optionally substituted monosaccharide group, or when R₁ and R₂ are interconnected and form, together with nitrogen atoms to which they are attached, an optionally substituted aliphatic heterocyclyl group:
Step B: 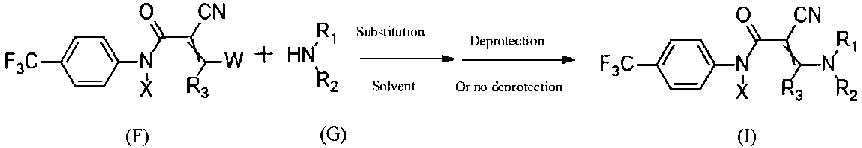
in Formula (F), X and R₃ are as defined in Formula (I), and W is dimethylamino, pyrrolyl, piperidinyl, or morpholinyl; in Formula (G), R₁ and R₂ are as defined in Formula (I);
optionally, the compound of Formula (I) is prepared by substitution reaction of Formula (F) and Formula (G) catalyzed by a base (such as pyridine, TEA, DIPEA, DBU, DBN, etc.), with or without deprotection, in a suitable solvent (such as DMF, DMAC, DMSO, THF, DCM, DCE, etc.) at a temperature ranging from 0°C to 100°C;
the method further comprises: obtaining the compound of Formula (I) by subjecting the compound of Formula (H) and the compound of Formula (J) to Step C when R₁ and R₂ in Formula (I) are different, and are individually and independently -S(=O)₂R₄₋₁, -C(=O)R₄₋₂, -C(=O)N(R₄₋₃)R₄₋₄, -A-Linker-R₄₋₆, -P(=O)(OR₄₋₇)OR₄₋₈:
Step C: 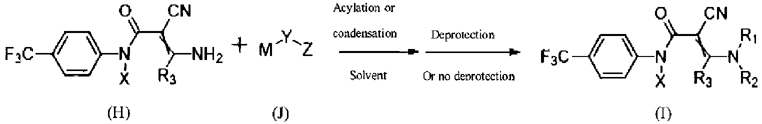
in Formula (H), X and R₃ are as defined in Formula (I); in Formula (J), M is halogen, hydroxy, imidazolyl, or p-nitrophenoxy, Y is carbonyl, sulfonyl, or phosphoryl; or M and Y form an isocyanate group; Z is optionally substituted alkyl, optionally substituted alkyloxy, optionally substituted aryl, optionally substituted aryloxy, optionally substituted amino, optionally substituted alkenyl, or optionally substituted alkylthio;
when M is halogen, and p-nitrophenoxy, imidazolyl, or M and Y form an isocyanate group, the compound of Formula (I) is optionally prepared by acylation reaction of Formula (H) and Formula (J) catalyzed by a base (such as pyridine, TEA, DIPEA, DBU, DBN, sodium hydride, etc.), with or without deprotection, in a suitable solvent (such as THF, DCM, DCE, ACN, DMF, DMAC, etc.) at a temperature ranging from -10°C to 30°C;
when M is hydroxy, the compound of Formula (I) is optionally prepared by condensation reaction of Formula (H) and Formula (J) catalyzed by a condensing agent (such as EDCI, HATU, HBTU, PyBOP, etc.), with or without deprotection, in a base (such as pyridine, TEA, DIPEA, DMAP, DBU, DBN, sodium hydride, etc.) and a suitable solvent (such as THF, DCM, DCE, ACN, DMF, DMAC, etc.) at a temperature ranging from -10°C to 50°C;
optionally, the method further comprises: when X and R₁ in Formula (I) jointly form the following cyclic amide structure 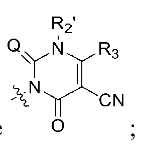
R₂' is as defined in Formula (I), and Formula (I) can be afforded by intramolecular cyclization of Formula (H) and reaction with the compound of Formula (J) via Step D:
Step D: 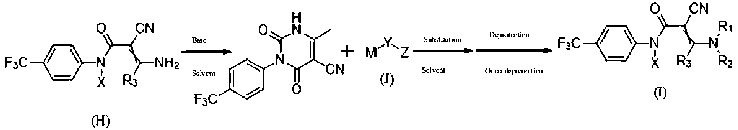
when R₂' is H, Q is O, and R₃ is CH₃; M is halogen (preferably, iodides or bromides), Y is alkyl, carbonyl, alkylene carbonyl, alkylene oxycarbonyl, alkylene oxyphosphoryl, or phosphoryl; Z is optionally substituted alkyl, C₃-C₇ monocyclic cycloalkyl, optionally substituted alkyloxy, optionally substituted aryl, or optionally substituted aryloxy; and
optionally, Formula (H) is capable of intramolecular cyclization by reacting with carbonyldiimidazole, or 4-nitrophenyl chloromethyl carbonate, or triphosgene under catalysis of a base (such as potassium carbonate, cesium carbonate, pyridine, TEA, DIPEA, DBU, DBN, NaHCO₃, sodium hydroxide, sodium hydride, etc.) in a suitable solvent (such as THF, DCM, DCE, ACN, DMF, acetone, DMAC, etc.) at a temperature ranging from -10°C to 60°C, and then the compound of Formula (I) is prepared by substitution reaction with Formula (J) under catalysis of a base (such as potassium carbonate, cesium carbonate, pyridine, TEA, DIPEA, DBU, DBN, sodium hydride, etc.), with or without deprotection, in a suitable solvent (such as THF, DCM, DCE, ACN, DMF, DMAC, etc.) at a temperature ranging from -10°C to 80°C.

10. A pharmaceutical composition comprising a compound of the Formula (I) according to any one of claims 1 to 8, or an optical isomer thereof, or a pharmaceutically acceptable salt thereof, or a solvate thereof (e.g., a hydrate), or an inclusion complex thereof, or a racemate thereof, or a co-crystal thereof, or an isotope-labeled compound thereof, or nitrogen oxides thereof, or a prodrug thereof, or an active metabolite thereof, and a pharmaceutically acceptable excipient;
optionally, the pharmaceutically acceptable excipient is selected from: fillers, disintegrants, lubricants, glidants, effervescent agents, flavoring agents, preservatives, solubilizers, cosolvents, antioxidants, photodegradation inhibitors, pH regulators, emulsifiers, bacteriostatic preservatives, local anesthetics, complexing agents, non-aqueous solvents, coating materials, or other excipients;
optionally, fillers include a combination of one or more of lactose, mannitol, and calcium carbonate;
optionally, the binders include a combination of one or more of sucrose, starch, povidone, and sodium carboxymethylcellulose;
optionally, the disintegrants include a combination of one or more of starch, crospovidone, croscarmellose sodium, and effervescent disintegrants;
optionally, non-aqueous solvents include a combination of one or more of soybean oil, castor oil, and peanut oil;
optionally, solubilizers include a combination of one or more of Tween 80, Tween 60, and poloxamer 68;
optionally, cosolvents include a combination of one or more of sodium benzoate, sodium salicylate, and sodium p-aminobenzoate;
optionally, the pharmaceutical composition can be formulated as a solid oral preparation, a liquid oral preparation, an injection, or a transdermal preparation;
optionally, the solid and liquid oral preparations include: tablets, dispersible tablets, sugar-coated tablets, granules, dry powders, capsules, syrups, and solutions;
optionally, the injection includes small-volume injections, large-volume infusions, and lyophilized powder injections; and
optionally, the transdermal preparation includes ointments, plasters, liniments, aerosols, conventional patches, adhesive dispersion-type patches, peripheral adhesive matrix-type patches, reservoir-type patches, and cataplasms.

11. Use of a compound of Formula (I) according to any one of claims 1 to 8, or an optical isomer thereof, or a pharmaceutically acceptable salt thereof, or a solvate thereof (e.g., a hydrate), or an inclusion complex thereof, or a racemate thereof, or a co-crystal thereof, or an isotope-labeled compound thereof, or nitrogen oxides thereof, or a prodrug thereof, or an active metabolite thereof, or the pharmaceutical composition according to claim 10, in the preparation of a medicament for the prophylaxis and/or treatment of inflammation, pain, multiple sclerosis, neuromyelitis optica, autoimmune diseases, or cancer;
optionally, the inflammation is selected from rheumatoid arthritis, and dermatitis;
optionally, the multiple sclerosis (MS) is selected from clinically isolated syndrome (CIS) MS, relapsing-remitting MS, primary progressive MS, and secondary progressive MS;
optionally, the neuromyelitis optica (NMO) is selected from monophasic NMO, relapsing NMO, and progressive NMO;
optionally, the autoimmune disease is selected from systemic lupus erythematosus, psoriasis, and anti-transplant rejection;
optionally, the cancer is selected from renal cancer, prostate cancer, and lung cancer; and
optionally, the cancer cells include human renal cancer cells 786-O, human prostate cancer cells LNCaP, PC3, DU145, human large cell lung cancer cells H460, and human non-small cell lung cancer A549.
